# EUROPEAN PATENT APPLICATION

(11) **EP 4 534 660 A1**
(43) Date of publication of application: **09.04.2025**
(21) Application number: 23811999.4
(22) Date of filing: 12.04.2023
(51) Int. Cl.: C12N 9/12, C12N 15/113, C12N 15/10, C12N 15/86

(54) **ENGINEERED CAS12F PROTEIN WITH EXPANDED TARGETABLE RANGE AND USES THEREOF**

(30) Priority: 27.05.2022 KR 20220065600
(71) Applicant: GenKOre Inc., Daejeon 34141 (KR)
(72) Inventor: KIM, Yong Sam, Seoul 06767 (KR); KIM, Do Yon, Daejeon 34069 (KR); LEE, Yu Jin, Daejeon 34141 (KR); JEONG, Dong Min, Daejeon 34141 (KR)
(74) Representative: Ullrich & Naumann PartG mbB
(86) International application number: PCT/KR2023/004971
(87) International publication number: WO 2023/229222

(57) **Abstract**

The present invention relates to an engineered Cas12 protein (for example, a Cas12f protein) having an expanded targeting range and use thereof. The engineered Cas protein comprises an amino acid substitution at one or more amino acid positions selected from the group consisting of 159, 164, 170, 174, 184, 188, 191, 225, 230 and 272 of SEQ ID NO: 1 or at one or more amino acid positions corresponding thereto, and comprises an amino acid sequence having at least 80% identity to the sequence represented by SEQ ID NO: 1.

## Description

### Technical Field

This application claims priority based on Korean Patent Application No. 2022-0065600, filed on May 27, 2022, the entire disclosure of which is incorporated herein by reference.

The present disclosure relates to an engineered Cas12 protein (for example, Cas12f protein) with an expanded targetable range and a use thereof.

### Background Art

A CRISPR/Cas system, which functions as an adaptive immune system in bacteria, archaea, and the like, is being developed as a multipurpose gene editing tool in various fields including gene therapy for various diseases (for example, genetic diseases, cancer, or the like). The CRISPR/Cas system generally includes a protein component (RNA-guided nuclease) and a nucleic acid component (guide RNA or gRNA), and these two components form a complex to interact with a specific target DNA sequence. In addition, the complex can edit or change a target sequence, for example, by site-specific DNA cleavage. In order for a target sequence of double-stranded DNA to interact with the complex, the double-stranded DNA must comprise a target strand containing a target sequence complementary to the guide sequence of a guide RNA and a non-target strand containing a protospacer adjacent motif (PAM) for target recognition by a Cas protein.

Small Cas proteins, such as Cas12f (also referred to as Cas14) and Cas12j (CasΦ), which are classified as type V CRISPR nucleases of class 2, have been identified from archaea and large bacteriophages (*see* Harrington, L. B. et al. Programmed DNA destruction by miniature CRISPR-Cas14 enzymes. Science 362, 839-842, 2018). These small CRISPR-Cas effectors consist of about 400 to 700 amino acid residues and contain a RuvC nuclease domain. The Cas12f effector was originally reported to exhibit only single-stranded DNA cleavage activity or extremely low indel activity in eukaryotic cells (see Karvelis, T. et al. PAM recognition by miniature CRISPR-Cas14 triggers programmable double-stranded DNA cleavage. Preprint at bioRxiv https://doi.org/10.1101/654897, 2019). However, the present inventors have identified that the natural guide RNA of archaeal Un1Cas12f1 can be engineered to increase its average indel activity in eukaryotic cells by more than 800-fold, and thus have successfully converted small Cas proteins, such as Cas12f effectors, into efficient gene editing tools (*see* Kim, D. Y et al. Efficient CRISPR editing with a hypercompact Cas12f1 and engineered guide RNAs delivered by adeno-associated virus. Nat. Biotechnol. 40, 94-102, 2021). This engineered guide RNA is also smaller than the natural guide RNA, which allows it to be delivered with a small Cas protein, such as Cas12f, using adeno-associated virus (AAV) that is considered a validated delivery platform and has a limited packaging size (about 4.7 kb). AAV is a vehicle approved by the U.S. FDA with proven safety, persistence, and mass production potential, and an AAV-deliverable CRISPR system is particularly promising as a therapeutic for genetic diseases *in vivo.*

Despite these advantages, similar to other Cas proteins, the Cas12f effector requires a specific PAM sequence for binding to target DNA, which limits its targetable range to a certain range of genomic sites where the corresponding PAM sequence is present. For example, the wild-type Cas12f protein has preference for the PAM sequence TTTR (TTTA or TTTG) (*see* Harrington, L. B. et al. Programmed DNA destruction by miniature CRISPR-Cas14 enzymes. Science 362, 839, 2018), and thus may exhibit limited editing ability for a target gene in a case where no PAM sequence is present near the target gene. This is because eukaryotes, in particular, mammals and plants have genomes with highly complex and heterogeneous DNA sequences. Moreover, editing based on homology-directed repair (HDR) or base editor (for example, dCas12f/cytidine deaminase and dCas12f/adenosine deaminase) requires a precise DNA binding site to achieve an optimal editing outcome.

Therefore, there is a need to develop a new CRISPR/Cas system that can recognize new PAM sequences for an expanded genomic targeting range.

### Disclosure of Invention

### Technical Problem

The object of the present disclosure is to solve the above-mentioned problems of the prior art.

An object of the present disclosure is to provide a Cas12f protein engineered to have expanded targetable sites.

Another object of the present disclosure is to provide an engineered Cas12f protein that can be programmed to have appropriate PAM specificity depending on a target gene.

Yet another object of the present disclosure is to provide a gene editing system, composition, or kit, comprising an engineered Cas12f protein that can be programmed to have expanded targetable sites or have appropriate PAM specificity depending on a target gene.

In addition, still yet another object of the present disclosure is to provide a vector or vector system, comprising the engineered Cas12f molecule, a method of using the engineered Cas12f molecule, and the like.

The object of the present disclosure is not limited to the above-mentioned objects. The objects of the present disclosure will become clearer from the following description and may be realized by means and combinations thereof as set forth in the claims.

### Solution to Problem

Representative configurations of the present disclosure to achieve the above-mentioned objects are as follows.

According to an aspect of the present disclosure, there is provided an engineered Cas protein, comprising amino acid substitution(s) at one or more amino acid residues selected from the group consisting of amino acids at positions 159, 164, 170, 174, 184, 188, 191, 225, 230, and 272 with respect to SEQ ID NO: 1 or at amino acid residue(s) corresponding thereto, wherein the engineered Cas protein comprises an amino acid sequence having at least 80% sequence identity to the sequence represented by SEQ ID NO: 1.

In an embodiment, the substitution(s) may comprise amino acid substitution(s) at one or more amino acid residues selected from the group consisting of amino acids at positions 170, 174, 184, 188, 191, 225, 230, and 272 or at amino acid residue(s) corresponding thereto, wherein the engineered Cas protein shows altered protospacer-adjacent motif (PAM) recognition specificity.

In an embodiment, the substitution(s) may comprise amino acid substitution(s) at one or more amino acid residues selected from the group consisting of amino acids at positions 159 and 164 or at amino acid residue(s) corresponding thereto, wherein the engineered Cas protein has an altered editing window for PAM-proximal regions.

In an embodiment, the substitution(s) may further comprise amino acid substitution(s) at one or more amino acid residues selected from the group consisting of amino acids at positions 159 and 164 or at amino acid residue(s) corresponding thereto.

In an embodiment, the substitution(s) may comprise one or more amino acid substitutions selected from the group consisting of following (1) to (10):
(1) 159W;
(2) 164Y;
(3) 170C or 170T;
(4) 174H, 174A, 174E, 174K, 174N, 174R, or 174T;
(5) 184H, 184N, 184R, 184S, or 184T;
(6) 188G, 188H, 188K, 188N, 188Q, 188R, 188S, 188T, or 188V;
(7) 191G, 191H, 191K, 191Q, or 191W;
(8) 225F or 225T;
(9) 230A, 230H, 230I, 230S, or 230T; and
(10) 272C, 272K, or 272R.

In an embodiment, the substitution(s) may comprise one or more substitutions selected from the group consisting of following (1) to (10):
(1) I159W;
(2) S164Y;
(3) S170C or S170T;
(4) Y174H, Y174A, Y174E, Y174K, Y174N, Y174R, or Y174T;
(5) A184H, A184N, A184R, A184S, or A184T;
(6) S188G, S188H, S188K, S188N, S188Q, S188R, S188S, S188T, or S188V;
(7) R191G, R191H, R191K, R191Q, or R191W;
(8) Q225F or Q225T;
(9) Y230A, Y230H, Y230I, Y230S, or Y230T; and
(10) Q272C, Q272K, or Q272R.

In an embodiment, the substitution(s) may comprise one or more amino acid substitutions selected from the group consisting of the amino acid substitutions described in Table 1.

In an embodiment, the engineered Cas protein may recognize a PAM sequence that a wild-type Cas12f protein either cannot recognize or poorly recognizes.

In an embodiment, the engineered Cas protein may recognize at least one PAM sequence selected from the group consisting of 5'-TVTN-3', 5'-TTVV-3', 5'-TGGG-3', and 5'-TTTN-3' (wherein N is A, T, G, or C, and V is A, G, or C).

In an embodiment, the engineered Cas protein may be an engineered Un1Cas12f1 protein or an engineered CWCas12f protein.

In an embodiment, the engineered Cas protein may further comprise at least one substitution selected from the group consisting of I159W and S164Y.

In an embodiment, the engineered Cas protein may have eliminated DNA cleavage activity.

In an embodiment, the engineered Cas protein may further comprise amino acid substitution(s) at one or more amino acid residues selected from the group consisting of amino acids at positions 354, 450, 518, and 538 with respect to SEQ ID NO: 1 or at amino acid residue(s) corresponding thereto.

In an embodiment, the substitution(s) may further comprise at least one amino acid substitution selected from following (a) to (d):
(a) 354A, 354Q, 354L, 354W, or 354V;
(b) 450A, 450Q, 450L, 450W, or 450V;
(c) 518A, 518Q, 518L, 518W, or 518V; and
(d) 538A, 538Q, 538L, 538W, or 538V.

In an embodiment, the engineered Cas protein may further comprise deletion, insertion, substitution, or addition of at least one amino acid and may be capable of forming a complex with a guide RNA.

In another aspect of the present disclosure, there is provided a fusion protein comprising (i) the above-described engineered Cas protein, (ii) a functional domain, and optionally (iii) a linker.

In an embodiment, the functional domain may comprise nuclease activity, nickase activity, recombinase activity, deaminase activity, methyltransferase activity, methylase activity, acetylase activity, acetyltransferase activity, transcriptional activation activity, transcriptional inhibition activity, or reverse transcriptase activity.

In an embodiment, the functional domain may have deaminase activity.

In an embodiment, the functional domain may comprise at least one expression regulatory domain.

In an embodiment, the expression regulatory domain may be selected from the group consisting of VP64, VPR, KRAB, MeCP2, DNMT, HAT, HDAC, TET, and p300.

In yet another aspect of the present disclosure, there is provided a polynucleotide encoding the engineered Cas protein of any one embodiment described above or the fusion protein of any one embodiment described above.

In still yet another aspect of the present disclosure, there is provided an engineered CRISPR/Cas system or composition, comprising:(i) the engineered Cas protein of any one embodiment described above, or a nucleic acid encoding the protein, and (ii) a guide RNA comprising a guide sequence hybridizable to a target sequence adjacent to a PAM, or a nucleic acid encoding the guide RNA.

In still yet another aspect of the present disclosure, there is provided a vector system, comprising at least one vector that comprises: (i) a first nucleic acid construct to which a nucleotide sequence encoding the above-described engineered Cas protein is operably linked; and (ii) a second nucleic acid construct to which a nucleotide sequence encoding a guide RNA is operably linked, the guide RNA comprising a guide sequence hybridizable with a target sequence adjacent to a PAM, wherein the nucleic acid constructs (i) and (ii) are located in the same vector or different vectors.

In an embodiment, the guide RNA may be an engineered guide RNA.

In an embodiment, the guide RNA may comprise a U-rich tail sequence linked to the 3'-end of the guide sequence, and the U-rich tail may be represented by 5'-(UₘV)ₙUₒ-3', wherein V is each independently A, C, or G; m and o are integers between 1 and 20; and n is an integer between 0 and 5.

In an embodiment, the guide RNA may comprise an engineered scaffold region, and the engineered scaffold region may comprise a nucleotide sequence having at least 50% sequence identity to a scaffold region of a wild-type Cas12f1 guide RNA sequence, which sequentially comprises, from the 5'-end, a first stem-loop region, a second stem-loop region, a third stem-loop region, a fourth stem-loop region, and a tracrRNA-crRNA complementarity region, wherein the guide RNA comprises at least one modification selected from the group consisting of following (1) to (4) with respect to the wild-type Cas12f1 guide RNA sequence:
deletion of at least a part of the first stem-loop region;
deletion of at least a part of the second stem-loop region;
deletion of at least a part of the tracrRNA-crRNA complementarity region; and
replacement of one or more uracil (U) residues with A, G, or C in three or more consecutive U residues when the consecutive U residues are present in the tracrRNA-crRNA complementarity region.

In an embodiment, the wild-type Cas12f1 guide RNA may comprise tracrRNA comprising the nucleotide sequence of SEQ ID NO: 11 and crRNA comprising the nucleotide sequence of SEQ ID NO: 12.

In an embodiment, the engineered scaffold region may comprise a sequence having at least 80% sequence identity to a sequence represented by Formula (I): 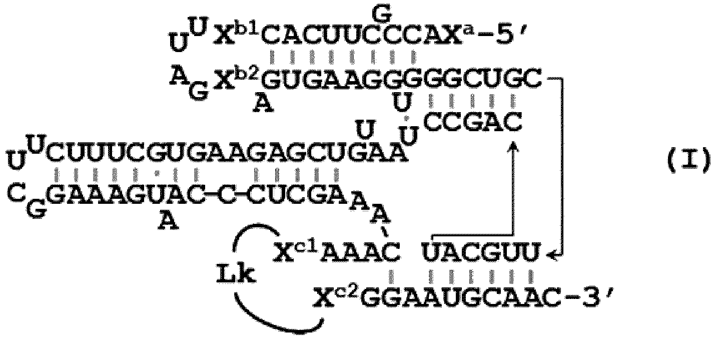 in Formula (I),
X^{a} comprises the nucleotide sequence of SEQ ID NO: 14 or a nucleotide sequence having the sequence of SEQ ID NO: 14 from which 1 to 20 nucleotides are deleted,
X^{b1} comprises the nucleotide sequence of SEQ ID NO: 25 or a nucleotide sequence having the sequence of SEQ ID NO: 25 from which 1 to 13 nucleotides are deleted,
X^{b2} comprises the nucleotide sequence of SEQ ID NO: 29 or a nucleotide sequence having the sequence of SEQ ID NO: 29 from which 1 to 14 nucleotides are deleted,
X^{c1} comprises the nucleotide sequence of SEQ ID NO: 39 or a nucleotide sequence having the sequence of SEQ ID NO: 39 from which 1 to 28 nucleotides are deleted,
X^{c2} comprises the nucleotide sequence of SEQ ID NO: 58 or a nucleotide sequence having the sequence of SEQ ID NO: 58 from which 1 to 27 nucleotides are deleted, and
Lk is a polynucleotide linker of 2 to 20 nucleotides in length or absent.

In an embodiment, in a case where three or more consecutive uracil (U) residues are present in a sequence of X^{c1}, the sequence of X^{c1} may comprise a modification in which at least one U residue thereof is replaced with A, G, or C.

In an embodiment, the deletion in the nucleotide sequence of X^{a}, the deletion in the nucleotide sequences of X^{b1} and X^{b2}, and/or the deletion in the nucleotide sequences of X^{c1} and X^{c2} may comprise deletion of one or more pairs of complementary nucleotides.

In an embodiment, the sequence 5'-X^{b1}UUAGX^{b2}-3' in Formula (I) is selected from the group consisting of SEQ ID NOs: 34 to 38 and 5'-UUAG-3'.

In an embodiment, the sequence 5'-X^{c1}-Lk-X^{c2}-3' in Formula (I) may be selected from the group consisting of SEQ ID NOs: 80 to 86 and 5'-Lk-3'.

In an embodiment, Lk may comprise a nucleotide sequence selected from the group consisting of 5'-GAAA-3', 5'-UUAG-3', 5'-UGAAAA-3', 5'-UUGAAAAA-3', 5'-UUCGAAAGAA-3' (SEQ ID NO: 76), 5'-UUCAGAAAUGAA-3' (SEQ ID NO: 77), 5'-UUCAUGAAAAUGAA-3' (SEQ ID NO: 78), and 5'-UUCAUUGAAAAAUGAA-3' (SEQ ID NO: 79).

In an embodiment, the scaffold region may comprise an engineered tracrRNA consisting of a nucleotide sequence selected from the group consisting of SEQ ID NOs: 87 to 132 and/or an engineered crRNA consisting of a nucleotide sequence selected from the group consisting of SEQ ID NOs: 133 to 148.

In an embodiment, the guide RNA may comprise a scaffold region sequence consisting of a nucleotide sequence selected from the group consisting of SEQ ID NOs: 13 and 149 to 186.

In an embodiment, the engineered Cas12 protein may form a complex with the guide RNA.

In an embodiment, the vector may be selected from the group consisting of a retroviral (retrovirus) vector, a lentiviral (lentivirus) vector, an adenoviral (adenovirus) vector, an adeno-associated viral (adeno-associated virus) vector, a vaccinia viral (vaccinia virus) vector, a poxviral (poxvirus) vector, a herpes simplex viral (herpes simplex virus) vector, and a phagemid vector.

In an embodiment, the vector may be selected from the group consisting of plasmid, naked DNA, DNA complex, mRNA (transcript), and amplicon.

In still yet another aspect of the present disclosure, there is provided a recombinant virus produced by the above-described vector system.

In still yet another aspect of the present disclosure, there is provided a method for modifying a target nucleic acid in a cell, comprising bringing, into contact with the cell, the above-described engineered Cas protein and a guide RNA, the above-described system or composition, the above-described vector system, or the above-described recombinant virus.

In an embodiment, the bringing-into-contact may be performed *in vitro.*

In an embodiment, the bringing-into-contact may be performed *in vivo.*

### Advantageous Effects of Invention

The present disclosure is based, at least in part, on identifying amino acid positions and amino acid substitutions, which can alter PAM specificity or editing window for PAM-proximal regions in a Cas12f protein (for example, Un1Cas12f1 or CWCas12f protein), thereby providing Cas12f protein variants that can be programmed to exhibit altered PAM specificity for gene editing of sequences that were previously not targetable. The Cas12f protein variants disclosed herein have specificity or preference for a PAM sequence that is different from that of the native protein, and can bring about alteration in the PAM sequence recognized by the original Cas12f protein by selecting or combining the positions and types of amino acid substitution(s). Accordingly, since it is possible to select and use an appropriate Cas12f protein variant depending on the PAM sequence in the vicinity of the sequence to be targeted, these PAM variants enable gene editing of sequences that could not be targeted by the wild-type Cas12f protein. Given that a hypercompact CRISPR/Cas12f system comprising a small Cas12 protein, in particular a Cas12f protein, and the engineered guide RNA as disclosed herein can be packaged into AAV, which is a validated vehicle for *in vivo* delivery and this makes it promising as a therapeutic for a variety of diseases, such expansion of the targetable sequence range enables treatment of a broader range of diseases.

### Brief Description of Drawings

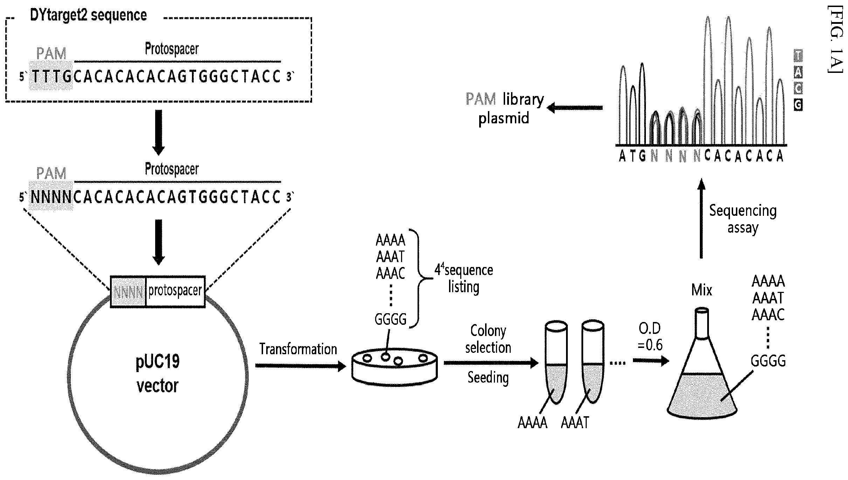

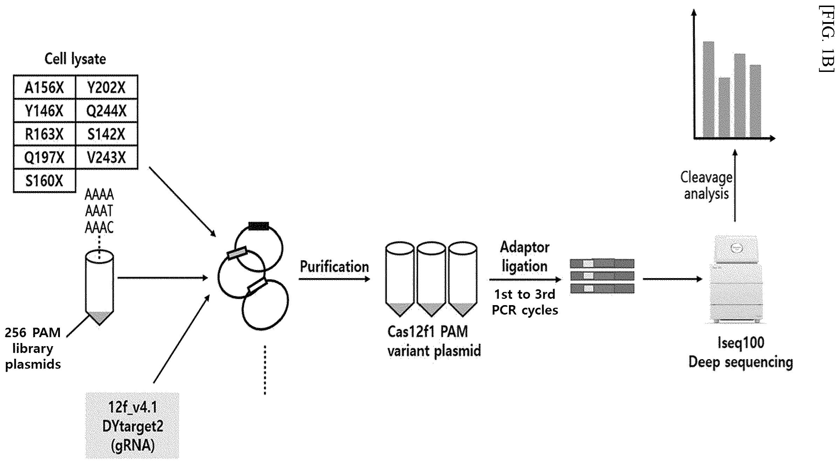

FIG. 1 illustrates flow charts showing construction of a PAM library for development of PAM variants of CWCas12f (FIG. 1A) and cleavage analysis of PAM variants of CWCas12f (FIG. 1B).

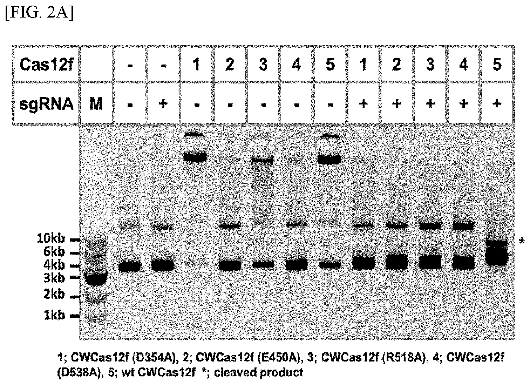

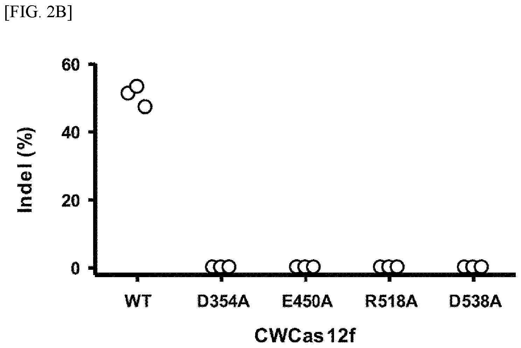

FIG. 2 illustrates results obtained by identifying configuration of catalytically inactive CWCas12fs (dCWCas12fs, dTnpBs) and indel-null activity thereof in HEK293T cells. FIG. 2A illustrates agarose gel images showing cleavage pattern of a plasmid vector caused by wild-type CWCas12f or various dCWCas12fs. Here, M represents a molecular ladder. The images are representative of three independent experiments. FIG. 2B illustrates a graph obtained by identifying indel efficiency of CWCas12f or dCWCas12fs at the *NLRC4* locus in HEK293T cells (n = 3 independent experiments).

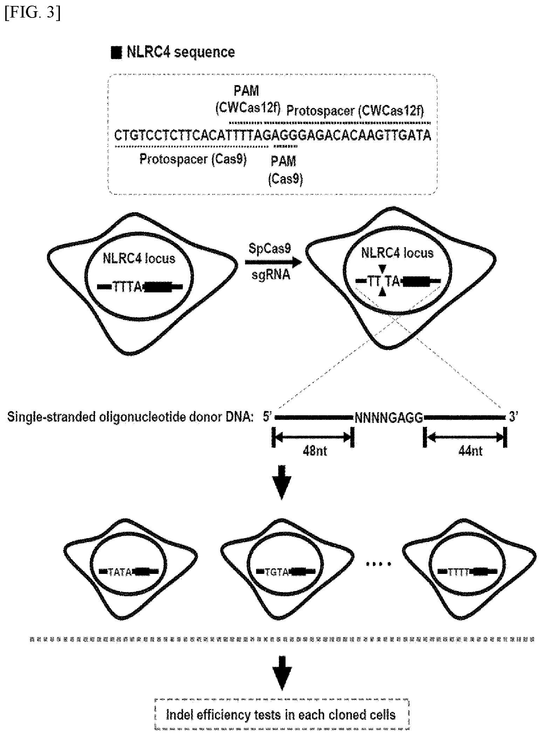

FIG. 3 illustrates a process of preparing HEK293T clones with different PAM sequences at the *NLRC4* locus by homology-directed repair using CRISPR-Cas9 system.

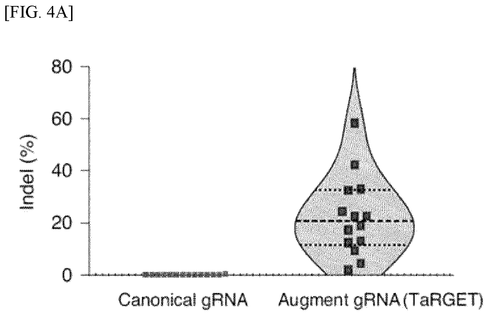

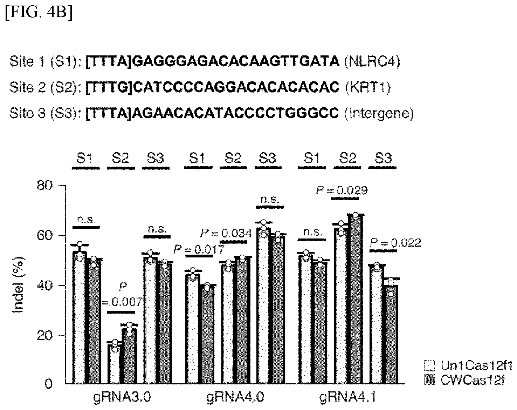

FIG. 4A illustrates a violin plot for indel efficiency of CWCas12f guided by naturally occurring or engineered gRNAs (n = 14 sites). FIG. 4B illustrates sequence information on the sites used to compare indel formation activity of Un1Cas12f1 and CWCas12f, and results obtained by comparing indel efficiency between Un1Cas12f1 and CWCas12f in the presence of various versions of engineered gRNA (mean ± standard deviation, n = 3 independent experiments). P values were derived by a two-tailed Student's t-test. ns, not significant.

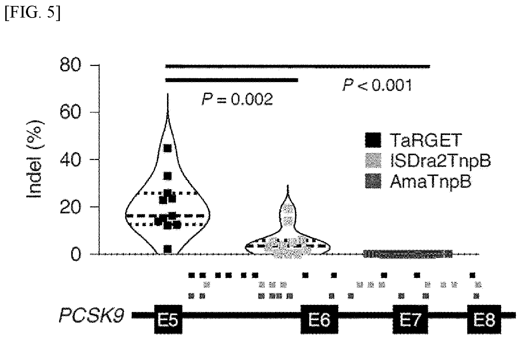

FIG. 5 illustrates a violin plot, suggesting that the TaRGET system provides a favorable scaffold for adenine base editors due to its high indel efficiency compared to other RNA-guided TnpB systems. Square dots represent target sites within the *PCSK9* gene. P values were derived by a two-tailed Welch t-test (TaRGET vs. ISDra2TnpB) and a two-tailed Mann-Whitney rank-sum test (TaRGET vs. AmaTnpB). n = 11 sites.

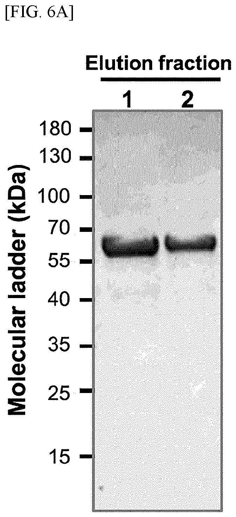

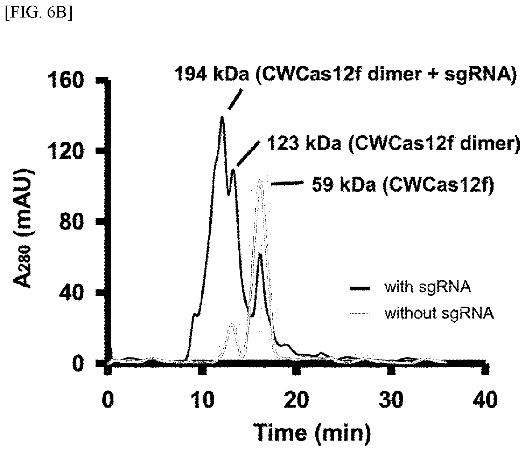

FIG. 6 illustrates dimerization of CWCas12f in the presence of single-stranded guide RNA. FIG. 6A illustrates an SDS-PAGE gel image for purified CWCas12f elution fractions, and FIG. 6B illustrates a size exclusion chromatography profile of CWCas12f protein in the presence or absence of sgRNA.

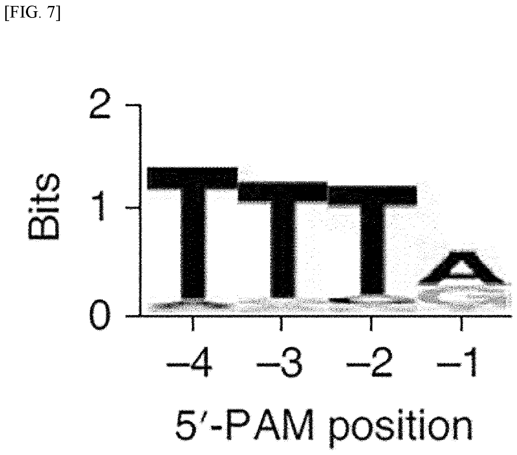

FIG. 7 illustrates results obtained by analyzing sequence logo for PAM preference of the wild-type CWCas12f.

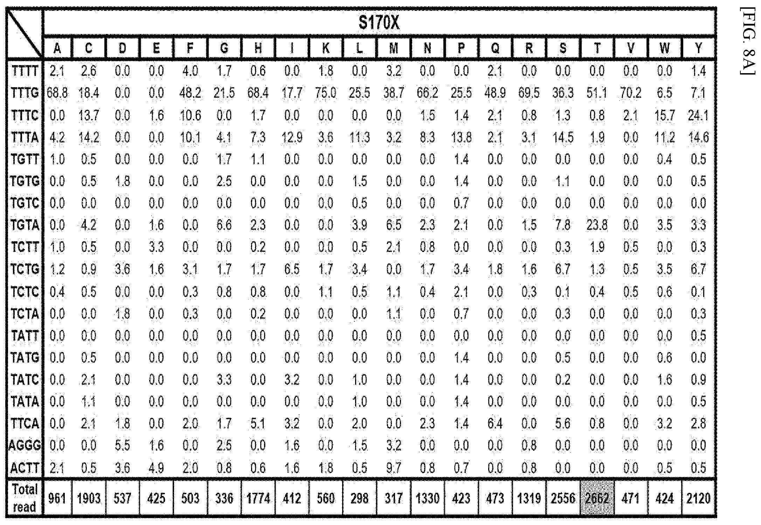

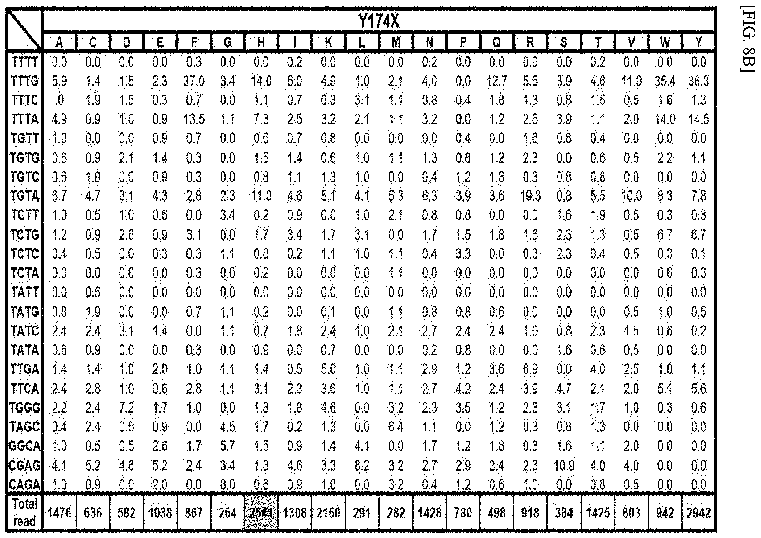

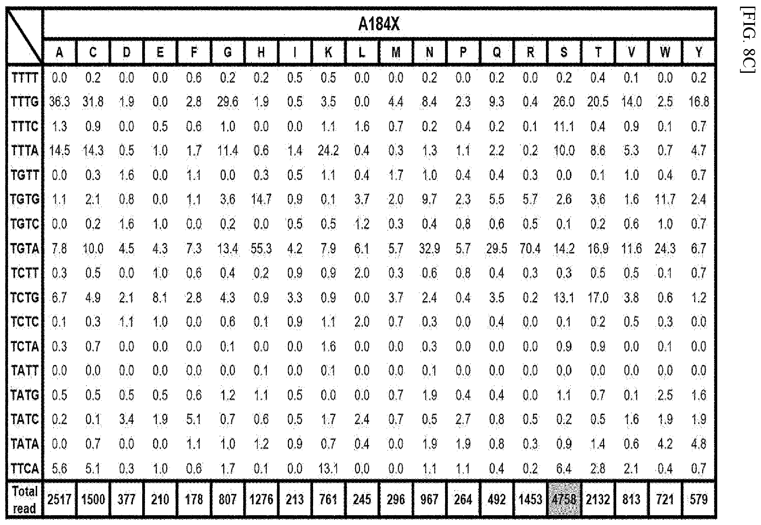

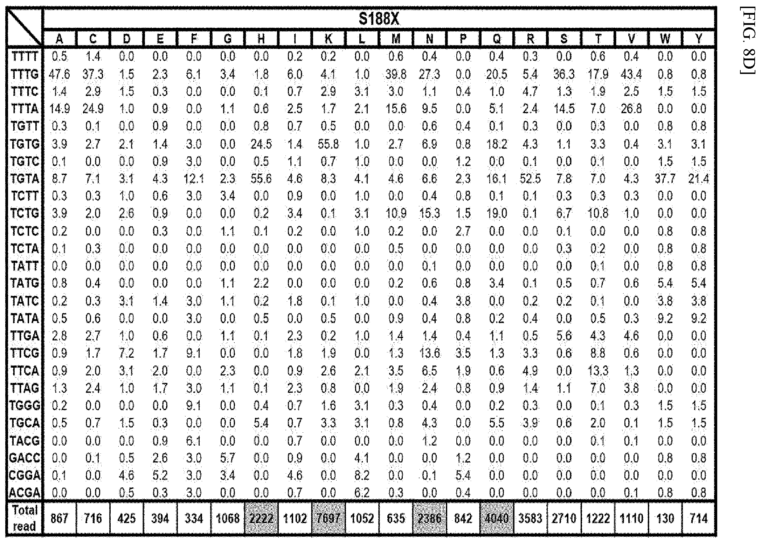

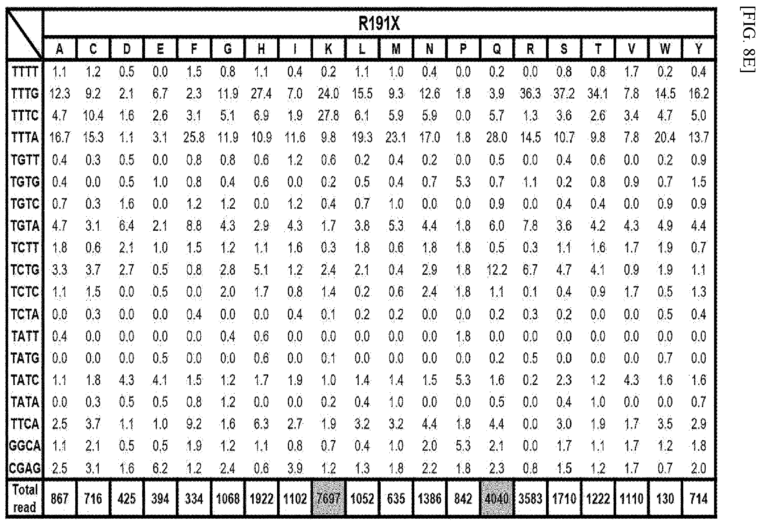

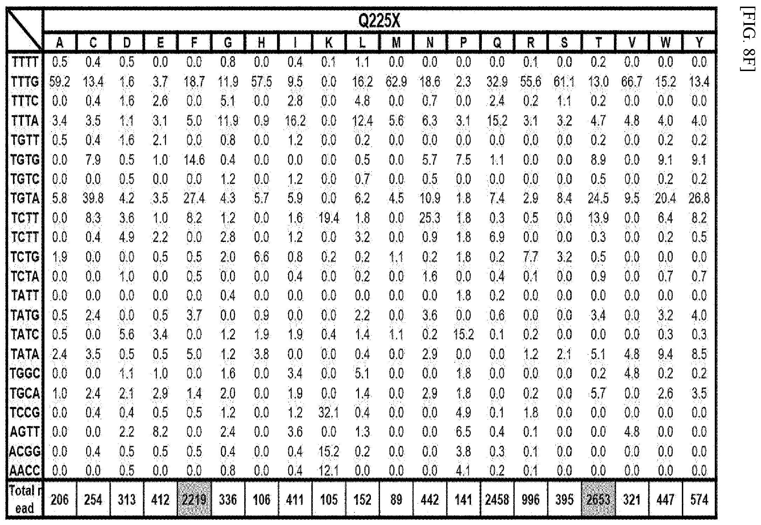

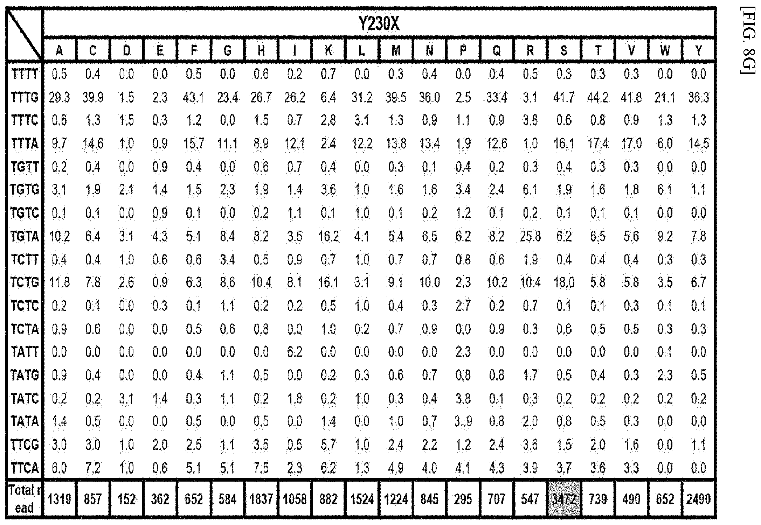

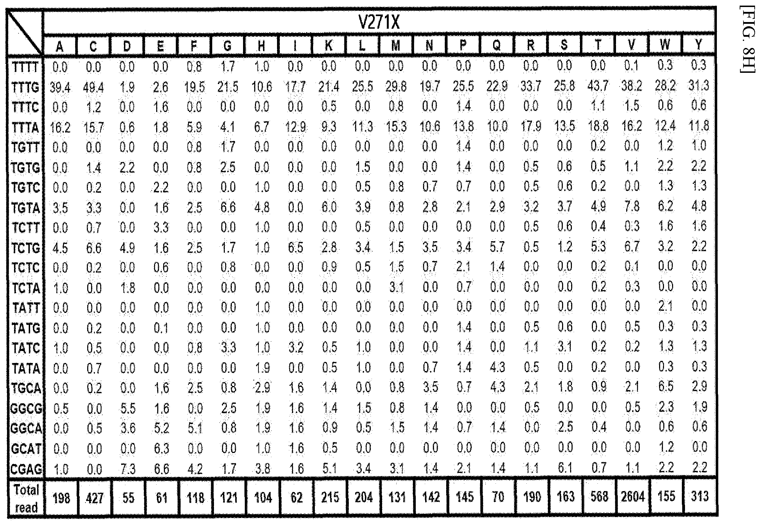

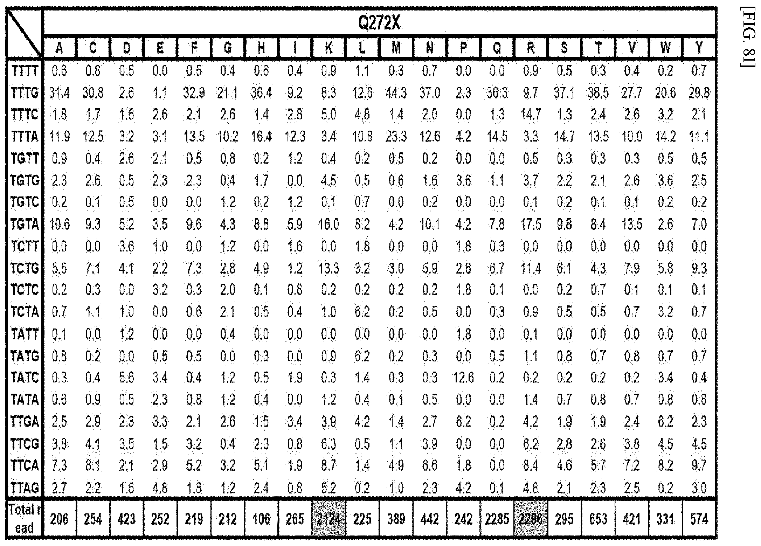

FIG. 8 illustrates results obtained by screening PAM variant candidates by *in vitro* DNA cleavage analysis: FIG. 8A (S170X), FIG. 8B (Y174X), FIG. 8C (A184X), FIG. 8D (S188X), FIG. 8E (R191X), FIG. 8F (Q225X), FIG. 8G (Y230X), FIG. 8H (V271X), and FIG. 8I (Q272X).

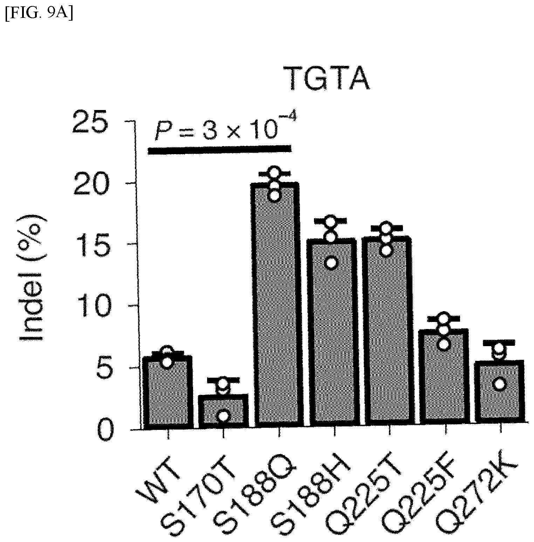

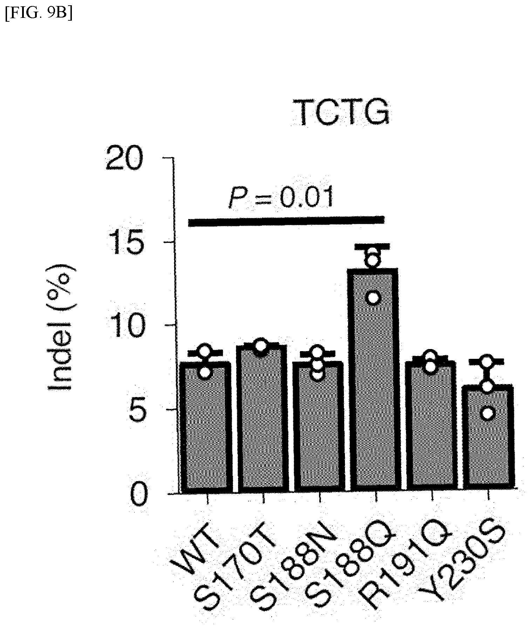

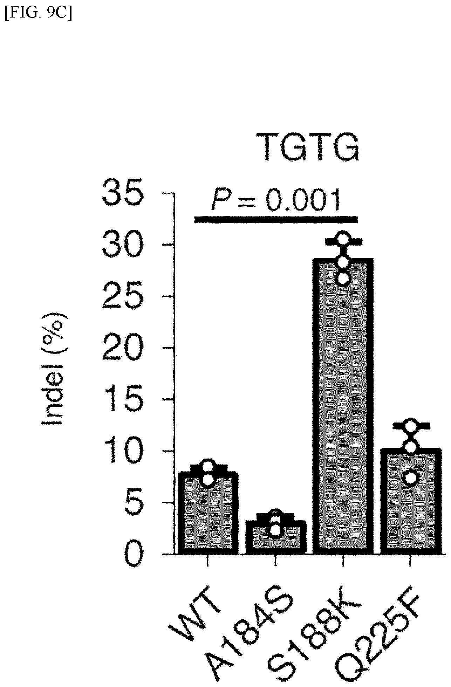

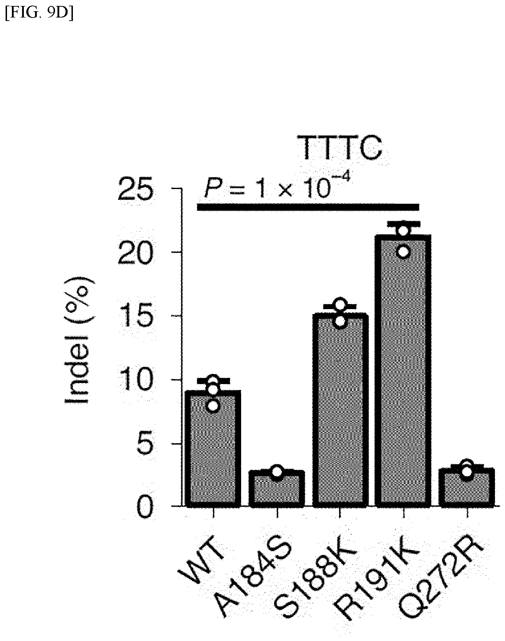

FIG. 9 illustrates results obtained by identifying indel formation activity of PAM variants for other PAM sequences than the canonical TTTR sequence (mean ± standard deviation, n = 3 independent experiments): TGTA (FIG. 9A), TCTG (FIG. 9B), TGTG (FIG. 9C), and TTTC (FIG. 9D). P values were derived by a two-tailed Student's t-test.

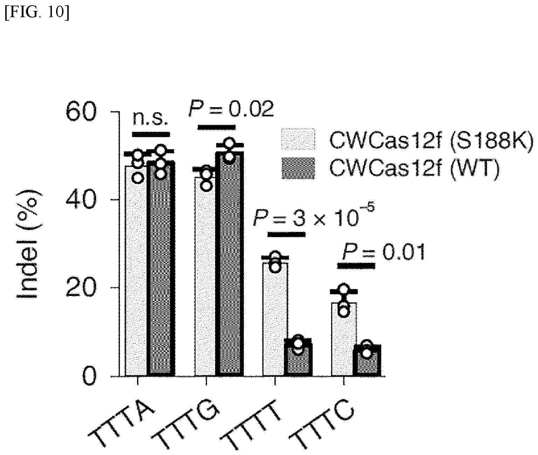

FIG. 10 illustrates a graph showing preference of S188K CWCas12f variant for the expanded PAM sequence TTTN (mean ± standard deviation, n = 3 independent experiments). P values were derived by a two-tailed Student's t-test.

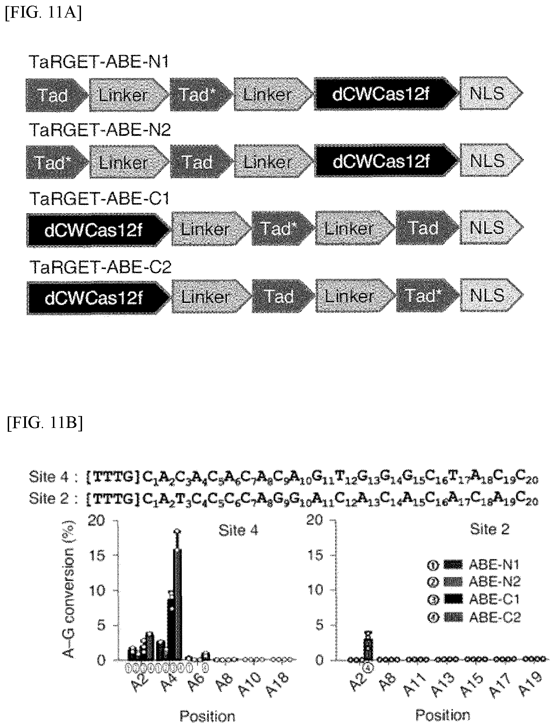

FIG. 11A illustrates a schematic diagram showing the structure and nomenclature of TaRGET-ABE modules depending on the orientation and order of wild-type Tad and mutant Tad (Tad*). FIG. 11B illustrates A to G conversion efficiency of TaRGET-ABE modules for two sites (sites 4 and 2) in HEK293T cells.

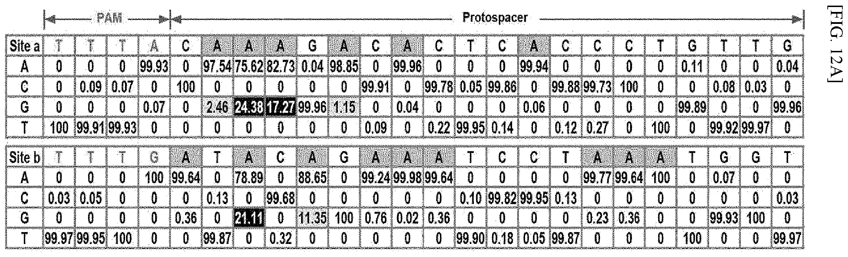

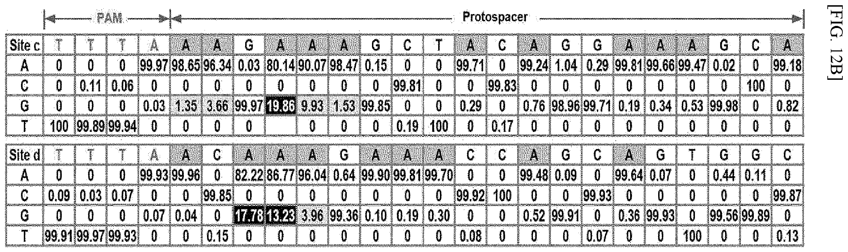

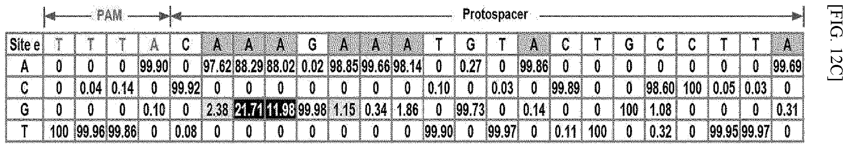

FIGS. 12A to 12C illustrate results obtained by identifying the base editing window of the TaRGET-ABE-C2 system.

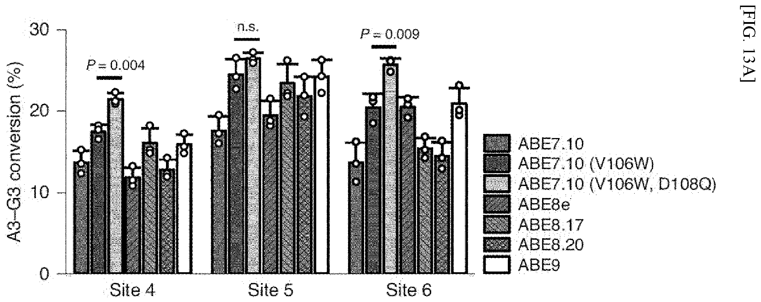

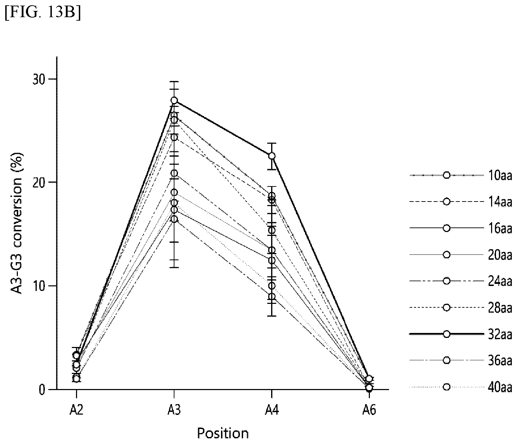

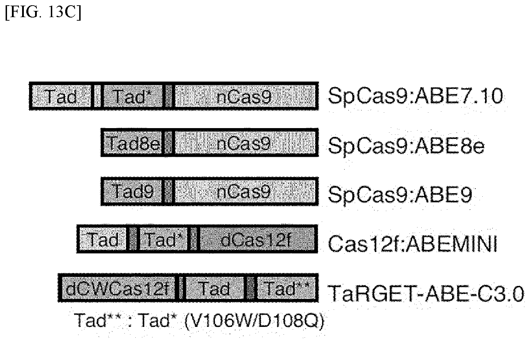

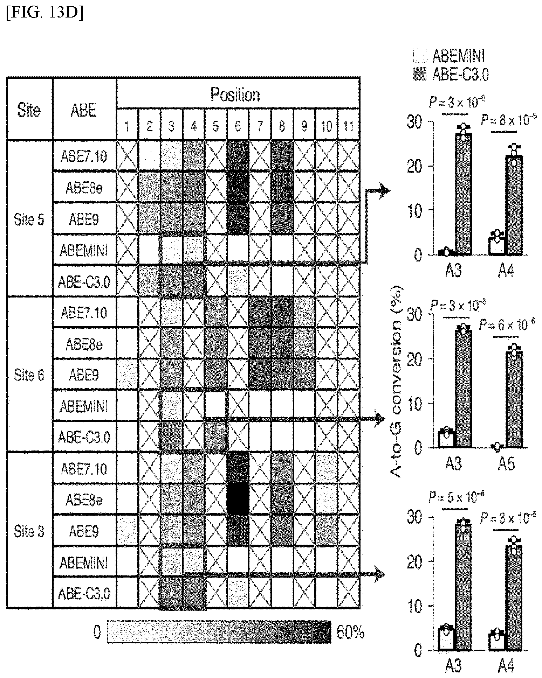

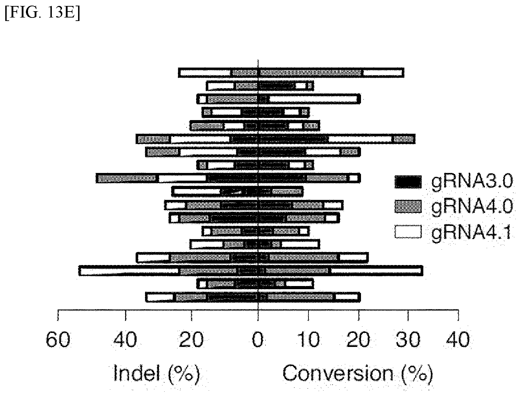

FIG. 13A illustrates conversion efficiency of various Tad modules in an engineered monomeric or heterodimeric form fused to the C-terminus of dCWCas12f (D354A). FIG. 13B illustrates identification of the optimized length of the linker used to connect dCWCas12f and Tad. FIG. 13C illustrates the architecture of SpCas9-, Un1Cas12f1-, and CWCas12f-based adenine base editors, wherein Tad** represents an engineered form of the Tad* protein with V106W and D108Q mutations. FIG. 13D illustrates heatmaps for A to G conversion efficiency at three different sites regarding SpCas9-, Un1Cas12f1-, and CWCas12f-based adenine base editors. Here, X represents a non-A sequence. In the right panel, a comparison was made between Cas12f-based ABEMINI and TaRGET-ABE-C3.0 in terms of conversion efficiency (mean ± standard deviation, n = 3 independent experiments). FIG. 13E illustrates dependency of indel efficiency and A to G conversion efficiency on the type of engineered sgRNA. n = 18 sites. P values were derived by a two-tailed Student's t-test.

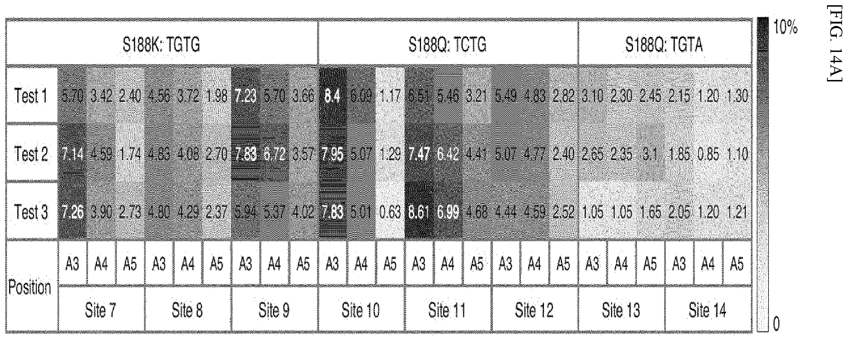

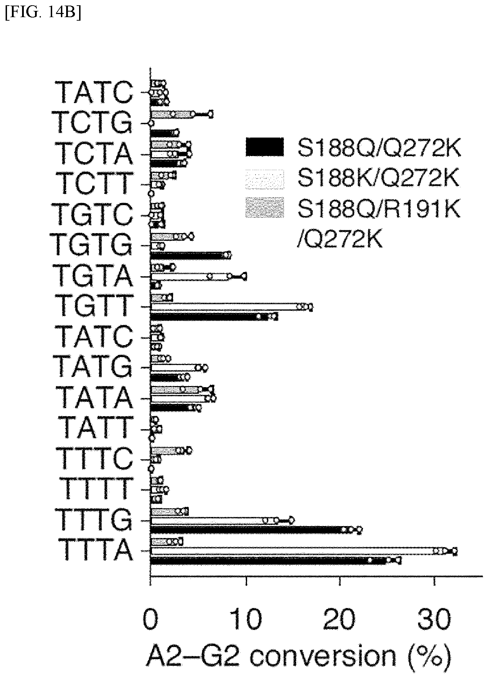

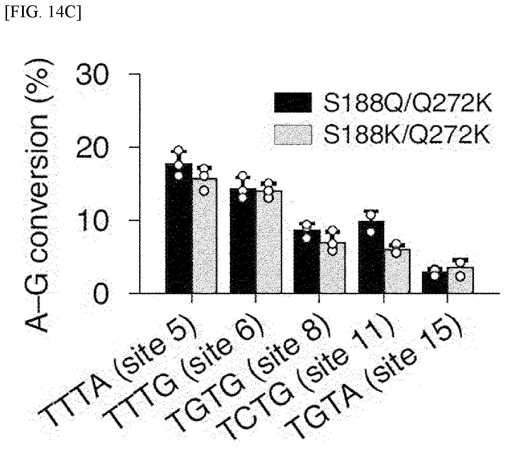

FIG. 14A illustrates heatmaps showing A to G conversion efficiency of CWCas12f PAM variants at non-TTTR PAM sites. FIG. 14B illustrates further expansion of targetable sites by stacked PAM mutations (S188Q/Q272K, S188K/Q272K, and S188Q/R191K/Q272K). FIG. 14C illustrates multiplexed adenine base editing at sites with various PAM sequences by S188Q/Q272K and S188K/Q272K PAM variants of CWCas12f (mean ± standard deviation, n = 3 independent experiments).

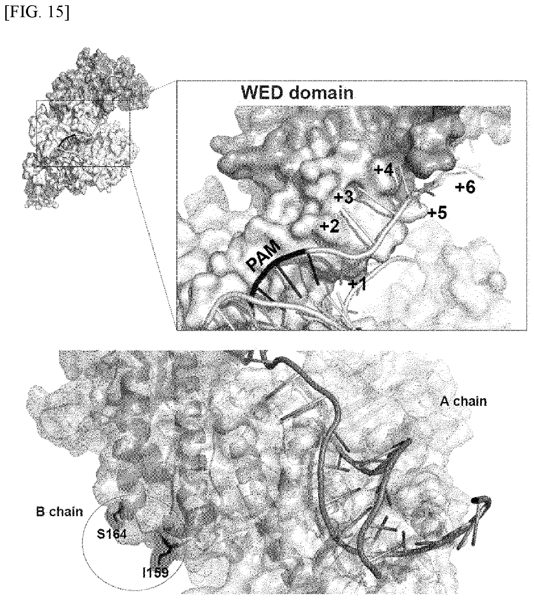

FIG. 15 illustrates structural modeling of CWCas12f bound to a PAM-containing protospacer. The positions of S164 and I159 are indicated on chain B.

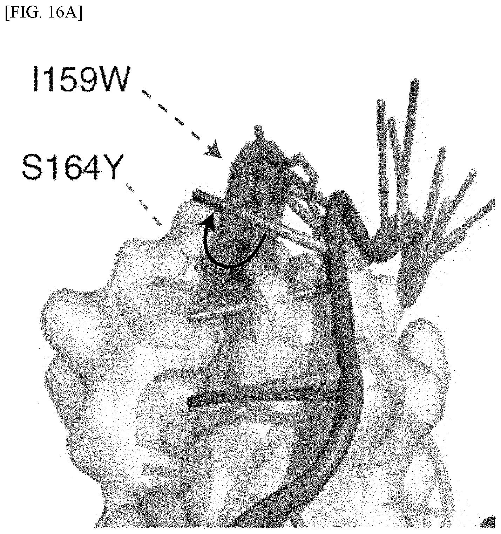

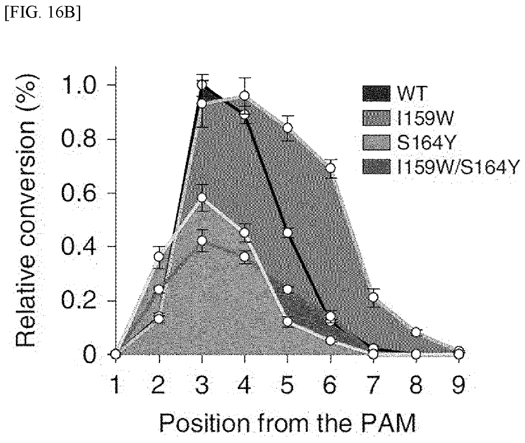

FIG. 16 illustrates expansion of the base editing window by CWCas12f mutations (I159W and/or S164Y). FIG. 16A illustrates amino acid substitution modeling to shift or alter the editing window in the PAM-proximal region. FIG. 16B illustrates results obtained by identifying expansion of the base editing window achieved in a case where Ile 159 is substituted with tryptophan. The relative values were obtained by calculating conversion efficiency at each position for three different sites (mean ± standard deviation, n = 3 independent experiments).

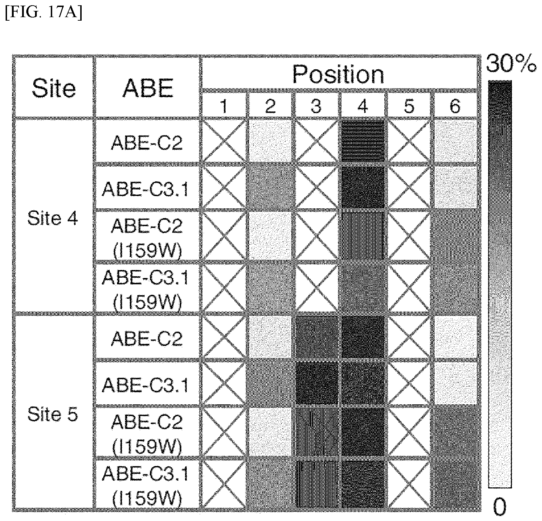

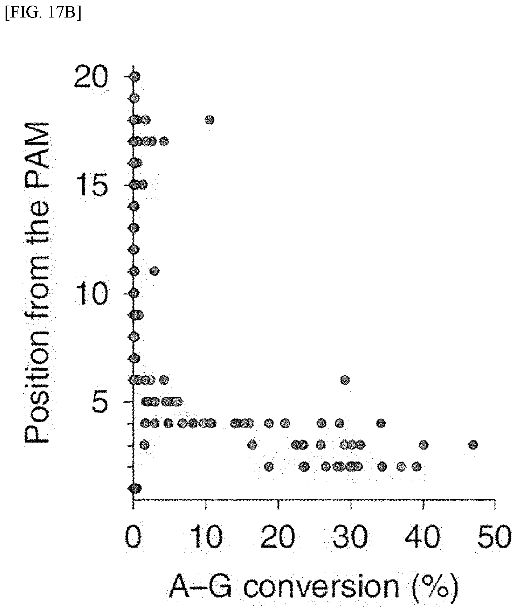

FIG. 17A illustrates results obtained by identifying expansion of the base editing window depending on various structures of the deaminase module. Each value is an average of three experiments. FIG. 17B illustrates results obtained by identifying A to G conversion efficiency of TaRGET-ABE-C3.1 for a total of 25 endogenous loci. Each value is an average of three experiments.

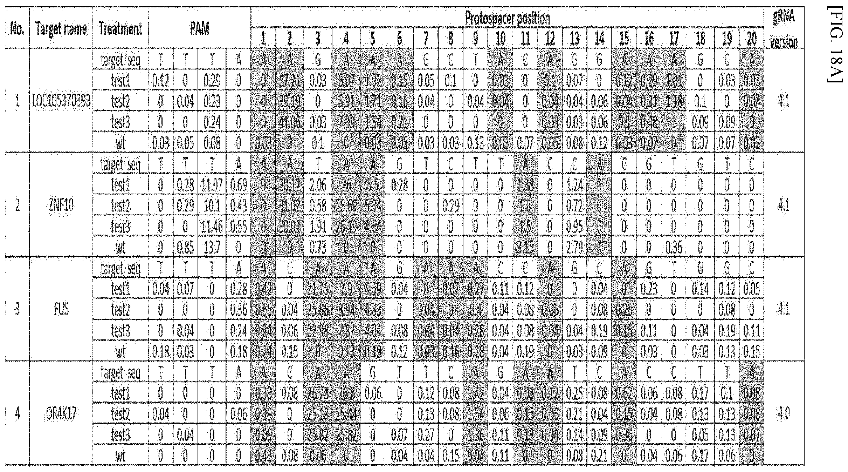

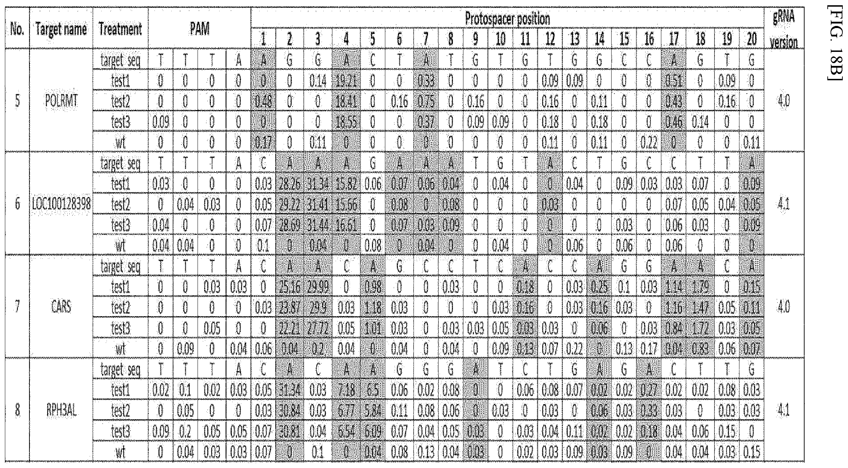

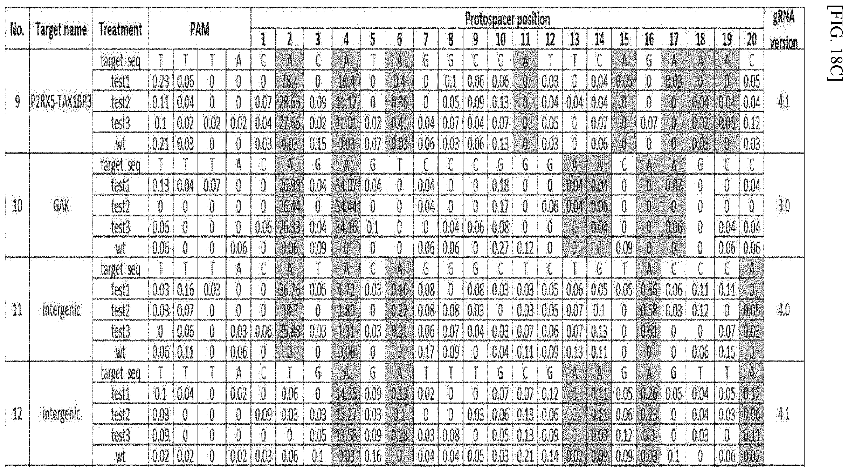

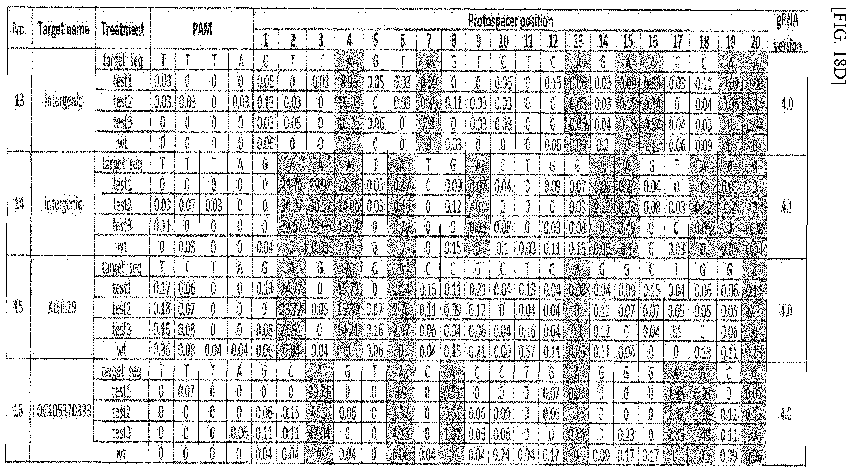

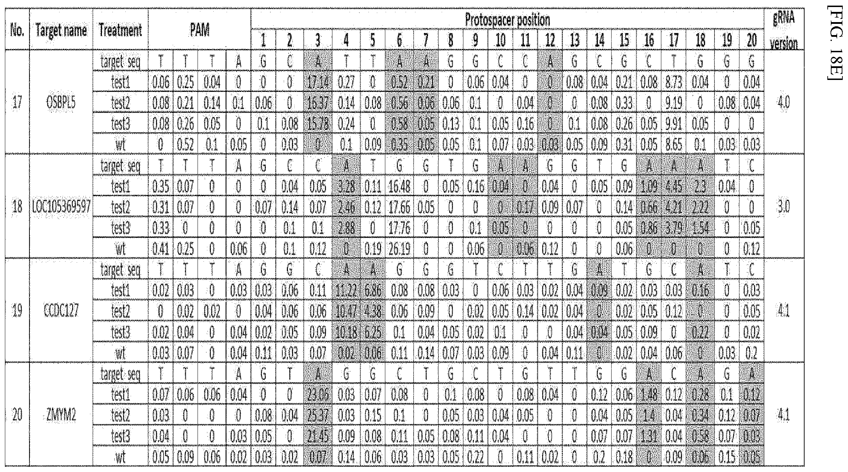

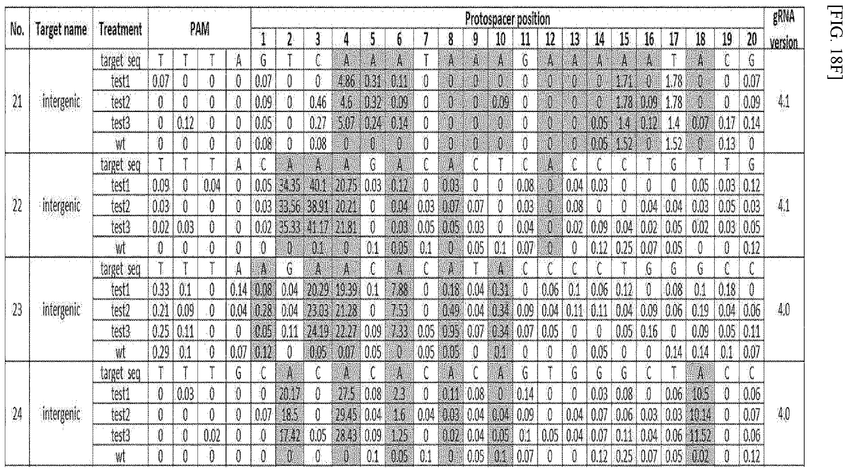

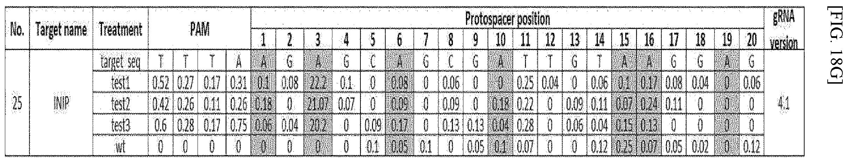

FIG. 18 illustrates results obtained by identifying A to G conversion activity of TaRGET-ABE-C3.1 at 25 multiple sites used in FIG. 17B: FIG. 18A (conversion efficiency of target genes indicated as nos. 1 to 4), FIG. 18B (conversion efficiency of target genes indicated as nos. 5 to 8), FIG. 18C (conversion efficiency of target genes indicated as nos. 9 to 12), FIG. 18D (conversion efficiency of target genes indicated as nos. 13 to 16), FIG. 18E (conversion efficiency of target genes indicated as nos. 17 to 20), FIG. 18F (conversion efficiency of target genes indicated as nos. 21 to 24), and FIG. 18G (conversion efficiency of target gene indicated as no. 25).

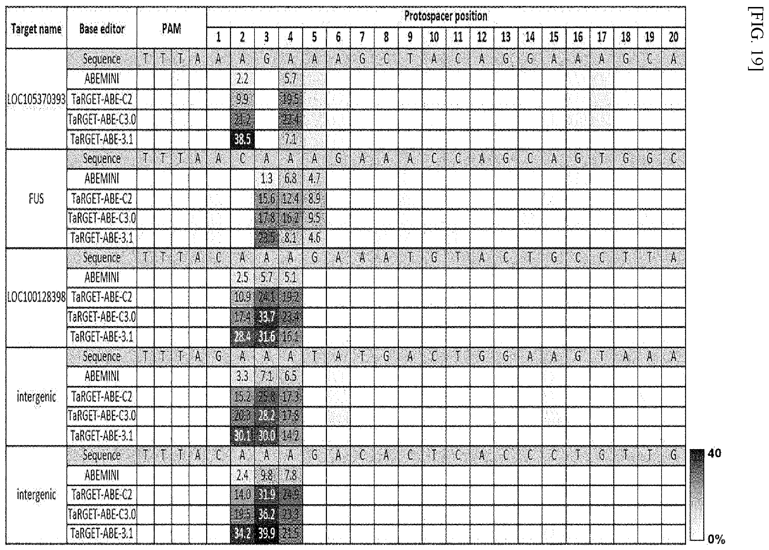

FIG. 19 illustrates results obtained by making a one-to-one comparison of A to G conversion efficiency between TaRGET-based adenine base editors (TaRGET-ABE-C2, TaRGET-ABE-C3.0, and TaRGET-ABE-C3.1) and Un1Cas12f-based ABEMINI. The intensity of the color represents conversion efficiency. Each value represents an average of three independent experiments.

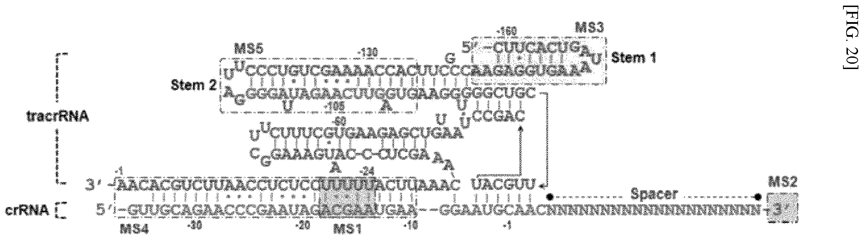

FIG. 20 illustrates modification sites in the engineered guide RNA of the system according to an embodiment.

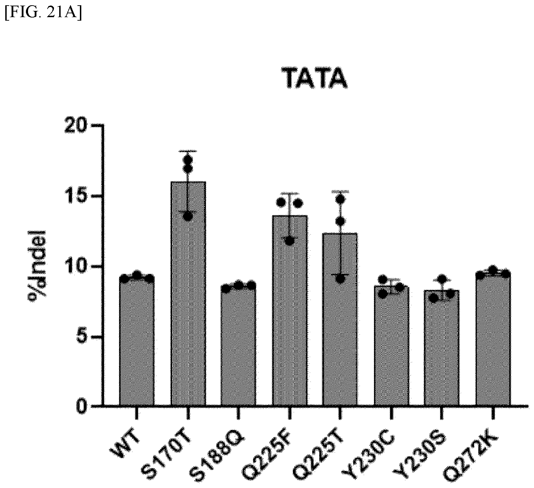

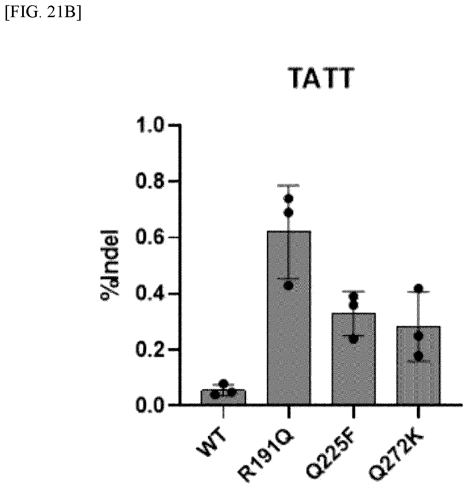

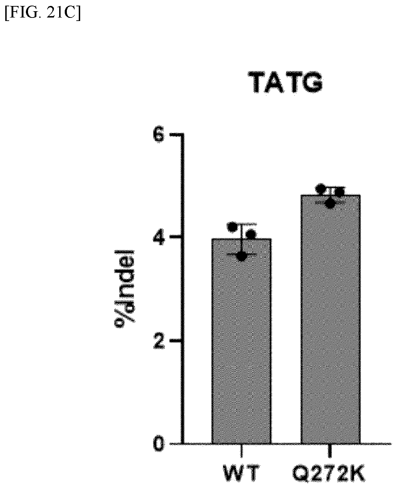

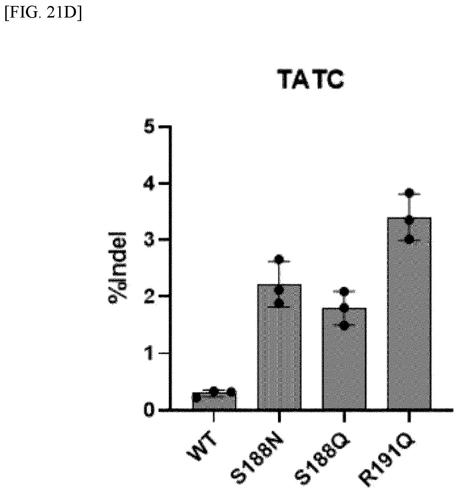

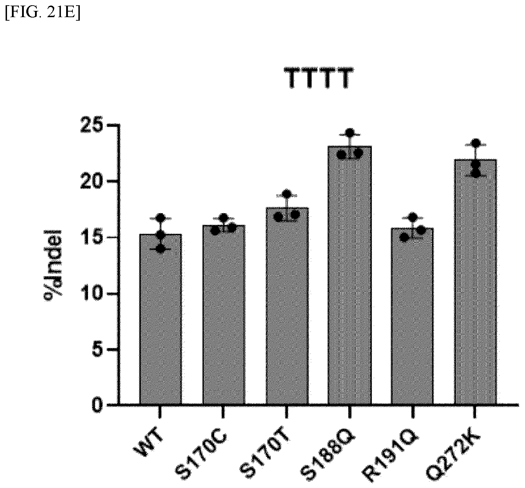

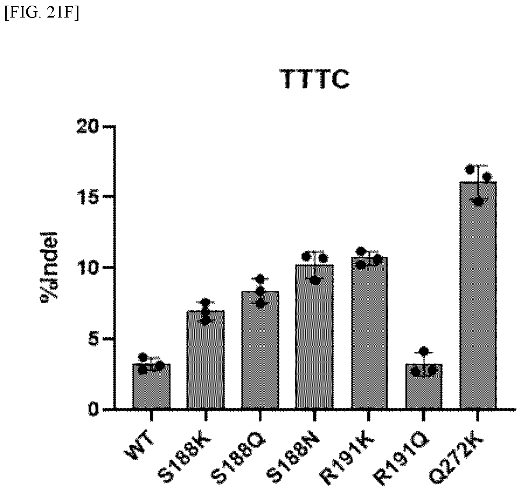

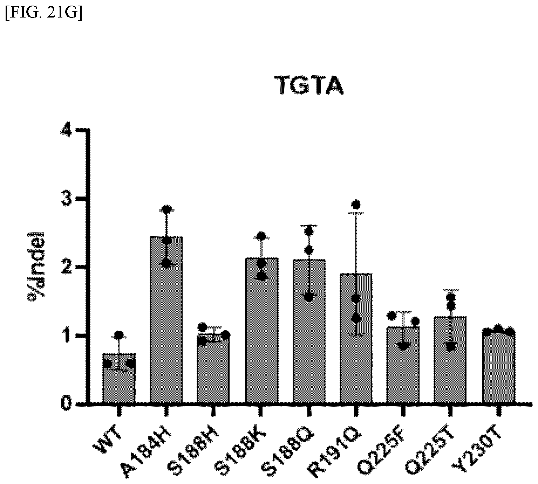

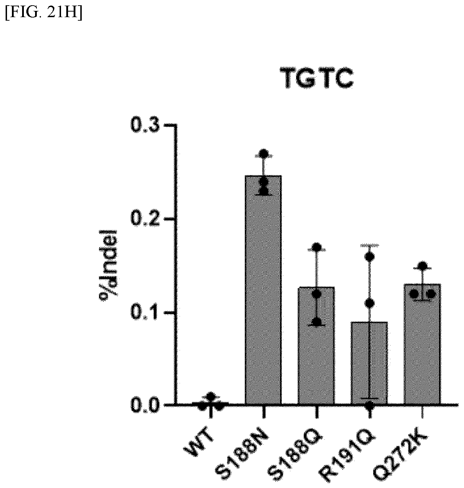

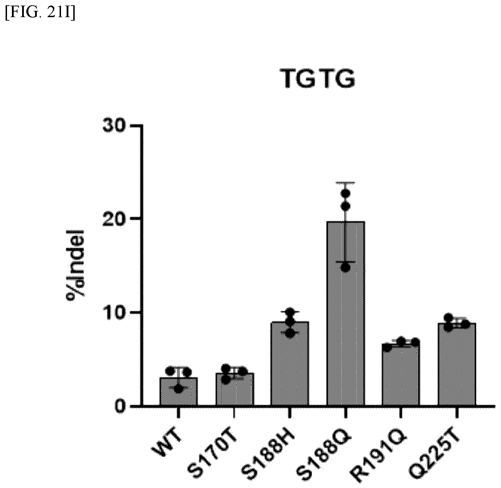

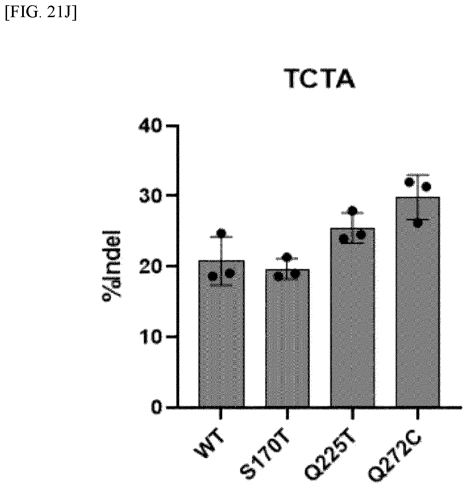

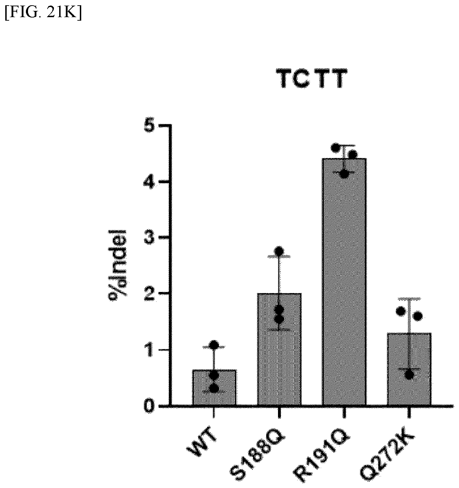

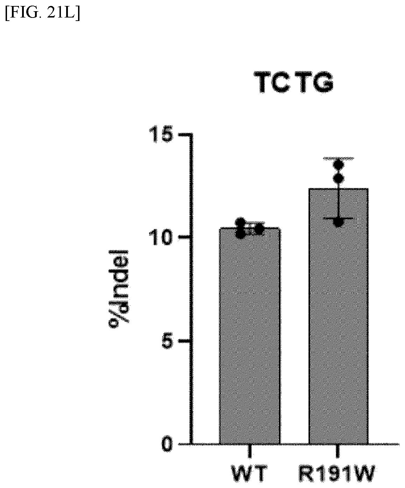

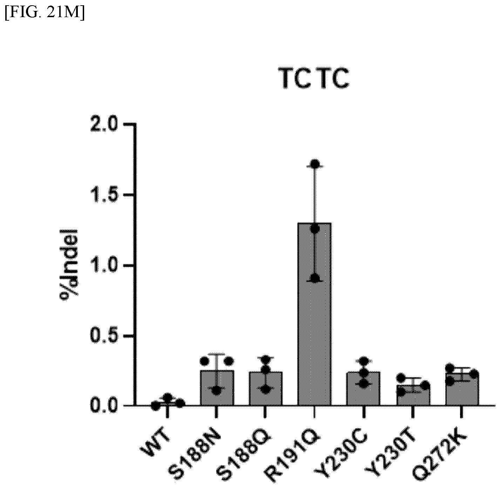

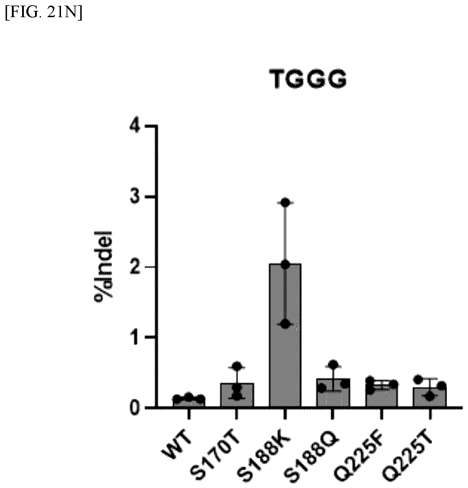

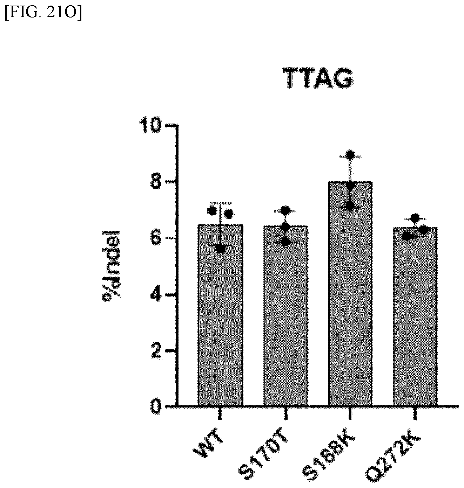

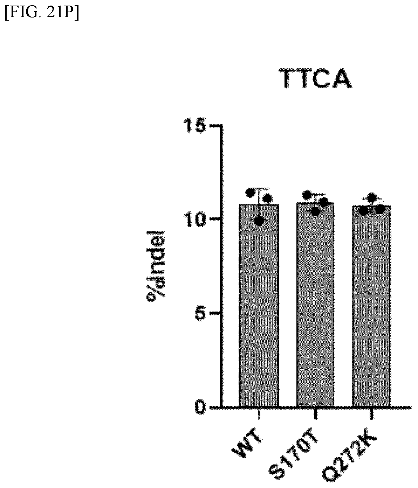

FIGS. 21A to 21P illustrate results obtained by identifying indel formation activity of PAM variants for PAM sequences other than the canonical TTTR sequence in a eukaryotic cell line (mean ± standard deviation, n = 3 independent experiments).

### Modes for Carrying out Invention

The detailed description of the present disclosure set forth below will be described with reference to specific drawings (only if relevant drawings are involved) with respect to specific embodiments in which the present disclosure may be practiced; however, the present disclosure is not limited thereto and is limited only by the appended claims with respect to the full scope equivalent to what is set forth in the claims. It should be understood that various embodiments/examples of the present disclosure, although different, are not necessarily mutually exclusive. For example, a particular feature, structure, or characteristic described herein may be changed from one embodiment/example to another embodiment/example or implemented in combinations of embodiments/examples without departing from the technical spirit and scope of the present disclosure. Unless defined otherwise, technical and scientific terms used herein have the same meaning as generally used in the art to which the present disclosure belongs. For purposes of interpreting this specification, the following definitions will apply, and any term expressed in a singular form should, where appropriate, be interpreted as referring to a plural form (for example, "at least one"), and vice versa.

Hereinafter, in order to enable those skilled in the art to easily practice the present disclosure, various preferred embodiments/examples of the present disclosure will be described in detail with reference to the attached drawings (only if relevant drawings are involved).

### I. Definition

Throughout the specification, the terms as defined below are used. Other definitions are also found elsewhere in the specification.

As used herein, "about" means approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%.

The term A, T, C, G, and U may be appropriately interpreted as a base, a nucleoside, or a nucleotide in DNA or RNA, depending on the context. For example, when A, T, C, G, and U refer to a base, they may be interpreted as adenine (A), guanine (G), cytosine (C), thymine (T), or uracil (U), respectively. When A, T, C, G and U refer to a nucleoside, they may be interpreted as adenosine (A), thymidine (T), cytidine (C), guanosine (G), or uridine (U), respectively, and when they refer to a nucleotide in a sequence, they should be interpreted as meaning a nucleotide containing each of the nucleosides.

The terms "nucleic acid," "nucleic acid molecule," and "polynucleotide" as used herein are used interchangeably herein. They refer to polymers of deoxyribonucleotides or ribonucleotides, which is double-stranded or single-stranded form. Unless otherwise specified, it encompasses known analogues of natural nucleotides that can function in a similar manner to naturally occurring nucleotides. The term encompasses nucleic acid-like structures having synthetic backbones, as well as amplicons. Both DNA and RNA are polynucleotides. The polymer may comprise natural nucleosides (that is, adenosine, thymidine, guanosine, cytidine, uridine, deoxyadenosine, deoxythymidine, deoxyguanosine, and deoxycytidine), nucleoside analogues (for example, 2-aminoadenosine, 2-thiothymidine, inosine, pyrrolo-pyrimidine, 3-methyl adenosine, C5-propynylcytidine, C5-propynyluridine, C5-bromouridine, C5-fluorouridine, C5-iodouridine, C5-methylcytidine, 7-deazaadenosine, 7-deazaguanosine, 8-oxoadenosine, 8-oxoguanosine, O(6)-methylguanine, and 2-thiocytidine), chemically modified bases, biologically modified bases (for example, methylated bases), intercalated bases, modified sugars (for example, 2'-fluororibose, ribose, 2'-deoxyribose, arabinose, and hexose), or modified phosphate groups (for example, phosphorothioate and 5'-N-phosphoramidite linkage).

The term "amino acid" collectively refers to the 20 types of amino acids that are synthesized through the transcription and translation of genes in an organism's body. Specifically, the amino acids comprise alanine (Ala, A), arginine (Arg, R), asparagine (Asn, N), aspartic acid (Asp, D), cysteine (Cys, C), glutamic acid (Glu, E), glutamine (Gln, Q), glycine (Gly, G), histidine (His, H), isoleucine (Ile, I), leucine (Leu, L), lysine (Lys K), methionine (Met, M), phenylalanine (Phe, F), proline (Pro, P), serine (Ser, S), threonine (Thr, T), tryptophan (Trp, W), tyrosine (Tyr, Y), and valine (Val, V). Each of these amino acids has a corresponding DNA codon. The term amino acid generally refers to a standard amino acid that occurs naturally, but may include non-naturally occurring amino acids, artificial amino acids, modified amino acids, and the like.

The term "identity" as used herein refers to the overall relatedness between polymer molecules, for example, between nucleic acid molecules (for example, DNA molecules and/or RNA molecules) and/or between polypeptide molecules. In some embodiments, polymer molecules may be considered "substantially identical" to one another if their sequences have at least 50%, 55%, 60%, 65%, 70%, 75%, 80%, 85%, 90%, 95%, or 99% identity. Calculating the percent identity between two nucleic acid or polypeptide sequences may be performed, for example, by aligning the two sequences for optimal comparison purposes (for example, gaps may be introduced into one or both of the first and second sequences for optimal alignment, and non-identical sequences may be disregarded for comparison purposes). In some embodiments, the length of a sequence aligned for comparison purposes is at least 50%, at least 60%, at least 70%, at least 80%, at least 90%, at least 95%, or substantially 100% of the length of a reference sequence. Comparison of sequences and determination of percent identity between two sequences may be accomplished using a mathematical algorithm. As is well known to those skill in the art, amino acid or nucleotide sequences may be compared using any of a variety of algorithms available from commercial computer programs, such as BLASTN for nucleotide sequences, and BLASTP, gapped BLAST, and PSI-BLAST for amino acid sequences.

As used herein, "conservative substitution" refers to substitution of an amino acid for an amino acid having similar functional or physical/chemical properties, and conservative substitution of amino acids is well known in the relevant art. For example, the conservative substitution may involve substitution of amino acids within the following groups: i) methionine, isoleucine, leucine, valine, ii) phenylalanine, tyrosine, tryptophan, iii) lysine, arginine, histidine, iv) alanine, glycine, v) serine, threonine, vi) glutamine, asparagine, and vii) glutamic acid, aspartic acid. In some embodiments, conservative amino acid substitution refers to amino acid substitution that does not alter relative charge or size characteristics of a protein in which the amino acid substitution has occurred.

The term "fusion protein" refers to a protein formed by joining two or more originally distinct proteins, or portions thereof. In some embodiments, a linker or spacer will be present between the respective proteins. Specifically, the linker may be a peptide linker comprising from 2 to 20 amino acids. The linker may be, for example, GGGS, GGGGS, or two, three, four or more repeats thereof.

The term "CRISPR/Cas system" is used interchangeably with "gene editing system." The CRISPR/Cas system refers to a system capable of interact with a target gene or a target nucleic acid and/or altering (for example, cleaving, editing, repairing, and/or restoring) a target site, wherein the system comprises a Cas protein and/or components related to a nucleic acid-targeting molecule (for example, a guide RNA). The CRISPR/Cas system may exist in any form that is capable of interacting with and/or altering a target site, for example, may be in the form of a composition comprising a complex that comprises a Cas protein and a guide RNA, or may be in the form of a kit in which a Cas protein and a guide RNA are each included in a separate composition. As another example, the system may be in the form of a vector system or composition, comprising at least one vector that comprises a nucleotide sequence encoding a Cas protein and a nucleotide sequence encoding a guide RNA.

The term "target site" as used herein refers to a nucleotide sequence that defines a portion of a nucleic acid to which a binding molecule will bind, if conditions sufficient for binding exist. In some embodiments, the target site is a nucleotide sequence to which the Cas protein described herein binds and/or is altered by such a Cas protein. In some embodiments, the target site is a nucleotide sequence to which the guide RNA described herein binds. The target site may be single-stranded or double-stranded. In the context of RNA-guided (for example, RNA-programmable) nucleases (for example, Cas protein), the target site may typically comprise a nucleotide sequence (target sequence, present on the target strand) complementary to a guide sequence of a guide RNA, a protospacer sequence complementary to the nucleotide sequence, and a protospacer adjacent motif (PAM) (present on the non-target strand) at the 3'-end or the 5'-end adjacent thereto. The protospacer sequence is a sequence located at the 5'-end or 3'-end of the PAM sequence, and is complementary to the target sequence or forms a complementary bond with the target sequence. The relationship between the protospacer sequence and the target sequence is similar to the relationship between the target sequence and the guide sequence. Due to these characteristics, the guide sequence may usually be designed using the protospacer sequence.

The term "guide RNA (gRNA)" refers to RNA that is capable of forming a complex with a molecule usually referred to as a Cas protein, and interacting with (for example, hybridizing to, forming a complementary bond(s) with, or forming a hydrogen bond(s) with) a target nucleotide sequence, and comprises a guide sequence having sufficient complementarity with the target nucleotide sequence to cause sequence-specific binding of the complex to the target nucleotide sequence. In this specification, the terms guide RNA or guide molecule may be used interchangeably. The guide RNA typically comprises a scaffold region and a spacer region comprising a guide sequence.

The term "guide sequence" may be used interchangeably with "spacer" or "spacer sequence," and refers to a polynucleotide within the CRISPR/Cas system which is capable of interacting with (for example, hybridizing to, forming a complementary bond(s) with, or forming a hydrogen bond(s) with) a target sequence portion. For example, the guide sequence refers to 10 to 50 consecutive nucleotides linked directly or indirectly through a linker or the like to or near the 3'-end of crRNA, which constitutes a guide RNA, in a gene editing system.

The term "scaffold region" refers to a portion of a guide RNA that may interact with a molecule referred to as a gene editing protein (for example, a Cas protein), and may be used to refer to the remaining portion of a guide RNA found in nature, excluding a spacer.

The term "stem" refers to a nucleic acid region having a secondary structure that comprises a nucleotide region capable of forming a double strand. A configuration in which a double strand is connected primarily by a region of single-stranded nucleotides (a loop region) is referred to as a "stem-loop." The terms "stem" and "stem-loop" may be used interchangeably and should be interpreted appropriately depending on the context.

The term "engineered" may be used to distinguish it from a substance or molecule having a naturally occurring configuration in nature, and means that an artificial modification has been applied to the substance or molecule. For example, an "engineered Cas protein" means a Cas protein obtained by applying an artificial modification to the configuration (for example, amino acid sequence) of a Cas protein existing in nature, and may also be referred to herein as a "variant" or "mutant." For example, an "engineered guide RNA" means a gRNA obtained by applying an artificial modification to the configuration (for example, sequence) of a guide RNA (gRNA) existing in nature, and may also be referred to herein as an "augmented RNA."

The term "variant" means an entity that shows significant structural identity with a reference entity (for example, a wild-type sequence) but differs structurally from the reference entity in the presence or level of one or more chemical moieties as compared to the reference entity. In some embodiments, a variant may differ functionally from its reference entity. In general, whether a particular entity is appropriately considered to be a "variant" of a reference entity is based on its degree of structural identity with the reference entity. For example, for a protein or polypeptide, a polypeptide of interest is considered to be a "variant" of a parent or reference polypeptide if the polypeptide of interest has an amino acid sequence that is identical to that of the parent but for a small number of sequence alterations at particular positions. In some embodiments, fewer than 50%, 40%, 30%, 20%, 15%, 10%, 9%, 8%, 7%, 6%, 5%, 4%, 3%, or 2% of the residues in the variant are substituted as compared to the parent. In some embodiments, the parent or reference polypeptide is one found in nature.

The term "vector," unless otherwise specified, refers to any material capable of transporting a genetic material into a cell. For example, a vector may be a nucleic acid, typically a DNA molecule, comprising a genetic material of interest, for example, a nucleic acid encoding an effector protein (Cas protein) of a CRISPR/Cas system, and/or a nucleic acid encoding a guide RNA; however, the vector is not limited thereto. In addition, as used herein, the "vector" may be an "expression vector" comprising essential regulatory elements operably linked to enable the inserted gene to be expressed normally.

The term "operably linked" means a functional linkage of two or more components arranged in such a way that allows the described component to function in an intended manner. For example, when a promoter sequence is operably linked to a sequence encoding protein A, it means that the promoter is linked to the sequence encoding the protein A so as to transcribe and/or express the sequence encoding the protein A in a cell. In addition, the term includes all other meanings generally recognized by those skilled in the relevant art and may be appropriately interpreted depending on the context.

### II. Engineered Cas protein

The present disclosure relates to an engineered Cas protein, specifically an engineered Cas12 protein, and more specifically an engineered Cas12f protein, which has been engineered to have at least partially altered PAM recognition specificity or to modify an editing window of a PAM-proximal region. The present inventors have surprisingly found that Cas12f can be mutated to have altered PAM recognition. This means that the engineered Cas12f proteins (that is, Cas12f protein variants) as disclosed herein can recognize an expanded range of PAM sequences. The engineered Cas12f proteins as disclosed herein can recognize PAM sequences that are different from those recognized by the corresponding wild-type protein. In addition, the present inventors have found that even if a Cas protein has an expanded targetable range through PAM mutations, the protein may face editing limitations due to structural interference with, for example, functions of other functional domains, and have discovered that an editing window in the PAM-proximal region can be altered through amino acid substitutions at specific positions in Cas12f. Cas12f variants with an altered editing window for PAM-proximal regions are useful for inducing precise base substitutions, for example, in a form fused with another functional domain such as a base editing domain.

Accordingly, according to an aspect of the present disclosure, there is provided an engineered Cas protein, specifically an engineered Cas12 protein, and more specifically an engineered Cas12f protein, comprising at least one mutation. The engineered Cas protein as disclosed herein has a significantly expanded range of accessible target sites compared to the wild-type Cas12f protein.

The Cas12f protein, which belongs to class 2, type V, is one of the effector proteins named Cas14 in previous studies (see Harrington et al., Science, 362, 839-842, 2018), and is also referred to as Cas14a1 protein. It has been reported that a Cas12f protein may form a complex with a guide RNA in the form of a dimer of two Cas12f protein molecules, and that all or part of the domain in Cas12f protein recognizes a specific part in the scaffold region of Cas12f guide RNA to form the CRISPR/Cas12f1 complex (see Takeda et al., Structure of the miniature type V-F CRISPR-Cas effector enzyme, Molecular Cell 81, 1-13, 2021 and Xiao et al., Structural basis for the dimerization-dependent CRISPR-Cas12f nuclease, bioRxiv, 2020). In an embodiment, the Cas12f protein may be derived from a Cas14 family (see Harrington et al., Science 362, 839-842, 2018; and US 2020/0172886 A1, the entire contents of which are incorporated herein by reference). In another embodiment, the Cas12f protein may be a Cas14a1 or Un1Cas12f1 protein derived from an uncultured archaeon (see Harrington et al., Science 362, 839-842 (2018); and US 2020/0172886 A1, the entire contents of which are incorporated herein by reference). In an embodiment, the wild-type Cas12f protein (for example, Cas14a1 or Un1Cas12f1 protein) may comprise or consist of the amino acid sequence of SEQ ID NO: 5 (see Table 2). In another embodiment, the Cas12f protein may be TnpB (transposon-associated transposase B) protein derived from the *Candidatus Woesearchaeota* archaeon. The TnpB protein is a protein conventionally known as a transposase and was named CWCas12f due to its sequence similarity to the Un1Cas12f1 protein (TnpB and CWCas12f are used interchangeably herein). A guide RNA for the CWCas12f protein has also not been known. The present inventors have confirmed for the first time that CWCas12f has excellent endonuclease activity of recognizing a target nucleic acid or a target gene and cleaving a double-stranded DNA of the target site while having a similar size to a Cas12f1 protein, which belongs to the group with the smallest molecular weight among nucleic acid degrading proteins, and have successfully constructed an engineered guide RNA that exhibits excellent gene editing activity when used together with the CWCas12f. For details regarding the engineered guide RNA, described below. As an example, the wild-type Cas12f protein may comprise or consist of the amino acid sequence of SEQ ID NO: 1 (see Table 2). In addition, the Cas12f1 protein may be any protein that may be classified as an equivalent protein based on its function, structure, and/or sequence similarity in the relevant technical field, even if it has a different name. In addition, a variant of the wild-type Cas12f protein may comprise at least one mutation for expanding the genomic targeting range as disclosed herein.

In an aspect, the engineered Cas protein may comprise amino acid substitution(s) at one or more amino acid residues selected from the group consisting of amino acids at positions 159, 164, 170, 174, 184, 188, 191, 225, 230, and 272 with respect to SEQ ID NO: 1 or at amino acid residue(s) corresponding thereto, wherein the engineered Cas protein may comprise an amino acid sequence having at least 80%, at least 85%, at least 86%, at least 87%, at least 88%, at least 89%, at least 90%, at least 91%, at least 92%, at least 93%, at least 94%, at least 95%, at least 96%, at least 97%, or at least 98% sequence identity to the sequence represented by SEQ ID NO: 1. As used herein, "with respect to SEQ ID NO: 1" means a reference sequence for describing positions where amino acid substitution occurs. For example, with respect to SEQ ID NO: 5, which is the amino acid sequence of Un1Cas12f1 (a sequence having the amino acid sequence of SEQ ID NO: 1 from which 28 amino acids at the N-terminus are deleted), when aligned with SEQ ID NO: 1, the "positions corresponding thereto" will be positions 131, 136, 142, 146, 156, 160, 163, 197, 202, 243, and 244. For Cas12f proteins with other similar sequences, the corresponding positions may also be easily determined through sequence alignment. In addition, in the amino acid sequence of SEQ ID NO: 1, the substitution positions correspond to S170, Y174, A184, S188, R191, Q225, Y230, and Q272, respectively; however, it should be understood that the amino acids corresponding to these substitution positions may differ in cases where there are conservative substitutions of amino acids or mutations thereof within a range that does not impair the function of the Cas protein.

In an embodiment, the substitution(s) may comprise amino acid substitution(s) at one or more amino acid residues selected from the group consisting of amino acids at positions 170, 174, 184, 188, 191, 225, 230, 271, and 272 with respect to SEQ ID NO: 1 or at amino acid residue(s) corresponding thereto, wherein the engineered Cas protein shows altered protospacer-adjacent motif (PAM) recognition specificity. The wild-type Cas12f1 protein, for example, the wild-type Un1Cas12f1 or CWCas12f protein, shows preference for the PAM 5'-TTTR-3' (5'-TTTA-3' or 5'-TTTG-3'), whereas the variants comprising amino acid substitution(s) at one or more amino acid residues at the aforementioned positions recognize at least one PAM sequence selected from the group consisting of, for example, 5'-TVTN-3', 5'-TTVV-3', 5'-TGGG-3', and 5'-TTTN-3' (wherein N is A, T, G, or C, and V is A, G, or C) and exhibited excellent indel effects. More specifically, for the PAM sequences recognized by the variants, see Table 1.

In an embodiment, the substitution(s) may comprise one or more amino acid substitutions selected from the group consisting of 170C or 170T; 174H, 174A, 174E, 174K, 174N, 174R, or 174T; 184H, 184N, 184R, 184S, or 184T; 188G, 188H, 188K, 188N, 188Q, 188R, 188S, 188T, or 188V; 191G, 191H, 191K, 191Q, or 191W; 225F or 225T; 230A, 230H, 230I, 230S, or 230T; and 272C, 272K, or 272R.

In another embodiment, the substitution(s) may comprise one or more substitutions selected from the group consisting of S170C or S170T; Y174H, Y174A, Y174E, Y174K, Y174N, Y174R, or Y174T; A184H, A184N, A184R, A184S, or A184T; S188G, S188H, S188K, S188N, S188Q, S188R, S188S, S188T, or S188V; R191G, R191H, R191K, R191Q, orR191W; Q225F or Q225T; Y230A, Y230H, Y230I, Y230S, or Y230T; and Q272K, Q272C, or Q272R. Specifically, the substitution(s) may comprise one, two, or three amino acid substitutions selected from the group consisting of S170C or S170T; Y174H, Y174A, Y174E, Y174K, Y174N, Y174R, or Y174T; A184H, A184N, A184R, A184S, or A184T; S188G, S188H, S188K, S188N, S188Q, S188R, S188S, S188T, or S188V; R191G, R191H, R191K, R191Q, orR191W; Q225F or Q225T; Y230A, Y230H, Y230I, Y230S, or Y230T; and Q272K, Q272C, or Q272R. In yet another embodiment, the substitution(s) may comprise (a) S188Q/Q272K; (b) S188K/Q272K; or (c) S188Q/R191K/Q272K.

Exemplary Cas12f1 protein variants of the present disclosure exhibit the following PAM sequence preference.

**[Table 1]**

| Cas protein | Type | Amino acid substitution | Altered PAM preference |
|---|---|---|---|
| Un1Cas12f1 | wild type | - | TTTA, TTTG |
| CWCas12f | wild type | - | TTTA, TTTG |
| | variant 8 | S170C | TTTT, TTTG, TTTA |
| | variant 9 | S170T | TATA, TTTA, TTTT, TTTG, TGTA, TCTA, TGGG, TTAG, TTCA, TCTG, TGTG, TGGG |
| | variant 11 | Y174H | TGTA, TTTG, TTTA |
| | variant 44 | Y174A | TGTA, TTTG, TTTA, CGAG |
| | variant 45 | Y174E | CGAG, TGTA, TTTG, GGCA |
| | variant 12 | Y174K | TGTA, TTGA, TTTG, TGGG |
| | variant 46 | Y174N | TGTA, TTTG, TTTA |
| | variant 47 | Y174R | TGTA, TTGA, TTTG |
| | variant 13 | Y174T | TGTA, TTTG, TTGA, CGAG |
| | variant 15 | A184H | TGTA, TGTG |
| | variant 16 | A184N | TTTC, TGTA, TGTG, TTTG |
| | variant 17 | A184R | TGTG, TGTA |
| | variant 18 | A184S | TCTG, TTTC, TGTA, TTTG, TTTA |
| | variant 48 | A184T | TTTG, TTTA, TGTA, TCTG |
| | variant 49 | S188G | TTTG, TCTT, GACC, CGGA |
| | variant 20 | S188H | TGTA, TGTG, TGCA |
| | variant 21 | S188K | TTTA, TTTG, TTTC, TGTA, TGTG, TGGG, TTAG |
| | variant 22 | S188N | TATC, TGTC, TCTC, TCTG, TTCG, TTTC, TTTG, TTTA, TGTG, TGTA, TTCA, TGCA |
| | variant 23 | S188Q | TATC, TTTT, TTTG, TTTC, TGTA, TGTC, TGTG, TCTT, TCTG, TCTC, TATA, TGGG, TTTA, TGCA |
| | variant 24 | S188R | TTTG, TTTC, TTTA, TGTG, TGTA, TTCG, TTCA, TGCA |
| | variant 50 | S188S | TTTG, TTTA, TGTA, TCTG, TTGA |
| | variant 51 | S188T | TTTG, TTTA, TGTA, TGTG, TCTG, TTGA, TTCG, TTCA, TTAG |
| | variant 52 | S188V | TTTG, TTTA, TGTA, TTGA, TTAG |
| | variant 53 | R191G | TTTA, TTTG, TTTC, TGTA |
| | variant 54 | R191H | TTTA, TTTG, TTTC, TTCA |
| | variant 25 | R191K | TTTC |
| | variant 26 | R191Q | TATT, TATC, TTTT, TTTG, TTTC, TGTA, TGTC, TCTT, TCTC, TTTA, TCTG, TGTG, TATG, TCTT |
| | variant 27 | R191W | TCTG |
| | variant 28 | Q225F | TATA, TATT, TGTA, TGTG, TCTT, TGGG, TTTG, TATG, TATA |
| | variant 30 | Q225T | TATA, TGTA, TGTG, TCTT, TGGG, TCTA, TGCA |
| | variant 55 | Y230A | TTTG, TTTA, TGTA, TCTG, TTCA |
| | variant 32 | Y230H | TCTC, TTTG, TTTA, TGTA, TCTG, TTCA |
| | variant 56 | Y230I | TTTG, TTTA, TGTA, TCTG, TATT |
| | variant 35 | Y230S | TTTG, TCTG, TATA, TTTA, TTCA |
| | variant 36 | Y230T | TGTA, TCTC |
| | variant 38 | Q272C | TCTA |
| | variant 39 | Q272K | TATT, TATG, TTTA, TTTT, TTTG, TGTC, TCTT, TCTC, TTAG, TTCA, TTTC, TGTA, TCTG, TCTT, TATA, TTCG |
| | variant 40 | Q272R | TTTC, TGTA, TCTG, TTTG, TTGA, TTCG, TTCA, TTAG |
| | variant 41 | S188Q/Q272K | TATC, TCTG, TCTA, TGTC, TGTG, TGTT, TATG, TATA, TTTG, TTTA, TGTA |
| | variant 42 | S188K/Q272K | TATC, TCTA, TCTT, TGTC, TGTA, TGTT, TATG, TATA, TTTG, TTTT, TTTA, TCTG, TGTG |
| | variant 43 | S188Q/R191K/Q2 72K | TATC, TCTG, TCTA, TCTT, TGTG, TGTT, TATA, TTTC, TTTG, TTTA, TGTC, TGTA, TATG, |

In another embodiment, the substitution(s) may comprise amino acid substitution(s) at one or more amino acid residues selected from the group consisting of amino acids at positions 159 and 164 or at amino acid residue(s) corresponding thereto, wherein the engineered Cas protein has an altered editing window for PAM-proximal regions. The PAM-proximal region means a range that is at least 0 nucleotides away from the PAM sequence or 3 to 30 nucleotides, specifically 2 to 10 or 15 to 20 nucleotides away from the PAM sequence, but is not limited thereto. The term "editing window" as used herein means a range within a target sequence where the engineered Cas protein or fusion protein as disclosed herein can reach and cause alteration.

In another embodiment, the substitution(s) may comprise one or more amino acid substitutions selected from the group consisting of 159W and 164Y. Specifically, the substitution(s) may comprise one or more substitutions selected from the group consisting of I159W and S164Y.

In yet another embodiment, the engineered Cas protein may comprise one or more amino acid substitutions selected from the group consisting of 170C or 170T; 174H, 174A, 174E, 174K, 174N, 174R, or 174T; 184H, 184N, 184R, 184S, or 184T; 188G, 188H, 188K, 188N, 188Q, 188R, 188S, 188T, or 188V; 191G, 191H, 191K, 191Q, or 191W; 225F or 225T; 230A, 230H, 230I, 230S, or 230T; and 272K, 272C, or 272R, and may further comprise one or more amino acid substitutions selected from the group consisting of 159W and 164Y. For example, the engineered Cas12f1 protein may comprise at least one (for example, one, two, or three) substitutions selected from the group consisting of S170C or S170T; Y174H, Y174A, Y174E, Y174K, Y174N, Y174R, or Y174T; A184H, A184N, A184R, A184S, or A184T; S188G, S188H, S188K, S188N, S188Q, S188R, S188S, S188T, or S188V; R191G, R191H, R191K, R191Q, or R191W; Q225F or Q225T; Y230A, Y230H, Y230I, Y230S, or Y230T; and Q272K, Q272C, or Q272R, and may further comprise one or more substitutions selected from the group consisting of I159W and S164Y.

Specific amino acid sequence information for the wild-type Cas12f1 protein (for example, Un1Cas12f1 or CWCas12f) and the exemplary engineered Cas proteins of the present disclosure is provided in Table 2 below.

**[Table 2]**

| Name | Amino acid sequence | SEQ ID NO |
|---|---|---|
| CWCas12f | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIASGLRSKIKSNFRLKELKNMKSGLPTT KSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 1 |
| Un1Cas12f1 | MAKNTITKTLKLRIVRPYNSAEVEKIVADEKNNREKIALEKNK DKVKEACSKHLKVAAYCTTQVERNACLFCKARKLDDKFYQK | 5 |
| | LRGQFPDAVFWQEISEIFRQLQKQAAEIYNQSLIELYYEIFIKGK GIANASSVEHYLSDVCYTRAAELFKNAAIASGLRSKIKSNFRL KELKNMKSGLPTTKSDNFPIPLVKQKGGQYTGFEISNHNSDFII KIPFGRWQVKKEIDKYRPWEKFDFEQVQKSPKPISLLLSTQRR KRNKGWSKDEGTEAEIKKVMNGDYQTSYIEVKRGSKIGEKS AWMLNLSIDVPKIDKGVDPSIIGGIDVGVKSPLVCAINNAFSRY SISDNDLFHFNKKMFARRRILLKKNRHKRAGHGAKNKLKPITI LTEKSERFRKKLIERWACEIADFFIKNKVGTVQMENLESMKRK EDSYFNIRLRGFWPYAEMQNKIEFKLKQYGIEIRKVAPNNTSK TCSKCGHLNNYFNFEYRKKNKFPHFKCEKCNFKENADYNAA LNISNPKLKSTKEEP | |
| Engineered CWCas12f (D354A) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIASGLRSKIKSNFRLKELKNMKSGLPTT KSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIAVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 223 |
| Engineered CWCas12f (E450A) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIASGLRSKIKSNFRLKELKNMKSGLPTT KSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMANLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 224 |
| Engineered CWCas12f (R518A) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIASGLRSKIKSNFRLKELKNMKSGLPTT KSDNFPIPLVKQKGGQYTGFEISNHNSDFIIKIPFGRWQVKKEI DKYRPWEKFDFEQVQKSPKPISLLLSTQRRKRNKGWSKDEGT | 225 |
| | EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYAKKNKFPHFKCEKCNFKENADYNAALNISNPKLKSTKEEP | |
| Engineered CWCas12f (D538A) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIASGLRSKIKSNFRLKELKNMKSGLPTT KSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENAAYNAALNISNPKLKSTKEEP | 226 |
| Engineered CWCas12f (I159W) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGWANASSVEHYL SDVCYTRAAELFKNAAIASGLRSKIKSNFRLKELKNMKSGLPT TKSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 227 |
| Engineered CWCas12f (S164Y) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASYVEHYLS DVCYTRAAELFKNAAIASGLRSKIKSNFRLKELKNMKSGLPTT KSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF | 228 |
| | EYRKKNKFPHFKCEKCNFKENADYNAALNISNPKLKSTKEEP | |
| Engineered CWCas12f (I159W/S16 4Y) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGWANASYVEHYL SDVCYTRAAELFKNAAIASGLRSKIKSNFRLKELKNMKSGLPT TKSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 229 |
| Engineered CWCas12f (S170C) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLC DVCYTRAAELFKNAAIASGLRSKIKSNFRLKELKNMKSGLPTT KSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 230 |
| Engineered CWCas12f (S170T) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLT DVCYTRAAELFKNAAIASGLRSKIKSNFRLKELKNMKSGLPTT KSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 231 |
| Engineered CWCas12f (S170Y) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE | 232 |
| | ISEIFRQLQKQAAEIYNQSLIELYYEIFIKGKGIANASSVEHYLY DVCYTRAAELFKNAAIASGLRSKIKSNFRLKELKNMKSGLPTT KSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | |
| Engineered CWCas12f (Y174H) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCHTRAAELFKNAAIASGLRSKIKSNFRLKELKNMKSGLPT TKSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 233 |
| Engineered CWCas12f (Y174K) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCKTRAAELFKNAAIASGLRSKIKSNFRLKELKNMKSGLPT TKSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 234 |
| Engineered CWCas12f (Y174T) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCTTRAAELFKNAAIASGLRSKIKSNFRLKELKNMKSGLPTT KSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID | 235 |
| | KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | |
| Engineered CWCas12f (A184G) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNGAIASGLRSKIKSNFRLKELKNMKSGLPT TKSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 236 |
| Engineered CWCas12f (A184H) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNHAIASGLRSKIKSNFRLKELKNMKSGLPT TKSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 237 |
| Engineered CWCas12f (A184N) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNNAIASGLRSKIKSNFRLKELKNMKSGLPTT KSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF | 238 |
| | EYRKKNKFPHFKCEKCNFKENADYNAALNISNPKLKSTKEEP | |
| Engineered CWCas12f (A184R) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNRAIASGLRSKIKSNFRLKELKNMKSGLPTT KSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 239 |
| Engineered CWCas12f (A184S) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNSAIASGLRSKIKSNFRLKELKNMKSGLPTT KSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 240 |
| Engineered CWCas12f (A184W) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNWAIASGLRSKIKSNFRLKELKNMKSGLPT TKSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 241 |
| Engineered CWCas12f (S188H) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE | 242 |
| | ISEIFRQLQKQAAEIYNQSLIELYYEIFIKGKGIANASSVEHYLS DVCYTRAAELFKNAAIAHGLRSKIKSNFRLKELKNMKSGLPT TKSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | |
| Engineered CWCas12f (S188K) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIAKGLRSKIKSNFRLKELKNMKSGLPT TKSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 243 |
| Engineered CWCas12f (S188N) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIANGLRSKIKSNFRLKELKNMKSGLPT TKSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 244 |
| Engineered CWCas12f (S188Q) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIAQGLRSKIKSNFRLKELKNMKSGLPT TKSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID | 245 |
| | KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | |
| Engineered CWCas12f (S188R) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIARGLRSKIKSNFRLKELKNMKSGLPT TKSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 246 |
| Engineered CWCas12f (R191K) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIASGLKSKIKSNFRLKELKNMKSGLPT TKSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 247 |
| Engineered CWCas12f (R191Q) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIASGLQSKIKSNFRLKELKNMKSGLPT TKSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF | 248 |
| | EYRKKNKFPHFKCEKCNFKENADYNAALNISNPKLKSTKEEP | |
| Engineered CWCas12f (R191W) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIASGLWSKIKSNFRLKELKNMKSGLPT TKSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 249 |
| Engineered CWCas12f (Q225F) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIASGLRSKIKSNFRLKELKNMKSGLPTT KSDNFPIPLVKFKGGQYTGFEISNHNSDFIIKIPFGRWQVKKEID KYRPWEKFDFEQVQKSPKPISLLLSTQRRKRNKGWSKDEGTE AEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 250 |
| Engineered CWCas12f (Q225R) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIASGLRSKIKSNFRLKELKNMKSGLPTT KSDNFPIPLVKRKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 251 |
| Engineered CWCas12f (Q225T) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE | 252 |
| | ISEIFRQLQKQAAEIYNQSLIELYYEIFIKGKGIANASSVEHYLS DVCYTRAAELFKNAAIASGLRSKIKSNFRLKELKNMKSGLPTT KSDNFPIPLVKTKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | |
| Engineered CWCas12f (Y230C) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIASGLRSKIKSNFRLKELKNMKSGLPTT KSDNFPIPLVKQKGGQCTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 253 |
| Engineered CWCas12f (Y230H) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIASGLRSKIKSNFRLKELKNMKSGLPTT KSDNFPIPLVKQKGGQHTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 254 |
| Engineered CWCas12f (Y230K) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIASGLRSKIKSNFRLKELKNMKSGLPTT KSDNFPIPLVKQKGGQKTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID | 255 |
| | KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | |
| Engineered CWCas12f (Y230R) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIASGLRSKIKSNFRLKELKNMKSGLPTT KSDNFPIPLVKQKGGQRTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 256 |
| Engineered CWCas12f (Y230S) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIASGLRSKIKSNFRLKELKNMKSGLPTT KSDNFPIPLVKQKGGQSTGFEISNHNSDFIIKIPFGRWQVKKEID KYRPWEKFDFEQVQKSPKPISLLLSTQRRKRNKGWSKDEGTE AEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 257 |
| Engineered CWCas12f (Y230T) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIASGLRSKIKSNFRLKELKNMKSGLPTT KSDNFPIPLVKQKGGQTTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF | 258 |
| | EYRKKNKFPHFKCEKCNFKENADYNAALNISNPKLKSTKEEP | |
| Engineered CWCas12f (V271T) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIASGLRSKIKSNFRLKELKNMKSGLPTT KSDNFPIPLVKQKGGQYTGFEISNHNSDFIIKIPFGRWQVKKEI DKYRPWEKFDFEQTQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 259 |
| Engineered CWCas12f (Q272C) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIASGLRSKIKSNFRLKELKNMKSGLPTT KSDNFPIPLVKQKGGQYTGFEISNHNSDFIIKIPFGRWQVKKEI DKYRPWEKFDFEQVCKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 260 |
| Engineered CWCas12f (Q272K) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIASGLRSKIKSNFRLKELKNMKSGLPTT KSDNFPIPLVKQKGGQYTGFEISNHNSDFIIKIPFGRWQVKKEI DKYRPWEKFDFEQVKKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 261 |
| Engineered CWCas12f (Q272R) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE | 262 |
| | ISEIFRQLQKQAAEIYNQSLIELYYEIFIKGKGIANASSVEHYLS DVCYTRAAELFKNAAIASGLRSKIKSNFRLKELKNMKSGLPTT KSDNFPIPLVKQKGGQYTGFEISNHNSDFIIKIPFGRWQVKKEI DKYRPWEKFDFEQVRKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | |
| Engineered CWCas12f (S188Q/Q2 72K) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIAQGLRSKIKSNFRLKELKNMKSGLPT TKSDNFPIPLVKQKGGQYTGFEISNHNSDFIIKIPFGRWQVKKEI DKYRPWEKFDFEQVKKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 263 |
| Engineered CWCas12f (S188K/Q2 72K) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIAKGLRSKIKSNFRLKELKNMKSGLPT TKSDNFPIPLVKQKGGQYTGFEISNHNSDFIIKIPFGRWQVKKEI DKYRPWEKFDFEQVKKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 264 |
| Engineered CWCas12f (S188Q/R19 1K/Q272K) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIAQGLKSKIKSNFRLKELKNMKSGLPT TKSDNFPIPLVKQKGGQYTGFEISNHNSDFIIKIPFGRWQVKKEI DKYRPWEKFDFEQVKKSPKPISLLLSTQRRKRNKGWSKDEGT EAEIKKVMNGDYQTSYIEVKRGSKIGEKSAWMLNLSIDVPKID | 265 |
| | KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | |
| Engineered CWCas12f (Y174A) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCATRAAELFKNAAIASGLRSKIKSNFRLKELKNMKSGLPTT KSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 345 |
| Engineered CWCas12f (Y174E) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCETRAAELFKNAAIASGLRSKIKSNFRLKELKNMKSGLPTT KSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 346 |
| Engineered CWCas12f (Y174N) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCNTRAAELFKNAAIASGLRSKIKSNFRLKELKNMKSGLPTT KSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF | 347 |
| | EYRKKNKFPHFKCEKCNFKENADYNAALNISNPKLKSTKEEP | |
| Engineered CWCas12f (Y174R) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCRTRAAELFKNAAIASGLRSKIKSNFRLKELKNMKSGLPTT KSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 348 |
| Engineered CWCas12f (A184T) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNTAIASGLRSKIKSNFRLKELKNMKSGLPTT KSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 349 |
| Engineered CWCas12f (S188G) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIAGGLRSKIKSNFRLKELKNMKSGLPT TKSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 350 |
| Engineered CWCas12f (S188S) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE | 351 |
| | ISEIFRQLQKQAAEIYNQSLIELYYEIFIKGKGIANASSVEHYLS DVCYTRAAELFKNAAIASGLRSKIKSNFRLKELKNMKSGLPTT KSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | |
| Engineered CWCas12f (S188T) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIATGLRSKIKSNFRLKELKNMKSGLPT TKSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 352 |
| Engineered CWCas12f (S188V) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIAVGLRSKIKSNFRLKELKNMKSGLPT TKSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 353 |
| Engineered CWCas12f (R191G) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIASGLGSKIKSNFRLKELKNMKSGLPT TKSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID | 354 |
| | KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | |
| Engineered CWCas12f (R191H) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIASGLHSKIKSNFRLKELKNMKSGLPT TKSDNFPIPLVKQKGGQYTGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 355 |
| Engineered CWCas12f (Y230A) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIASGLRSKIKSNFRLKELKNMKSGLPTT KSDNFPIPLVKQKGGQATGFEISNHNSDFIIKIPFGRWQVKKEI DKYRPWEKFDFEQVQKSPKPISLLLSTQRRKRNKGWSKDEGT EAEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF EYRKKNKFPHFKCEKCNFKENADYNAALNISNPKLKSTKEEP | 356 |
| Engineered CWCas12f (Y230I) | MGEKSSRRRRNGKSGAWTAAITSCVGGKMAKNTITKTLKLRI VRPYNSAEVEKIVADEKNNREKIALEKNKDKVKEACSKHLKV AAYCTTQVERNACLFCKARKLDDKFYQKLRGQFPDAVFWQE ISEIFRQLQKQAAEIYNQSLIELYYEIFIKGKGIANASSVEHYLS DVCYTRAAELFKNAAIASGLRSKIKSNFRLKELKNMKSGLPTT KSDNFPIPLVKQKGGQITGFEISNHNSDFIIKIPFGRWQVKKEID KYRPWEKFDFEQVQKSPKPISLLLSTQRRKRNKGWSKDEGTE AEIKKVMNGDYQTSYIEVKRGSKIGEKSAWMLNLSIDVPKID KGVDPSIIGGIDVGVKSPLVCAINNAFSRYSISDNDLFHFNKKM FARRRILLKKNRHKRAGHGAKNKLKPITILTEKSERFRKKLIER WACEIADFFIKNKVGTVQMENLESMKRKEDSYFNIRLRGFWP YAEMQNKIEFKLKQYGIEIRKVAPNNTSKTCSKCGHLNNYFNF | 357 |
| | EYRKKNKFPHFKCEKCNFKENADYNAALNISNPKLKSTKEEP | |

In another embodiment, the engineered Cas protein may be a catalytically inactive variant (with DNA cleavage activity eliminated). Specifically, the engineered Cas protein may be in a catalytically inactive dead form that further comprises amino acid substitution(s) at one or more amino acid residues selected from the group consisting of amino acids at positions 354, 450, 518, and 538 with respect to SEQ ID NO: 1 or at amino acid residue(s) corresponding thereto. More specifically, the engineered Cas12f1 protein may further comprise at least one amino acid substitution selected from the group consisting of (a) 354A, 354Q, 354L, 354W, or 354V; (b) 450A, 450Q, 450L, 450W, or 450V; (c) 518A, 518Q, 518L, 518W, or 518V; and (d) 538A, 538Q, 538L, 538W, or 538V. In an embodiment, the engineered Cas protein may be in a catalytically inactive dead form that further comprises at least one amino acid substitution selected from the group consisting of D354A, E450A, R518A, and D538A.

In yet another embodiment, the engineered Cas12f1 protein may further comprise modification such as deletion, insertion, substitution, or addition of at least one amino acid. The deletion, insertion, substitution or addition of at least one amino acid may be any mutation that affects functions such as catalytic activity, stability or the like of the Cas12f1 protein. The mutation may be any mutation that does not affect alteration of the PAM recognition specificity and/or the editing window for the PAM-proximal regions, provided that the deletion, insertion, substitution, or addition of at least one amino acid does not impair the function of the Cas12f1 protein in forming a complex with a guide RNA and interacting with a target gene sequence. For example, the engineered Cas12f1 protein may further comprise a sequence in which at least one amino acid residue is deleted, substituted, inserted, and/or added at the C-terminus, the N-terminus, or internally within the sequence.

In an embodiment, the engineered Cas12f1 protein may further comprise at least one arbitrary amino acid added to the N-terminus and/or the C-terminus. The present inventors have confirmed that among the variants of the wild-type Cas12f1 protein with amino acids added to the N-terminus and/or C-terminus, there are variants that exhibit a function equivalent to the wild-type Cas12f1. For this purpose, reference may be made to Korean Patent Application Nos. 10-2021-0181875 and 10-2022-0128219, the disclosures of which are considered incorporated herein in their entirety. Preferably, the engineered Cas protein may further comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acids at the N-terminus and/or C-terminus. As an example, the engineered Cas12f1 protein may comprise an amino acid sequence with 1 to 28 amino acids added to the N-terminus. As a specific example, the engineered Cas12f1 protein may comprise one or more amino acid substitutions for altering the PAM recognition specificity or the editing window for the PAM-proximal regions at corresponding positions in the CWCas12f-v1 protein (SEQ ID NO: 2), which further comprises 26 amino acids derived from the N-terminus of CasX at the N-terminus, the CWCas12f-v2 protein (SEQ ID NO: 3), which further comprises 28 random amino acids at the N-terminus, or the CWCas12f-v3 protein (SEQ ID NO: 4), which further comprises 26 random amino acids at the N-terminus. The specific amino acid sequences of the CWCas12f-v1 protein (SEQ ID NO: 2), the CWCas12f-v2 protein (SEQ ID NO: 3), and the CWCas12f-v3 protein (SEQ ID NO: 4) are as follows:
"CWCas12f-v1 protein", 
MEKRINKIRKKLSADNATKPVSRSGPMAKNTITKTLKLRIVRPYNSAEVEKIVADEKN
NREKIALEKNKDKVKEACSKHLKVAAYCTTQVERNACLFCKARKLDDKFYQKLRG
QFPDAVFWQEISEIFRQLQKQAAEIYNQSLIELYYEIFIKGKGIANASSVEHYLSDVCYT
RAAELFKNAAIASGLRSKIKSNFRLKELKNMKSGLPTTKSDNFPIPLVKQKGGQYTGF
EISNHNSDFIIKIPFGRWQVKKEIDKYRPWEKFDFEQVQKSPKPISLLLSTQRRKRNKG
WSKDEGTEAEIKKVMNGDYQTSYIEVKRGSKIGEKSAWMLNLSIDVPKIDKGVDPSII
GGIDVGVKSPLVCAINNAFSRYSISDNDLFHFNKKMFARRRILLKKNRHKRAGHGAK
NKLKPITILTEKSERFRKKLIERWACEIADFFIKNKVGTVQMENLESMKRKEDSYFNIR
LRGFWPYAEMQNKIEFKLKQYGIEIRKVAPNNTSKTCSKCGHLNNYFNFEYRKKNKF
PHFKCEKCNFKENADYNAALNISNPKLKSTKEEP (SEQ ID NO: 2);
"CWCas12f-v2 protein", 
MAGGPGAGSAAPVSSTSSLPLAALNMRVMAKNTITKTLKLRIVRPYNSAEVEKIVAD
EKNNREKIALEKNKDKVKEACSKHLKVAAYCTTQVERNACLFCKARKLDDKFYQK
LRGQFPDAVFWQEISEIFRQLQKQAAEIYNQSLIELYYEIFIKGKGIANASSVEHYLSDV
CYTRAAELFKNAAIASGLRSKIKSNFRLKELKNMKSGLPTTKSDNFPIPLVKQKGGQY
TGFEISNHNSDFIIKIPFGRWQVKKEIDKYRPWEKFDFEQVQKSPKPISLLLSTQRRKR
NKGWSKDEGTEAEIKKVMNGDYQTSYIEVKRGSKIGEKSAWMLNLSIDVPKIDKGV
DPSIIGGIDVGVKSPLVCAINNAFSRYSISDNDLFHFNKKMFARRRILLKKNRHKRAGH
GAKNKLKPITILTEKSERFRKKLIERWACEIADFFIKNKVGTVQMENLESMKRKEDSY
FNIRLRGFWPYAEMQNKIEFKLKQYGIEIRKVAPNNTSKTCSKCGHLNNYFNFEYRK
KNKFPHFKCEKCNFKENADYNAALNISNPKLKSTKEEP (SEQ ID NO: 3);
"CWCas12f-v3 protein",

MAGGPGAGSAAPVSSTSSLPLAALNMMAKNTITKTLKLRIVRPYNSAEVEKIVADEK
NNREKIALEKNKDKVKEACSKHLKVAAYCTTQVERNACLFCKARKLDDKFYQKLR
GQFPDAVFWQEISEIFRQLQKQAAEIYNQSLIELYYEIFIKGKGIANASSVEHYLSDVC
YTRAAELFKNAAIASGLRSKIKSNFRLKELKNMKSGLPTTKSDNFPIPLVKQKGGQYT
GFEISNHNSDFIIKIPFGRWQVKKEIDKYRPWEKFDFEQVQKSPKPISLLLSTQRRKRN
KGWSKDEGTEAEIKKVMNGDYQTSYIEVKRGSKIGEKSAWMLNLSIDVPKIDKGVD
PSIIGGIDVGVKSPLVCAINNAFSRYSISDNDLFHFNKKMFARRRILLKKNRHKRAGHG
AKNKLKPITILTEKSERFRKKLIERWACEIADFFIKNKVGTVQMENLESMKRKEDSYF
NIRLRGFWPYAEMQNKIEFKLKQYGIEIRKVAPNNTSKTCSKCGHLNNYFNFEYRKK
NKFPHFKCEKCNFKENADYNAALNISNPKLKSTKEEP (SEQ ID NO: 4).

For the human codon-optimized nucleotide sequences encoding the CWCas12f-v1 protein, CWCas12f-v2 protein, and CWCas12f-v3 protein, see SEQ ID NO: 7 to 9.

In another embodiment, the engineered Cas12f1 protein may additionally comprise 1 to 600 amino acids at the N-terminus or C-terminus. Here, there is no limitation on the added sequence of 1 to 600 amino acids. For example, the added 1 to 600 amino acids may be the amino acid sequence of SEQ ID NO: 190 or SEQ ID NO: 191. Meanwhile, an NLS or NES sequence may further be included between the added sequence and the Cas12f1 variant protein. For details regarding the NLS or NES, see the entire contents described below.

In yet another embodiment, the engineered Cas protein may further comprise deletion or substitution of at least one amino acid residue within a RuvC domain. The RuvC (or RuvC-like) domain, also referred to as an endonuclease domain, comprises a catalytically active site that catalyzes nucleic acid cleavage and is therefore directly related to nucleic acid cleavage efficiency. Thus, by modification of the RuvC domain, the Cas12f1 protein may be engineered to exhibit equal or enhanced effects (for example, improved nucleic acid cleavage efficiency) compared to the wild-type Cas12f1 protein while retaining the same function (for example, nucleic acid cleavage function).

### III. Fusion protein

The engineered Cas protein disclosed herein may be used in fusion with a functional domain having a different function or activity. Accordingly, according to another aspect of the present disclosure, there is provided a fusion protein comprising (i) the engineered Cas proteins disclosed herein and (ii) a functional domain.

In an embodiment, the functional domain may be, but is not limited to, a domain having nuclease activity, nickase activity, recombinase activity, deaminase activity, methyltransferase activity, methylase activity, acetylase activity, acetyltransferase activity, transcriptional activation activity, transcriptional inhibition activity, nucleic acid binding activity, or reverse transcriptase activity. For example, when fused with a domain having deaminase activity, the fusion protein may function as a base editor. Adenosine deaminase for adenine base editing includes tRNA deaminase (TadA) derived from *E. coli* and/or variants thereof. The functional domain of the fusion protein of the present disclosure may comprise at least one selected from TadA and variants thereof, for example. In addition, the functional domain may be an enzyme that causes various quantitative and/or qualitative changes in intracellular gene expression, such as DNMT, TET, KRAB, DHAC, LSD, p300, M-MLV (moloney murine leukemia virus) reverse transcriptase. When fused with reverse transcriptase, the fusion protein may function as a prime editor.

In an embodiment, the functional domain may have deaminase activity. For example, the functional domain may be an adenosine deaminase or a cytidine deaminase. The adenosine deaminase includes, but is not limited to, tRNA adenosine deaminase (TadA) from *E. coli,* variants of TadA, or combinations thereof. The cytidine deaminase includes, but is not limited to, human AID (activation-induced cytidine deaminase), human APOBEC3G, mouse APOBEC1, APOBEC3A, APOBEC3B, or AID, and lamprey PmCDA1.

In another embodiment, the functional domain may comprise at least one expression regulatory domain having transcriptional activation activity or transcriptional inhibition activity. For example, the transcriptional activation domain may be VP64, Sun Tag, VPR (VP64, p65, Rta), or TV (TAL, VP64). In addition, the transcriptional inhibition domain may be p300, KRAB, DNMT, MeCP2, HDAC, LSD, SRDX, SALL1, or SDS3.

In another embodiment, the functional domain may be a tag or reporter protein for separation and/or purification. For example, the tag or reporter protein includes, but is not limited to, a tag protein such as a histidine (His) tag, a V5 tag, a FLAG tag, an influenza hemagglutinin (HA) tag, a Myc tag, a VSV-G tag, and a thioredoxin (Trx) tag; a fluorescent protein such as green fluorescent protein (GFP), yellow fluorescent protein (YFP), cyan fluorescent protein (CFP), blue fluorescent protein (BFP), HcRED, and DsRed; and a reporter protein (enzyme) such as glutathione-S-transferase (GST), horseradish peroxidase (HRP), chloramphenicol acetyltransferase (CAT), beta-galactosidase, beta-glucuronidase, and luciferase.

In yet another embodiment, the functional domain may comprise one or more nuclear localization signal (NLS) sequences that localize the engineered Cas12f1 protein into the nucleus. For example, one or more nuclear localization signal sequences may have a sufficient amount or activity to induce the Cas12f1 molecule to be targeted or transported into the nucleus of a eukaryotic cell (for example, a mammalian cell) in a detectable amount. For example, differences in the strength of activity may result from the number of NLSs included in the Cas12f1 protein, the type of specific NLS(s) used, or a combination of these factors. Specifically, the NLS fused to the engineered Cas12f1 protein of the present disclosure may be variously selected from about 1, 2, 3, 4, 5, 6, 7, 8, 9, or 10 or more NLSs at or near the N-terminus, about 1, 2, 3, 4, 5, 6, 7, 8, 9, or 10 or more NLSs at or near the C-terminus, or combinations thereof. For example, the Cas12f1 protein may comprise 0 or at least one NLS sequence at the N-terminus and/or 0 or at least one NLS sequence at the C-terminus. When more than one NLS sequence is present, a single NLS may be present in more than one copy and each NLS sequence may be selected independently of the others such that it exists in combination with more than one other NLS present in more than one copy.

In addition, the engineered Cas protein may be fused with a nuclear export signal (NES). The NES sequence refers to a peptide of a certain length or a sequence thereof that attaches to a protein to be transported and acts as a type of "tag" when a material is transported from the inside of the nucleus to the outside of the nucleus by nuclear transport in a cell.

### IV. Guide RNA

The guide RNA comprises a spacer region (or guide region) that comprises a guide sequence hybridizable to a target sequence adjacent to PAM and a scaffold region that can interact with a Cas protein to form a complex. As used herein, the "target sequence" refers to a sequence in a target gene or target region, which is recognized by the guide RNA or is subject to modification by the gene editing system. A guide sequence of the guide RNA is designed to have complementarity to the target sequence. Hybridization between the guide sequence and the target sequence enables the Cas protein to be positioned at the target sequence. Perfect complementarity is not necessarily required, and it is sufficient if there is enough complementarity between the guide sequence and the target sequence to allow for hybridization. In an embodiment, the complementary bonds between the guide sequence and the target sequence may include one or more mismatch bonds, preferably 0 to 7, more preferably 0 to 6, even more preferably 0 to 5 mismatches.

The wild-type Cas12f guide RNA comprises tracrRNA and crRNA. These tracrRNA and crRNA may be linked to form sgRNA. Specifically, the wild-type gRNA may comprise a wild-type tracrRNA having the nucleotide sequence of SEQ ID NO: 11, or a wild-type crRNA having the nucleotide sequence of SEQ ID NO: 12. In addition, the wild-type gRNA may be fused in the form of a single guide RNA to become a single guide RNA (sgRNA) having the nucleotide sequence of SEQ ID NO: 13. Representative sequences of the wild-type tracrRNA, crRNA, and sgRNA are presented in Table 3.

**[Table 3]**

| **Name** | **Nucleotide sequence (5'→3')** | **SEQ ID NO** |
|---|---|---|
| Wild-type tracrRNA | CUUCACUGAUAAAGUGGAGAACCGCUUCACCAAAAGCUG UCCCUUAGGGGAUUAGAACUUGAGUGAAGGUGGGCUGCU UGCAUCAGCCUAAUGUCGAGAAGUGCUUUCUUCGGAAAG UAACCCUCGAAACAAAUUCAUUUUUCCUCUCCAAUUCUG CACAA | 11 |
| Wild-type crRNA | GUUGCAGAACCCGAAUAGACGAAUGAAGGAAUGCAAC | 12 |
| Canonical sgRNA | CUUCACUGAUAAAGUGGAGAACCGCUUCACCAAAAGCUG UCCCuuagGGGAUUAGAACUUGAGUGAAGGUGGGCUGCUU GCAUCAGCCUAAUGUCGAGAAGUGCUUUCUUCGGAAAGU AACCCUCGAAACAAAUUCAUUUUUCCUCUCCAAUUCUGC ACAAgaaaGUUGCAGAACCCGAAUAGacgaaUGAAGGAAUGC AACNNNNNNNNNNNNNNNNNNNN | 13 |

In Table 3 and throughout the specification, the sequence indicated by 'NNNNNNNNNNNNNNNNNNNN' refers to any guide sequence (spacer sequence) having any length (for example, 15 to 40 nucleotides in length) that can hybridize with a target sequence in a target site. The crRNA of the wild-type Cas12f guide RNA may comprise a direct repeat sequence and a guide sequence, and the direct repeat sequence may be located at the 5'-end of the guide sequence. In addition, the crRNA may be located at the 3'-end of the tracrRNA. The scaffold region of the wild-type Cas12f guide RNA comprises tracrRNA and a portion of crRNA. The scaffold region may further comprise one or more stem regions (for example, a first stem region, a second stem region, a third stem region, and a fourth stem region) and a tracrRNA-crRNA complementarity region (which may be referred to as a fifth stem region). In addition, the wild-type Cas12f guide RNA may comprise (i) at least one stem-loop region, (ii) a tracrRNA-crRNA complementarity region, and optionally (iii) a region comprising three or more, four or more, or five or more consecutive uracil (U) residues. For detailed information on the structure of the Cas12f guide RNA, see Takeda et al., Structure of the miniature type V-F CRISPR-Cas effector enzyme, Molecular Cell 81, 1-13 (2021), the entire disclosures of which are incorporated herein by reference.

In another embodiment, the guide RNA disclosed herein may be an engineered guide RNA. The present inventors increased efficiency of the CRISPR/Cas12f1 system, which is a new CRISPR/Cas system, through a previous study and named it TaRGET (Tiny nuclease augmented RNA-based Genome Editing Technology) system. The CRISPR/Cas12f1 system is a novel CRISPR/Cas system that was first reported in a previous study [see Harrington et al., Science, 362, 839-842, 2018], and despite the advantage of having a remarkably small effector protein, it has been reported that there is no or very low double-strand DNA cleavage activity, which limits its application in gene editing technology. To overcome these limitations, the present inventors have researched, developed, and completed an engineered guide RNA that has enhanced cleavage activity for double-stranded DNA (dsDNA) so that it can be utilized for gene editing. For detailed descriptions of engineered guide RNAs, see Korean Patent Application Nos. 10-2021-0051552, 10-2021-0050093, and 10-2021-0044152; International Application Nos. PCT/KR2021/013898, PCT/KR2021/013923, and PCT/KR2021/013933; and Kim, D. Y. et al. Efficient CRISPR editing with a hypercompact Cas12f1 and engineered guide RNAs delivered by adeno-associated virus. Nat. Biotechnol. 40, 94-102, 2021, the contents of which are considered to be incorporated herein in their entirety. In an embodiment of the present disclosure, the TaRGET system may be a system comprising the Un1Cas12f1 or CWCas12 based protein (the wild type or engineered form disclosed herein) and the engineered gRNA.

In an embodiment, the engineered gRNA is an engineered gRNA comprising a sequence having the wild-type gRNA sequence in which at least one nucleotide has been substituted, deleted, inserted, or added, wherein the sequence excluding the guide sequence has at least 50%, 55%, 60%, 65%, 70%, 75%, 80%, 85%, 90%, 91%, 92%, 93%, 94%, 95%, 96%, 97%, 98%, 99%, or 100% sequence identity to the wild-type Cas12f1 gRNA.

Hereinafter, the structures of wild-type and engineered gRNA and modifications thereof will be described in detail for each of the five modification sites. The modification site is abbreviated as "MS" throughout this specification, and the numbers following "modification site" or "MS" are sequentially assigned depending on engineering flow of each modification site according to an embodiment. However, this does not mean that engineering (modification) at a modification site with a later number necessarily includes engineering (modification) at a modification site with an earlier number. FIG. 20 illustrates modification sites MS1 to MS5 included in the engineered guide RNA according to an embodiment of the present disclosure on the wild-type guide RNA sequence.

In an embodiment, among the subdivided regions of the gRNA as described above, the first stem-loop region comprising modification site 3 (MS3), the second stem-loop region comprising modification site 5 (MS5), and the tracrRNA-crRNA complementarity region (the fifth stem region or the fifth stem-loop region) comprising modification site 1 (MS1) and modification site 4 (MS4) may be defined as corresponding to or included in regions marked by single dotted line boxes with different shades of color in FIG. 20. In addition, the third stem-loop region may be defined as corresponding to or included in the G(-90)-C(-74) sequence in FIG. 20, and the fourth stem-loop region may be defined as corresponding to or included in the U(-68)-A(-35) sequence in FIG. 20.

The modifications applied to the engineered guide RNA (gRNA) of the present disclosure are ultimately intended to achieve high gene editing efficiency while deriving a gRNA that is shorter in length. That is, the modifications disclosed in the present disclosure are intended to produce an engineered gRNA of a shorter length having equal or improved recognition/cleavage efficiency for a target nucleic acid compared to the wild-type gRNA of a longer length, thereby allowing more space to be allocated to other components (for example, additional guide RNAs, shRNAs for inhibiting specific gene expression, and the like) for various purposes or uses within the packaging limit (about 4.7 kb) of a delivery vehicle such as adeno-associated virus (AAV). This provides a highly efficient gene editing effect that could not be achieved with the existing CRISPR/Cas system.

In another embodiment, compared to a wild-type Cas12f1 gRNA comprising (i) at least one stem-loop region, (ii) a tracrRNA-crRNA complementarity region and optionally (iii) a region comprising three or more, four or more, or five or more consecutive uracil (U) residues, the engineered gRNA of the present disclosure may comprise at least one modification selected from the group consisting of (a) deletion of at least a part of the at least one stem-loop region; (b) deletion of at least a part of the tracrRNA-crRNA complementarity region; (c) replacement of one or more of uracil (U) residues when three or more, four or more, or five or more consecutive uracil (U) residues are present; and (d) addition of one or more uridine residues to the 3'-end of the crRNA sequence.

In yet another embodiment, the engineered guide RNA may comprise at least one modification selected from the group consisting of (a1) deletion of at least a part of the first stem-loop region; (a2) deletion of at least a part of the second stem-loop region; (b) deletion of at least a part of the tracrRNA-crRNA complementarity region; (c) replacement of one or more U residues with A, G, or C in three or more, four or more, or five or more consecutive uracil (U) residues when the consecutive U residues are present in the tracrRNA-crRNA complementarity region; and (d) addition of a U-rich tail to the 3'-end of the crRNA sequence (in which a sequence of the U-rich tail is represented by 5'-(UₘV)ₙUₒ-3', wherein V is each independently A, C, or G, m and o are integers between 1 and 20, and n is an integer between 0 and 5).

In still yet another embodiment, the engineered guide RNA comprises a U-rich tail sequence. The U-rich tail is represented by 5'-(UₘV)ₙUₒ-3', wherein V is each independently A, C, or G, m and o are integers between 1 and 20, and n is an integer between 0 and 5. A detailed description of the U-rich tail sequence is provided below.

In still yet another embodiment, the engineered guide RNA may comprise a scaffold region comprising a (scaffold) sequence represented by the following Formula (I).

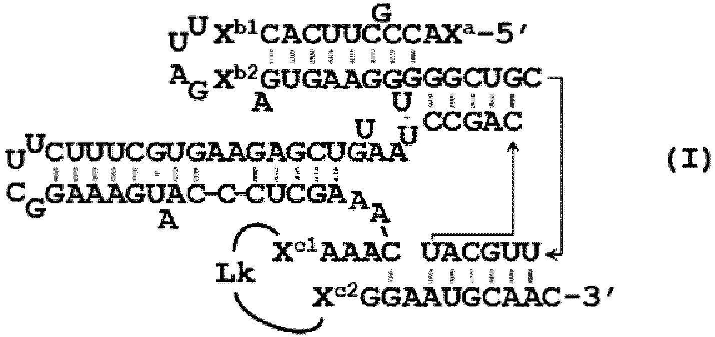

In Formula (I), X^{a}, X^{b1}, X^{b2}, X^{c1}, and x^{c2} each independently consist of 0 to 35 (poly)nucleotides, and Lk is a polynucleotide linker of 2 to 20 nucleotides in length or absent.

[In Formula (I), the black solid line refers to a chemical bond (for example, phosphodiester bond) between nucleotides or specific molecules, and the gray thick line refers to a complementary bond between nucleotides].

In Formula (I), in a case where X^{a}, X^{b1}, X^{b2}, X^{c1}, or x^{c2} consists of 0 nucleotides, it is interpreted to mean that X^{a}, X^{b1}, X^{b2}, X^{c1}, or X^{c2} is absent.

In addition, in Formula (I), in a case where X^{a}, X^{b1}, X^{b2}, X^{c1}, or x^{c2} consists of 0 nucleotides or is absent, it is interpreted that if there are two or more nucleotides linked through X^{a}, X^{b1}, X^{b2}, X^{c1}, or x^{c2}, these nucleotides are directly linked to each other in any way. For example, in Formula (I), in a case where X^{b1} consists of 0 nucleotides or is absent, the nucleotide directly linked to the 5'-end of X^{b1} and the nucleotide directly linked to the 3'-end of X^{b1} may be directly linked, for example, by a phosphodiester bond.

In an embodiment, X^{a} may be absent or a (poly)nucleotide having a stem-loop conformation. In another embodiment, X^{a} may consist of 0 to 20 (poly)nucleotides.

In an embodiment, X^{b1} and X^{b2} may be (poly)nucleotides capable of complementary binding. In another embodiment, X^{b1} may consist of 0 to 13 (poly)nucleotides, or X^{b2} may consist of 0 to 14 (poly)nucleotides.

In an embodiment, X^{c1} and X^{c2} may be (poly)nucleotides capable of complementary binding. In another embodiment, X^{c1} may consist of 0 to 28 (poly)nucleotides, or x^{c2} may consist of 0 to 27 (poly)nucleotides.

In an embodiment, Lk is a polynucleotide linker of 2 to 20, 2 to 15, 2 to 10, or 2 to 8 nucleotides, or is absent.

In yet another embodiment, the scaffold region of the engineered gRNA may be a gRNA consisting of a scaffold sequence represented by Formula (I) or having at least 80%, at least 85%, at least 90%, or at least 95% sequence identity with the sequence. Here, the sequence identity with Formula (I) is based on the sequence excluding the regions indicated by the symbols.

When referring to the scaffold region of the wild-type guide RNA, the first stem-loop region of the scaffold sequence may be a region corresponding to or comprising X^{a} in Formula (I). The second stem-loop region of the scaffold sequence may be a region corresponding to or comprising X^{b1} and X^{b2} in Formula (I). For example, the second stem-loop region comprising X^{b1} and X^{b2} may be a region corresponding to the sequence 5'-CCGCUUCAC-X^{b1}-uuag-X^{b2}-AGUGAAGGUG-3'. The third stem region of the scaffold sequence may be a region corresponding to or comprising the sequence 5'-GGCUGCUUGCAUCAGCC-3' in Formula (I). The fourth stem-loop region of the scaffold sequence may be a region corresponding to or comprising the sequence 5'-UCGAGAAGUGCUUUCUUCGGAAAGUAACCCUCGA-3' in Formula (I). In addition, the tracrRNA-crRNA complementarity region (the fifth stem(-loop) region) of the scaffold sequence may be a region corresponding to X^{c1} and x^{c2} in Formula (I).

Hereinafter, modifications at respective modification sites in the engineered gRNA will be described in detail.

### (1) Modification at modification site 1 (MS1)

This section describes a modification at MS1 (FIG. 20). In an embodiment, wild-type tracrRNA (for example, SEQ ID NO: 11), which may be included in a naturally occurring guide RNA (gRNA) may have a sequence containing five consecutive uracil (U) residues therein. This poses a problem in that, in a case of attempting to express the wild-type tracrRNA in a cell using a vector or the like, such a sequence acts as a transcription termination signal under certain conditions, thereby causing unintended early termination of transcription. That is, in a case where the sequence containing five consecutive U residues acts as a transcription termination signal, normal or complete expression of the tracrRNA is inhibited, and formation of normal or complete gRNA is also inhibited, which consequently decreases gene editing efficiency.

Therefore, in order to solve the above-mentioned problem, the engineered gRNA may be such that at least one uracil (U) of three or more, four or more, or five or more consecutive U residues, preferably four or five U residues, which are contained in the wild-type tracrRNA (for example, SEQ ID NO: 11), is artificially modified into another nucleotide such as A, C, T, or G.

In an embodiment, the engineered gRNA is provided which comprises a modification in which at least one of three or more, four or more, or five or more consecutive U residues is substituted with a different type of nucleotide in a region containing three or more, four or more, or five or more consecutive U residues, referred to as MS1. As an example, the three or more, four or more, or five or more consecutive U residues may be present in the tracrRNA-crRNA complementarity region of the tracrRNA, wherein a modification may be made by substituting at least one of the three or more, preferably four or more, or five or more U residues with A, G, or C such that no sequence with three or more, preferably four or more, or five or more consecutive U residues exists.

Here, it is preferable that the sequence within the tracrRNA-crRNA complementarity region of crRNA, which corresponds to the sequence to be modified, is also modified together. In an embodiment, when there is the sequence 5'-ACGAA-3' within the tracrRNA-crRNA complementarity region of crRNA, which forms a partial complementary bond with the sequence 5'-UUUUU-3' within the tracrRNA-crRNA complementarity region of tracrRNA, this sequence may be replaced with 5'-NGNNN-3'. Here, N is each independently A, C, G, or U.

In an embodiment, the engineered gRNA of Formula (I) may comprise a modification in which one or more of the U residues are substituted with A, G, or C, when three or more, four or more, or five or more consecutive uracil (U) residues are present in the X^{c1} sequence. For example, when the sequence 5'-UUUUU-3' is present in the X^{c1} sequence, the sequence may be replaced with 5'-NNNCN-3', wherein N is each independently A, C, G, or U. As a more specific example, the sequence 5'-UUUUU-3' in the X^{c1} sequence may be replaced by any one nucleotide sequence selected from the group consisting of the following sequences; however, the replacing sequence is not limited to the following sequences as long as it prevents appearance of a sequence containing three or more, preferably four or more or five or more consecutive U residues: 5'-UUUCU-3', 5'-GUUCU-3', 5'-UCUCU-3', 5'-UUGCU-3', 5'-UUUCC-3', 5'-GCUCU-3', 5'-GUUCC-3', 5'-UCGCU-3', 5'-UCUCC-3', 5'-UUGCC-3', 5'-GCGCU-3', 5'-GCUCC-3', 5'-GUGCC-3', 5'-UCGCC-3', 5'-GCGCC-3', and 5'-GUGCU-3'.

In another embodiment, in the engineered gRNA of Formula (I), the x^{c2} sequence comprises a region in which at least a part of the sequence forms a complementary bond with the X^{c1} sequence (also referred to as a tracrRNA-crRNA complementarity region), wherein a corresponding sequence in the x^{c2} sequence, which forms at least one complementary bond with 3 or more, 4 or more, or 5 or more consecutive U residues present in the X^{c1} sequence, may also be modified. For example, when the sequence 5'-ACGAA-3' is present in the x^{c2} sequence of Formula (I), the sequence may be replaced with 5'-NGNNN-3', wherein N is each independently A, C, G, or U. As a more specific example, the sequence 5'-ACGAA-3' in the X^{c1} sequence of Formula (I) may be replaced by any one nucleotide sequence selected from the group consisting of the following sequences; however, the replacing sequence is not limited to the following sequences: 5'-AGGAA-3', 5'-AGCAA-3', 5'-AGAAA-3', 5'-AGCAU-3', 5'-AGCAG-3', 5'-AGCAC-3', 5'-AGCUA-3', 5'-AGCGA-3', 5'-AGCCA-3', 5'-UGCAA-3', 5'-UGCUA-3', 5'-UGCGA-3', 5'-UGCCA-3', 5'-GGCAA-3', 5'-GGCUA-3', 5'-GGCGA-3', 5'-GGCCA-3', 5'-CGCAA-3', 5'-CGCUA-3', 5'-CGCGA-3', and 5'-CGCCA-3'.

In another embodiment, when a sequence containing 3 or more, 4 or more, or 5 or more consecutive U residues in the X^{c1} sequence of Formula (I) is modified to another sequence, it is preferred that the corresponding nucleotides in the x^{c2} sequence (that is, at least some of which forms a complementary bond therewith) are modified so that they can form a complementary bond with the modified nucleotides. For example, when the sequence 5'-UUUUU-3' in the X^{c1} sequence is modified to 5'-GUGCU-3', it is preferred that the sequence 5'-ACGAA-3' in the x^{c2} sequence is modified to 5'-AGCAA-3'; however, complementary bonding is not necessarily required.

### (2) Modification at modification site 2 (MS2)

This section describes a modification at MS2 (FIG. 20). In an embodiment, the engineered guide RNA (gRNA) may be obtained by adding a new configuration to the gRNA found in nature, and may be such that one or more uridine residues are added to the 3'-end of the crRNA sequence, more specifically, the 3'-end of the spacer sequence included in the crRNA. Here, the 3'-end of the crRNA sequence may be the 3'-end of the guide sequence (spacer). In the present disclosure, the one or more uridine residues added to the 3'-end are also referred to herein as a "U-rich tail." The engineered gRNA comprising one or more uridines or a U-rich tail added to the 3'-end serves to increase nucleic acid cleavage or indel efficiency of the hypercompact CRISPR/Cas12f1 system for a target gene or target nucleic acid.

The term "U-rich tail" as used herein may refer not only to an RNA sequence itself that is rich in uridine (U), but also a DNA sequence encoding the same, and this may be appropriately interpreted depending on the context. The present inventors have experimentally elucidated the structure and effects of the U-rich tail sequence in detail. The U-rich tail sequence will be described in more detail with specific embodiments.

In an embodiment, the U-rich tail sequence may be represented by Ux, wherein x may be 1, 2, 3, 4, 5, 6, 7, 8, 9, 10, 11, 12, 13, 14, 15, 16, 17, 18, 19, or 20. As an example, x may be an integer within a range of two numerical values selected from the numerical values listed above. For example, x may be an integer between 1 and 6. As another example, x may be an integer between 1 and 20. In an embodiment, x may be an integer of 20 or higher.

In another embodiment, the U-rich tail sequence is represented by 5'-(UₘV)ₙUₒ-3', wherein V may be each independently A, C, or G, m and o may be integers between 1 and 20, and n may be an integer between 0 and 5. As an example, n may be 0, 1, or 2. As an example, m and o may be each independently 1, 2, 3, 4, 5, 6, 7, 8, 9, or 10.

In yet another embodiment, the U-rich tail sequence may be a U-rich tail represented by 5'-(UₘV)ₙUₒ-3', wherein (i) n is 0, o is an integer between 1 and 6, or (ii) V is each independently A or G, m and o are each independently an integer between 3 and 6, and n is an integer between 1 and 3. In a specific example, the U-rich tail may consist of any one sequence selected from the group consisting of 5'-U-3', 5'-UU-3', 5'-UUU-3', 5'-UUUU-3', 5'-UUUUU-3', 5'-UUUUUU-3', 5'-UUURUUU-3', 5'-UUURUUURUUU-3', 5'-UUUURU-3', 5'-UUUURUU-3', 5'-UUUURUUU-3', 5'-UUUURUUUU-3', 5'-UUUURUUUUU-3', and 5'-UUUURUUUUUU-3', wherein R is A or G. For example, the U-rich tail may be a sequence consisting of or comprising the sequence 5'-UUUUUUUUUU-3' (SEQ ID NO: 341), 5'-UUAUUUAUUU-3' (SEQ ID NO: 342), 5'-UUUCUAUUUU-3' (SEQ ID NO: 343), or 5'-UUAUGUUUUU-3' (SEQ ID NO: 344).

In still yet another embodiment, the U-rich tail sequence may comprise a modified uridine repeat sequence that contains a non-uridine ribonucleoside (A, C, or G) for every 1 to 5 repetitions of uridine. The modified uridine repeat sequence is particularly useful in a case of designing a vector that expresses an engineered crRNA. In an embodiment, the U-rich tail sequence may comprise a sequence in which UV, UUV, UUUV, UUUUV, and/or UUUUUV are repeated one or more times. Here, V is one of A, C or G.

In addition, the U-rich tail sequence may be a combination of the sequence represented by Ux and the sequence represented by 5'-(Uₘ)ₙ-3'. In an embodiment, the U-rich tail sequence may be represented by (U)n1-V1-(U)n2-V2-Ux. Here, V1 and V2 are each one of adenine (A), cytidine (C), and guanine (G). Here, n1 and n2 may each be an integer between 1 and 4. Here, x may be an integer between 1 and 20. In addition, the U-rich tail sequence may have a length of 1 nt, 2 nts, 3 nts, 4 nts, 5 nts, 6 nts, 7 nts, 8 nts, 9 nts, 10 nts, 11 nts, 12 nts, 13 nts, 14 nts, 15 nts, 16 nts, 17 nts, 18 nts, 19 nts, or 20 nts. In an embodiment, the U-rich tail sequence may have a length of 20 nts or longer.

In still yet another embodiment, when the engineered gRNA is expressed in a cell, the U-rich tail may be expressed as one or more sequences due to premature termination of transcription. For example, according to an embodiment, when a gRNA intended to contain a U-rich tail of the sequence 5'-UUUUAUUUUUU-3' is transcribed in a cell, four or more or five or more T residues may act as a termination sequences, and thus gRNAs containing a U-rich tail such as 5'-UUUUAUUUU-3', 5'-UUUUAUUUUUU-3', or 5'-UUUUAUUUUUU-3' may be produced simultaneously. Therefore, in the present disclosure, a U-rich tail containing four or more U residues may be understood to also include a U-rich tail sequence having a shorter length than the intended length.

In still yet another embodiment, the U-rich tail sequence may comprise additional nucleotides other than uridine, depending on the environment where the gene editing system of the present disclosure is actually used and expression environment, such as the internal environment of a eukaryotic cell or a prokaryotic cell.

### (3) Modification at modification site 3 (MS3)

This section describes a modification at MS3 (FIG. 20). As described above, MS3 refers to a region (which may be referred to as the first stem-loop region) that comprises at least a part of the nucleotides forming a stem-loop structure within a complex of the gRNA with an effector protein. The MS3 may comprise a region that does not interact with the effector protein when the gRNA and effector protein form a complex. The modification at MS3 involves removal of at least a part of first stem-loop region near the 5'-end of tracrRNA.

In an embodiment, the engineered gRNA comprises a modification in which at least a part of first stem-loop region (for example, the sequence of SEQ ID NO: 14) is deleted.

In another embodiment, the engineered gRNA comprises a modification in which at least a part of first stem-loop region on tracrRNA is deleted, wherein at least a part of the first stem-loop region to be deleted may consist of 1 to 20 nucleotides. Specifically, at least a part of the first stem-loop region may consist of 2 to 20, 3 to 20, 4 to 20, 5 to 20, 6 to 20, 7 to 20, 8 to 20, 9 to 20, 10 to 20, 11 to 20, 12 to 20, 13 to 20, 14 to 20, 15 to 20, 16 to 20, 17 to 20, 18 to 20, 19, or 20 nucleotides.

In yet another embodiment, the MS3 or the first stem-loop region is a portion corresponding to the polynucleotide indicated by X^{a} of Formula (I), wherein due to a modification in which at least a part of the first stem-loop region is deleted, X^{a} may consist of 0 to 35 (poly)nucleotides, preferably 0 to 20, 0 to 19, 0 to 18, 0 to 17, 0 to 16, 0 to 15, 0 to 14, 0 to 13, 0 to 12, 0 to 11, 0 to 10, 0 to 9, 0 to 8, 0 to 7, 0 to 6, 0 to 5, 0 to 4, 0 to 3, 0 to 2, 1 or 0 (poly)nucleotides.

In an embodiment, in the scaffold sequence of Formula (I), X^{a} may comprise the nucleotide sequence of SEQ ID NO: 14 or may comprise a nucleotide sequence having at least a part thereof, preferably a nucleotide sequence having the sequence of SEQ ID NO: 14 from which 1 to 20 nucleotides are deleted. For example, the nucleotide deletion may involve random deletion of at least 1, 2, 3, 5, 6, 7, 8, 9, 10, 11, 12, 13, 14 15, 16, 17, 18, 19, or 20 nucleotides from the sequence of SEQ ID NO: 14. As a preferred example, the nucleotide deletion may involve sequential deletion of at least 1, 2, 3, 5, 6, 7, 8, 9, 10, 11, 12, 13, 14, 15, 16, 17, 18, 19, or 20 nucleotides from the 5'-end of the sequence of SEQ ID NO: 14. More specifically, X^{a} of Formula (I) may comprise or consist of 5'-CUUCACUGAUAAAGUGGAGA-3' (SEQ ID NO: 14), 5'-UUCACUGAUAAAGUGGAGA-3' (SEQ ID NO: 15), 5'-UCACUGAUAAAGUGGAGA-3' (SEQ ID NO: 16), 5'-CACUGAUAAAGUGGAGA-3' (SEQ ID NO: 17), 5'-ACUGAUAAAGUGGAGA-3' (SEQ ID NO: 18), 5'-CUGAUAAAGUGGAGA-3' (SEQ ID NO: 19), 5'-UGAUAAAGUGGAGA-3' (SEQ ID NO: 20), 5'-GAUAAAGUGGAGA-3' (SEQ ID NO: 21), 5'-AUAAAGUGGAGA-3' (SEQ ID NO: 22), 5'-UAAAGUGGAGA-3' (SEQ ID NO: 23), 5'-AAAGUGGAGA-3' (SEQ ID NO: 24), 5'-AAGUGGAGA-3', 5'-AGUGGAGA-3', 5'-GUGGAGA-3', 5'-UGGAGA-3', 5'-GGAGA-3', 5'-GAGA-3', 5'-AGA-3', 5'-GA-3', or 5'-A-3', or X^{a} may be absent.

### (4) Modification at modification site 4 (MS4)

This section describes a modification at MS4 (FIG. 20). MS4 refers to a region spanning the 3'-end of tracrRNA and the 5'-end of crRNA, or, in a case of a single guide RNA form, a region where the sequence corresponding to tracrRNA and the sequence corresponding to crRNA form at least partial complementary bonding. MS4 may comprise at least a part of the sequence referred to as the tracrRNA-crRNA complementarity region (which may also be referred to as the fifth stem region). In the present disclosure, the tracrRNA-crRNA complementarity region may comprise both modification site 1 (MS1) and modification site 4 (MS4). The modification at MS4 comprises deletion of at least a part of the tracrRNA-crRNA complementarity region. The tracrRNA-crRNA complementarity region may comprise a part of tracrRNA and a part of crRNA. In this regard, the tracrRNA-crRNA complementarity region may comprise nucleotides such that partial nucleotides contained in tracrRNA can form complementary bonds with partial nucleotides contained in crRNA within a complex of gRNA with the nucleic acid degrading protein, and may comprise nucleotides adjacent thereto. The tracrRNA-crRNA complementarity region of tracrRNA may comprise a region that does not interact with the nucleic acid degrading protein within a complex of gRNA with the nucleic acid degrading protein.

In some embodiments, the engineered gRNA comprises deletion of at least a part of the tracrRNA-crRNA complementarity region in tracrRNA, deletion of at least a part of the tracrRNA-crRNA complementarity region in crRNA, or deletion of at least a part of the tracrRNA-crRNA complementarity region in both the tracrRNA and the crRNA.

In an embodiment, the tracrRNA-crRNA complementarity region may comprise the nucleotide sequence of SEQ ID NO: 39 and/or the nucleotide sequence of SEQ ID NO: 58.

In another embodiment, the tracrRNA-crRNA complementarity region may further comprise a linker (for example, a polynucleotide) linking the 3'-end of the tracrRNA and the 5'-end of the crRNA.

In an embodiment, the engineered gRNA comprises a modification in which a part of the tracrRNA-crRNA complementarity region is deleted, wherein the part of the complementary region to be deleted may consist of 1 to 54 nucleotides.

In another embodiment, the engineered gRNA comprises a modification in which the entire tracrRNA-crRNA complementarity region is deleted, wherein the entire complementary region to be deleted may consist of 55 nucleotides.

Specifically, at least a part of the tracrRNA-crRNA complementarity region may consist of 3 to 55, 5 to 55, 7 to 55, 9 to 55, 11 to 55, 13 to 55, 15 to 55, 17 to 55, 19 to 55, 21 to 55, 23 to 55, 25 to 55, 27 to 55, 29 to 55, 31 to 55, 33 to 55, 35 to 55, 37 to 55, 39 to 55, or 41 to 55 nucleotides, preferably 42 to 55, 43 to 55, 44 to 55, 45 to 55, 46 to 55, 47 to 55, 48 to 55, 49 to 55, 50 to 55, 51 to 55, 52 to 55, 53 to 55, 54, or 55 nucleotides.

In yet another embodiment, MS4 or the tracrRNA-crRNA complementarity region is a region corresponding to or comprising the polynucleotide indicated by X^{c1} and x^{c2} in Formula (I), in which due to the modification where at least a part of the tracrRNA-crRNA complementarity region is deleted, X^{c1} and x^{c2} may each independently consist of 0 to 35 (poly)nucleotides.

Preferably, X^{c1} may consist of 0 to 28, 0 to 27, 0 to 26, 0 to 25, 0 to 24, 0 to 23, 0 to 22, 0 to 21, 0 to 20, 0 to 19, 0 to 18, 0 to 17, 0 to 16, 0 to 15, 0 to 14, 0 to 13, 0 to 12, 0 to 11, 0 to 10, 0 to 9, 0 to 8, 0 to 7, 0 to 6, 0 to 5, 0 to 4, 0 to 3, 0 to 2, 1, or 0 (poly)nucleotides. In addition, preferably, x^{c2} may consist of 0 to 27, 0 to 26, 0 to 25, 0 to 24, 0 to 23, 0 to 22, 0 to 21, 0 to 20, 0 to 19, 0 to 18, 0 to 17, 0 to 16, 0 to 15, 0 to 14, 0 to 13, 0 to 12, 0 to 11, 0 to 10, 0 to 9, 0 to 8, 0 to 7, 0 to 6, 0 to 5, 0 to 4, 0 to 3, 0 to 2, 1, or 0 (poly)nucleotides.

In an embodiment, in the scaffold sequence of Formula (I), X^{c1} may comprise the nucleotide sequence of SEQ ID NO: 39 or a nucleotide sequence having the sequence of SEQ ID NO: 39 from which 1 to 28 nucleotides are deleted. Preferably, the nucleotide deletion may involve sequential removal of at least 1, 2, 3, 5, 6, 7, 8, 9, 10, 11, 12, 13, 14, 15, 16, 17, 18, 19, 20, 21, 22, 23, 24, 25, 26, 27, or 28 nucleotides from the 5'-end of the sequence of SEQ ID NO: 39. More specifically, X^{c1} may comprise or consist of 5'-UUCAUUUUUCCUCUCCAAUUCUGCACAA-3' (SEQ ID NO: 39), 5'-UUCAUUUUUCCUCUCCAAUUCUGCACA-3' (SEQ ID NO: 40), 5'-UUCAUUUUUCCUCUCCAAUUCUGCAC-3' (SEQ ID NO: 41), 5'-UUCAUUUUUCCUCUCCAAUUCUGCA-3' (SEQ ID NO: 42), 5'-UUCAUUUUUCCUCUCCAAUUCUGC-3' (SEQ ID NO: 43), 5'-UUCAUUUUUCCUCUCCAAUUCUG-3' (SEQ ID NO: 44), 5'-UUCAUUUUUCCUCUCCAAUUCU-3' (SEQ ID NO: 45), 5'-UUCAUUUUUCCUCUCCAAUUC-3' (SEQ ID NO: 46), 5'-UUCAUUUUUCCUCUCCAAUU-3' (SEQ ID NO: 47), 5'-UUCAUUUUUCCUCUCCAAU-3' (SEQ ID NO: 48), 5'-UUCAUUUUUCCUCUCCAA-3' (SEQ ID NO: 49), 5'-UUCAUUUUUCCUCUCCA-3' (SEQ ID NO: 50), 5'-UUCAUUUUUCCUCUCC-3' (SEQ ID NO: 51), 5'-UUCAUUUUUCCUCUC-3' (SEQ ID NO: 52), 5'-UUCAUUUUUCCUCU-3' (SEQ ID NO: 53), 5'-UUCAUUUUUCCUC-3' (SEQ ID NO: 54), 5'-UUCAUUUUUCCU-3' (SEQ ID NO: 55), 5'-UUCAUUUUUCC-3' (SEQ ID NO: 56), 5'-UUCAUUUUUC-3' (SEQ ID NO: 57), 5'-UUCAUUUUU-3', 5'-UUCAUUUU-3', 5'-UUCAUUU-3', 5'-UUCAUU-3', 5'-UUCAU-3', 5'-UUCA-3', 5'-UUC-3', 5'-UU-3', or 5'-U-3', or X^{c1} may be absent.

Here, in a case where there is a region containing 3, 4, or 5 or more uracil (U) residues in the sequence of X^{c1} from which some nucleotides have been removed, the modification at MS1 as described above may also apply. For details about MS1, see the section "(1) Modification at modification site 1 (MS1)."

In yet another embodiment, in the scaffold sequence of Formula (I), x^{c2} may comprise the nucleotide sequence of SEQ ID NO: 222 or a nucleotide sequence having the sequence of SEQ ID NO: 222 from which 1 to 27 nucleotides are deleted. Preferably, the nucleotide deletion may involve sequential removal of at least 1, 2, 3, 5, 6, 7, 8, 9, 10, 11, 12, 13, 14, 15, 16, 17, 18, 19, 20, 21, 22, 23, 24, 25, 26, or 27 nucleotides from the 5'-end of the sequence of SEQ ID NO: 222. More specifically, x^{c2} may comprise or consist of 5'-GUUGCAGAACCCGAAUAGACGAAUGAA-3' (SEQ ID NO: 58), 5'-UUGCAGAACCCGAAUAGACGAAUGAA-3' (SEQ ID NO: 59), 5'-UGCAGAACCCGAAUAGACGAAUGAA-3' (SEQ ID NO: 60), 5'-GCAGAACCCGAAUAGACGAAUGAA-3' (SEQ ID NO: 61), 5'-CAGAACCCGAAUAGACGAAUGAA-3' (SEQ ID NO: 62), 5'-AGAACCCGAAUAGACGAAUGAA-3' (SEQ ID NO: 63), 5'-GAACCCGAAUAGACGAAUGAA-3' (SEQ ID NO: 64), 5'-AACCCGAAUAGACGAAUGAA-3' (SEQ ID NO: 65), 5'-ACCCGAAUAGACGAAUGAA-3' (SEQ ID NO: 66), 5'-CCCGAAUAGACGAAUGAA-3' (SEQ ID NO: 67), 5'-CCGAAUAGACGAAUGAA-3' (SEQ ID NO: 68), 5'-CGAAUAGACGAAUGAA-3' (SEQ ID NO: 69), 5'-GAAUAGACGAAUGAA-3' (SEQ ID NO: 70), 5'-AAUAGACGAAUGAA-3' (SEQ ID NO: 71), 5'-AUAGACGAAUGAA-3' (SEQ ID NO: 72), 5'-UAGACGAAUGAA-3' (SEQ ID NO: 73), 5'-AGACGAAUGAA-3' (SEQ ID NO: 74), 5'-GACGAAUGAA-3' (SEQ ID NO: 75), 5'-ACGAAUGAA-3', 5'-CGAAUGAA-3', 5'-GAAUGAA-3', 5'-AAUGAA-3', 5'-AUGAA-3', 5'-UGAA-3', 5'-GAA-3', 5'-AA-3', or 5'-A-3', or x^{c2} may be absent.

Here, in a case where there is a sequence corresponding a sequence containing 3 or more, or 3, 4, or 5 or more uracil (U) residues in the sequence of x^{c2} from which some nucleotides have been removed, the modification at MS1 as described above may also apply. For details regarding MS1, see the section "(1) Modification at modification site 1 (MS1)."

In the scaffold sequence of Formula (I), the regions corresponding to X^{c1} and x^{c2} may each independently undergo the above-described modification. However, MS4 or the tracrRNA-crRNA complementarity region is a region where tracrRNA and crRNA form complementary bonds. For the tracrRNA and the crRNA to function as a dual guide RNA, it is preferable that the position and number of nucleotides to be deleted in each of X^{c1} and x^{c2} be identical with or similar to each other. That is, in order to preserve complementarity between the X^{c1} and x^{c2} sequences, in a case of sequentially deleting nucleotides from the 3'-end of tracrRNA in MS4 (tracrRNA-crRNA complementarity region), it is preferable to sequentially delete nucleotides from the 5'-end of crRNA. In an embodiment according to this viewpoint, deletion of the X^{c1} and x^{c2} nucleotide sequences may involve deletion of one or more pairs of complementary nucleotides.

In an embodiment, the 3'-end of X^{c1} and the 5'-end of x^{c2} in the scaffold sequence of Formula (I) may be linked by a linker (Lk) so that the gRNA is modified into a single guide RNA (sgRNA) form. Lk is a sequence that physically or chemically connects tracrRNA and crRNA, and may be a polynucleotide sequence having a length of 1 to 30 nucleotides. In an embodiment, Lk may be a sequence of 1 to 5, 5 to 10, 10 to 15, 2 to 20, 15 to 20, 20 to 25, or 25 to 30 nucleotides. For example, Lk may be, but is not limited to, 5'-GAAA-3'. As another example, Lk may be a linker comprising or consisting of 5'-UUAG-3', 5'-UGAAAA-3', 5'-UUGAAAAA-3', 5'-UUCGAAAGAA-3' (SEQ ID NO: 76), 5'-UUCAGAAAUGAA-3' (SEQ ID NO: 77), 5'-UUCAUGAAAAUGAA-3' (SEQ ID NO: 78), or 5'-UUCAUUGAAAAAUGAA-3' (SEQ ID NO: 79).

Meanwhile, while it is possible to use a linker (Lk) to make a single guide RNA (sgRNA), it is also possible to directly connect the 3'-end of tracrRNA, of which a partial sequence has been removed, to the 5'-end of crRNA of which a partial sequence has been removed.

In another embodiment, a case where X^{c1} and x^{c2} in the scaffold sequence of Formula (I) are linked by a linker may be indicated by 5'-X^{c1}-Lk-X^{c2}-3' as in Formula (I), and the 5'-X^{c1}-Lk-X^{c2}-3' may be any one nucleotide sequence selected from the group consisting of SEQ ID NOs: 80 to 86 and 5'-Lk-3' (a form in which both X^{c1} and X^{c2} are deleted), but is not limited thereto.

### (5) Modification at modification site 5 (MS5)

This section describes a modification at MS1 (FIG. 20). As described above, MS5 corresponds to a region located toward the 3'-end of tracrRNA, which is referred to as the second stem-loop region. The second stem-loop region may comprise nucleotides that form a stem structure within a complex of the guide RNA (gRNA) with nucleic acid editing protein, and may comprise nucleotides adjacent thereto. Here, the stem or stem-loop structure is distinct from the stem included in the above-described first stem-loop region.

In an embodiment, the second stem-loop region may comprise the nucleotide sequence of SEQ ID NO: 25 and/or the nucleotide sequence of SEQ ID NO: 29.

In another embodiment, MS5 or the second stem-loop region is a region comprising a (poly)nucleotide (comprising a loop of 5'-UUAG-3') that is adjacent to the polynucleotide indicated by X^{b1} and X^{b2} in Formula (I), in which due to the modification where at least the part of the second stem-loop region is deleted, X^{b1} and X^{b2} may each independently consist of 0 to 35 (poly)nucleotides.

In an embodiment, the engineered gRNA comprises a modification in which at least a part of the second stem-loop region is deleted.

In another embodiment, the engineered gRNA comprises deletion of at least a part of the second stem-loop region, wherein at least a part of the second stem-loop region to be deleted may consist of 1 to 27 nucleotides. Specifically, the at least a part of the second stem region may consist of 2 to 27, 3 to 27, 4 to 27, 5 to 27, 6 to 27, 7 to 27, 8 to 27, 9 to 27, 10 to 27, 11 to 27, 12 to 27, 13 to 27, 14 to 27, 15 to 27, 16 to 27, 17 to 27, 18 to 27, 19 to 27, 20 to 27, 21 to 27, 22 to 27, 23 to 27, 24 to 27, 25 to 27, 26, or 27 nucleotides.

Preferably, X^{b1} in Formula (I) may consist of 0 to 13, 0 to 12, 0 to 11, 0 to 10, 0 to 9, 0 to 8, 0 to 7, 0 to 6, 0 to 5, 0 to 4, 0 to 3, 0 to 2, 1, or 0 (poly)nucleotides. In addition, preferably, X^{b2} may consist of 0 to 14, 0 to 13, 0 to 12, 0 to 11, 0 to 10, 0 to 9, 0 to 8, 0 to 7, 0 to 6, 0 to 5, 0 to 4, 0 to 3, 0 to 2, 1, or 0 (poly)nucleotides.

In an embodiment, in the scaffold sequence of Formula (I), X^{b1} may comprise the nucleotide sequence of SEQ ID NO: 25 or a nucleotide sequence having the sequence of SEQ ID NO: 25 from which 1 to 13 nucleotides are deleted. Preferably, the nucleotide deletion may involve sequential removal of at least 1, 2, 3, 5, 6, 7, 8, 9, 10, 11, 12, or 13 nucleotides from the 5'-end of the sequence of SEQ ID NO: 25. More specifically, X^{b1} may comprise or consist of 5'-CAAAAGCUGUCCC-3' (SEQ ID NO: 25), 5'-CAAAAGCUGUCC-3' (SEQ ID NO: 26), 5'-CAAAAGCUGUC-3' (SEQ ID NO: 27), 5'-CAAAAGCUGU-3' (SEQ ID NO: 28), 5'-CAAAAGCUG-3', 5'-CAAAAGCU-3', 5'-CAAAAGC-3', 5'-CAAAAG-3', 5'-CAAAA-3', 5'-CAAA-3', 5'-CAA-3', 5'-CA-3', or 5'-C-3', or X^{b1} may be absent.

In another embodiment, in the scaffold sequence of Formula (I), X^{b2} may comprise the nucleotide sequence of SEQ ID NO: 29 or a nucleotide sequence having the sequence of SEQ ID NO: 29 from which 1 to 14 nucleotides are deleted. Preferably, the nucleotide deletion may involve sequential removal of at least 1, 2, 3, 5, 6, 7, 8, 9, 10, 11, 12, 13, or 14 nucleotides from the 5'-end of the sequence of SEQ ID NO: 29. More specifically, X^{b2} may comprise or consist of 5'-GGGAUUAGAACUUG-3' (SEQ ID NO: 29), 5'-GGAUUAGAACUUG-3' (SEQ ID NO: 30), 5'-GAUUAGAACUUG-3' (SEQ ID NO: 31), 5'-AUUAGAACUUG-3' (SEQ ID NO: 32), 5'-UUAGAACUUG-3' (SEQ ID NO: 33), 5'-UAGAACUUG-3', 5'-AGAACUUG-3', 5'-GAACUUG-3', 5'-AACUUG-3', 5'-ACUUG-3', 5'-CUUG-3', 5'-UUG-3', 5'-UG-3', or 5'-G-3', or X^{b2} may be absent.

In the scaffold sequence of Formula (I), the regions corresponding to X^{b1} and X^{b2} may be each independently modified. However, for normal preservation of the stem-loop structure, it is preferable that the position and number of nucleotides to be deleted in each of X^{b1} and X^{b2} be identical with or similar to each other. For example, in a case of sequentially deleting nucleotides from the 5'-end direction in X^{b1}, it is preferable to sequentially delete nucleotides from the 3'-end direction in X^{b2}. In an embodiment according to this viewpoint, the deletion of the nucleotide sequences X^{b1} and X^{b2} may be deletion of one or more pairs of complementary nucleotides.

In another embodiment, a sequence of the loop portion connecting X^{b1} and X^{b2} in the scaffold sequence of Formula (I) is indicated by 5'-UUAG-3', and this may be replaced with another sequence such as 5'-NNNN-3' and 5'-NNN-3', if necessary. Here, N is each independently A, C, G, or U. For example, the 5'-NNNN-3' may be 5'-GAAA-3', and the 5'-NNN-3' may be 5'-CGA-3'.

For example, in the scaffold sequence of Formula (I), a sequence of the loop portion connecting X^{b1} and X^{b2} is 5'-UUAG-3', and the sequence 5'-X^{b1}UUAG X^{b2}-3' in Formula (I) may comprise or consist of any one nucleotide sequence selected from the group consisting of SEQ ID NOs: 34 to 38 and 5'-UUAG-3' (a form in which both X^{b1} and X^{b2} are deleted).

### (6) Examples of gRNAs to which modifications at modification sites 1 to 5 have been applied

The engineered guide RNA disclosed herein may comprise modifications at two or more of the above-mentioned modification sites 1 (MS1) to 5 (MS5).

In an embodiment, the engineered guide RNA may comprise one or more modifications selected from the group consisting of (a1) deletion of at least a part of the first stem-loop region; (a2) deletion at least a part of the second stem-loop region; (b) deletion of at least a part of the tracrRNA-crRNA complementarity region; (c) replacement of one or more uracil (U) residues with A, G, or C in three or more, four or more, or five or more consecutive U residues when the consecutive U residues are present in the tracrRNA-crRNA complementarity region; and (d) addition of a U-rich tail to the 3'-end of the crRNA sequence. The U-rich tail sequence may be represented by 5'-(UₘV)ₙUₒ-3', wherein V is each independently A, C, or G, m and o are integers between 1 to 20, and n is an integer between 0 to 5.

For example, the engineered guide RNA may comprise (d) addition of a U-rich tail to the 3'-end of the crRNA sequence and (c) replacement of one or more uracil (U) residues with A, G, or C in three or more, four or more, or five or more consecutive U residues when the consecutive U residues are present in the tracrRNA-crRNA complementarity region.

As another example, the engineered guide RNA may comprise (d) addition of a U-rich tail to the 3'-end of the crRNA sequence, (c) replacement of one or more U with A, G or C in three or more, four or more, or five or more consecutive U residues when the consecutive U residues are present in the tracrRNA-crRNA complementarity region, and (a1) deletion of at least a part of first stem-loop region.

As yet another example, the engineered guide RNA may comprise (d) addition of a U-rich tail to the 3'-end of the crRNA sequence, (c) replacement of one or more U with A, G or C in three or more, four or more, or five or more consecutive U residues when the consecutive U residues are present in the tracrRNA-crRNA complementarity region, and (a1) deletion of at least a part of first stem-loop region.

As another example, the engineered guide RNA may comprise (d) addition of a U-rich tail to the 3'-end of the crRNA sequence, (a1) deletion of at least a part of the first stem-loop region, and (b) deletion of at least a part of the tracrRNA-crRNA complementarity region, wherein the engineered guide RNA may further comprise replacement of one or more U with A, G or C in three or more, four or more, or five or more consecutive U residues when the consecutive U residues are present in the tracrRNA-crRNA complementarity region containing partial deletion.

As another example, the engineered guide RNA may comprise (d) addition of a U-rich tail to the 3'-end of the crRNA sequence, (a1) deletion of at least a part of the first stem-loop region, (b) deletion of at least a part of the tracrRNA-crRNA complementarity region, and (a2) deletion of at least a part of the second stem-loop region, wherein the engineered guide RNA may further comprise replacement of one or more U with A, G or C in three or more, four or more, or five or more consecutive U residues when the consecutive U residues are present in the tracrRNA-crRNA complementarity region containing partial deletion.

As an example of tracrRNA to which modifications at the plurality of modification sites (MS) as described above have been applied, there is provided an engineered tracrRNA comprising the nucleotide sequence of any one of SEQ ID NOs: 87 to 132.

Specifically, the engineered tracrRNA may comprise or consist of the nucleotide sequence of SEQ ID NO: 87 (MS1), SEQ ID NO: 88 (MS1/MS3-1), SEQ ID NO: 89 (MS1/MS3-2), SEQ ID NO: 90 (MS1/MS3-3), SEQ ID NO: 91 (MS1/MS4*-1), SEQ ID NO: 92 (MS1/MS4*-2), SEQ ID NO: 93 (MS1/MS4*-3), SEQ ID NO: 94 (MS1/MS5-1), SEQ ID NO: 95 (MS1/MS5-2), SEQ ID NO: 96 (MS1/MS5-3), SEQ ID NO: 97 (MS1/MS3-3/MS4*-1), SEQ ID NO: 98 (MS1/MS3-3/MS4*-2), SEQ ID NO: 99 (MS1/MS3-3/MS4*-3), SEQ ID NO: 100 (MS1/MS4*-2/MS5-1), SEQ ID NO: 101 (MS1/MS4*-2/MS5-2), SEQ ID NO: 102 (MS1/MS4*-2/MS5-3), SEQ ID NO: 103 (MS1/MS3-3/MS5-1), SEQ ID NO: 104 (MS1/MS3-3/MS5-2), SEQ ID NO: 105 (MS1/MS3-3/MS5-3), SEQ ID NO: 106 (MS1/MS3-3/MS4*-2/MS5-3), SEQ ID NO: 107 (mature form, MF), SEQ ID NO: 108 (MF/MS3-1), SEQ ID NO: 109 (MF/MS3-2), SEQ ID NO: 110 (MF/MS3-3), SEQ ID NO: 111 (MF/MS4-1), SEQ ID NO: 112 (MF/MS4-2), SEQ ID NO: 113 (MF/MS4-3), SEQ ID NO: 114 (MF/MS5-1), SEQ ID NO: 115 (MF/MS5-2), SEQ ID NO: 116 (MF/MS5-3), SEQ ID NO: 117 (MF/MS5), SEQ ID NO: 118 (MF/MS3-3/MS4-1), SEQ ID NO: 119 (MF/MS3-3/MS4-2), SEQ ID NO: 120 (MF/MS3-3/MS4-3), SEQ ID NO: 121 (MF/MS4-3/MS5-1), SEQ ID NO: 122 (MF/MS4-3/MS5-2), SEQ ID NO: 123 (MF/MS4-3/MS5-3), SEQ ID NO: 124 (MF/MS4-3/MS5-F), SEQ ID NO: 125 (MF/MS3-3/MS5-1), SEQ ID NO: 126 (MF/MS3-3/MS5-2), SEQ ID NO: 127 (MF/MS3-3/MS5-3), SEQ ID NO: 128 (MF/MS3-3/MS5), SEQ ID NO: 129 (MF/MS3-3/MS4-3/MS5-3), SEQ ID NO: 130 (MF/MS3-3/MS4-1/MS5), SEQ ID NO: 131 (MF/MS3-3/MS4-2/MS5), or SEQ ID NO: 132 (MF/MS3-3/MS4-3/MS5).

As a more specific example, exemplary sequences of the engineered tracrRNA, which has one or more modifications at any one or more of the modification sites selected from MS1, MS3, MS4, and MS5, are provided in Table 4 below. Such an engineered tracrRNA constitutes part of the scaffold sequence of the scaffold region.

**[Table 4]**

| tracrRNA | Nucleotide sequence | SEQ ID NO |
|---|---|---|
| MS1 | CUUCACUGAUAAAGUGGAGAACCGCUUCACCAAAAGC UGUCCCUUAGGGGAUUAGAACUUGAGUGAAGGUGGG CUGCUUGCAUCAGCCUAAUGUCGAGAAGUGCUUUCUU CGGAAAGUAACCCUCGAAACAAAUUCAGUGCUCCUCU CCAAUUCUGCACAA | 87 |
| MS1/MS3-1 | GAUAAAGUGGAGAACCGCUUCACCAAAAGCUGUCCCU UAGGGGAUUAGAACUUGAGUGAAGGUGGGCUGCUUG CAUCAGCCUAAUGUCGAGAAGUGCUUUCUUCGGAAAG UAACCCUCGAAACAAAUUCAGUGCUCCUCUCCAAUUC UGCACAA | 88 |
| MS1/MS3-2 | UGGAGAACCGCUUCACCAAAAGCUGUCCCUUAGGGGA UUAGAACUUGAGUGAAGGUGGGCUGCUUGCAUCAGCC UAAUGUCGAGAAGUGCUUUCUUCGGAAAGUAACCCUC GAAACAAAUUCAGUGCUCCUCUCCAAUUCUGCACAA | 89 |
| MS1/MS3-3 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AAUUCAGUGCUCCUCUCCAAUUCUGCACAA | 90 |
| MS1/MS4*-1 | CUUCACUGAUAAAGUGGAGAACCGCUUCACCAAAAGC UGUCCCUUAGGGGAUUAGAACUUGAGUGAAGGUGGG | 91 |
| | CUGCUUGCAUCAGCCUAAUGUCGAGAAGUGCUUUCUU CGGAAAGUAACCCUCGAAACAAAUUCAGUGCUCCUCU CCAAUUC | |
| MS1/MS4*-2 | CUUCACUGAUAAAGUGGAGAACCGCUUCACCAAAAGC UGUCCCUUAGGGGAUUAGAACUUGAGUGAAGGUGGG CUGCUUGCAUCAGCCUAAUGUCGAGAAGUGCUUUCUU CGGAAAGUAACCCUCGAAACAAAUUCAGUGCUCCUCU C | 92 |
| MS1/MS4*-3 | CUUCACUGAUAAAGUGGAGAACCGCUUCACCAAAAGC UGUCCCUUAGGGGAUUAGAACUUGAGUGAAGGUGGG CUGCUUGCAUCAGCCUAAUGUCGAGAAGUGCUUUCUU CGGAAAGUAACCCUCGAAACAAAUUCAGUGCU | 93 |
| MS1/MS5-1 | CUUCACUGAUAAAGUGGAGAACCGCUUCACCAAAAGC UGUUUAGAUUAGAACUUGAGUGAAGGUGGGCUGCUU GCAUCAGCCUAAUGUCGAGAAGUGCUUUCUUCGGAAA GUAACCCUCGAAACAAAUUCAGUGCUCCUCUCCAAUU CUGCACAA | 94 |
| MS1/MS5-2 | CUUCACUGAUAAAGUGGAGAACCGCUUCACCAAAAGC UUAGGAACUUGAGUGAAGGUGGGCUGCUUGCAUCAG CCUAAUGUCGAGAAGUGCUUUCUUCGGAAAGUAACCC UCGAAACAAAUUCAGUGCUCCUCUCCAAUUCUGCACA A | 95 |
| MS1/MS5-3 | CUUCACUGAUAAAGUGGAGAACCGCUUCACCAAUUAG UUGAGUGAAGGUGGGCUGCUUGCAUCAGCCUAAUGUC GAGAAGUGCUUUCUUCGGAAAGUAACCCUCGAAACAA AUUCAGUGCUCCUCUCCAAUUCUGCACAA | 96 |
| MS1/MS3-3/MS4*-1 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AAUUCAGUGCUCCUCUCCAAUUC | 97 |
| MS1/MS3-3/MS4*-2 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AAUUCAGUGCUCCUCUC | 98 |
| MS1/MS3-3/MS4*-3 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AAUUCAGUGCU | 99 |
| MS1/MS4*-2/MS5-1 | CUUCACUGAUAAAGUGGAGAACCGCUUCACCAAAAGC UGUUUAGAUUAGAACUUGAGUGAAGGUGGGCUGCUU | 100 |
| | GCAUCAGCCUAAUGUCGAGAAGUGCUUUCUUCGGAAA GUAACCCUCGAAACAAAUUCAGUGCUCCUCUC | |
| MS1/MS4*-2/MS5-2 | CUUCACUGAUAAAGUGGAGAACCGCUUCACCAAAAGC UUAGGAACUUGAGUGAAGGUGGGCUGCUUGCAUCAG CCUAAUGUCGAGAAGUGCUUUCUUCGGAAAGUAACCC UCGAAACAAAUUCAGUGCUCCUCUC | 101 |
| MS1/MS4*-2/MS5-3 | CUUCACUGAUAAAGUGGAGAACCGCUUCACCAAUUAG UUGAGUGAAGGUGGGCUGCUUGCAUCAGCCUAAUGUC GAGAAGUGCUUUCUUCGGAAAGUAACCCUCGAAACAA AUUCAGUGCUCCUCUC | 102 |
| MS1/MS3-3/MS5-1 | ACCGCUUCACCAAAAGCUGUUUAGAUUAGAACUUGAG UGAAGGUGGGCUGCUUGCAUCAGCCUAAUGUCGAGAA GUGCUUUCUUCGGAAAGUAACCCUCGAAACAAAUUCA GUGCUCCUCUCCAAUUCUGCACAA | 103 |
| MS1/MS3-3/MS5-2 | ACCGCUUCACCAAAAGCUUAGGAACUUGAGUGAAGGU GGGCUGCUUGCAUCAGCCUAAUGUCGAGAAGUGCUUU CUUCGGAAAGUAACCCUCGAAACAAAUUCAGUGCUCC UCUCCAAUUCUGCACAA | 104 |
| MS1/MS3-3/MS5-3 | ACCGCUUCACCAAUUAGUUGAGUGAAGGUGGGCUGCU UGCAUCAGCCUAAUGUCGAGAAGUGCUUUCUUCGGAA AGUAACCCUCGAAACAAAUUCAGUGCUCCUCUCCAAU UCUGCACAA | 105 |
| MS1/MS3-3/MS4*-2/MS5-3 | ACCGCUUCACCAAUUAGUUGAGUGAAGGUGGGCUGCU UGCAUCAGCCUAAUGUCGAGAAGUGCUUUCUUCGGAA AGUAACCCUCGAAACAAAUUCAGUGCUCCUCUC | 106 |
| Mature Form(MF) | CUUCACUGAUAAAGUGGAGAACCGCUUCACCAAAAGC UGUCCCUUAGGGGAUUAGAACUUGAGUGAAGGUGGG CUGCUUGCAUCAGCCUAAUGUCGAGAAGUGCUUUCUU CGGAAAGUAACCCUCGAAACAAAUUCAUUU | 107 |
| MF/MS3-1 | GAUAAAGUGGAGAACCGCUUCACCAAAAGCUGUCCCU UAGGGGAUUAGAACUUGAGUGAAGGUGGGCUGCUUG CAUCAGCCUAAUGUCGAGAAGUGCUUUCUUCGGAAAG UAACCCUCGAAACAAAUUCAUUU | 108 |
| MF/MS3-2 | UGGAGAACCGCUUCACCAAAAGCUGUCCCUUAGGGGA UUAGAACUUGAGUGAAGGUGGGCUGCUUGCAUCAGCC UAAUGUCGAGAAGUGCUUUCUUCGGAAAGUAACCCUC GAAACAAAUUCAUUU | 109 |
| MF/MS3-3 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA | 110 |
| | AAUUCAUUU | |
| MF/MS4-1 | CUUCACUGAUAAAGUGGAGAACCGCUUCACCAAAAGC UGUCCCUUAGGGGAUUAGAACUUGAGUGAAGGUGGG CUGCUUGCAUCAGCCUAAUGUCGAGAAGUGCUUUCUU CGGAAAGUAACCCUCGAAACAAAUUCAU | 111 |
| MF/MS4-2 | CUUCACUGAUAAAGUGGAGAACCGCUUCACCAAAAGC UGUCCCUUAGGGGAUUAGAACUUGAGUGAAGGUGGG CUGCUUGCAUCAGCCUAAUGUCGAGAAGUGCUUUCUU CGGAAAGUAACCCUCGAAACAAAUUC | 112 |
| MF/MS4-3 | CUUCACUGAUAAAGUGGAGAACCGCUUCACCAAAAGC UGUCCCUUAGGGGAUUAGAACUUGAGUGAAGGUGGG CUGCUUGCAUCAGCCUAAUGUCGAGAAGUGCUUUCUU CGGAAAGUAACCCUCGAAACAAA | 113 |
| MF/MS5-1 | CUUCACUGAUAAAGUGGAGAACCGCUUCACCAAAAGC UGUUUAGAUUAGAACUUGAGUGAAGGUGGGCUGCUU GCAUCAGCCUAAUGUCGAGAAGUGCUUUCUUCGGAAA GUAACCCUCGAAACAAAUUCAUUU | 114 |
| MF/MS5-2 | CUUCACUGAUAAAGUGGAGAACCGCUUCACCAAAAGC UUUAGAGAACUUGAGUGAAGGUGGGCUGCUUGCAUC AGCCUAAUGUCGAGAAGUGCUUUCUUCGGAAAGUAAC CCUCGAAACAAAUUCAUUU | 115 |
| MF/MS5-3 | CUUCACUGAUAAAGUGGAGAACCGCUUCACCAAUUAG UUGAGUGAAGGUGGGCUGCUUGCAUCAGCCUAAUGUC GAGAAGUGCUUUCUUCGGAAAGUAACCCUCGAAACAA AUUCAUUU | 116 |
| MEF/MSS5 | CUUCACUGAUAAAGUGGAGAACCGCUUCACUUAGAGU GAAGGUGGGCUGCUUGCAUCAGCCUAAUGUCGAGAAG UGCUUUCUUCGGAAAGUAACCCUCGAAACAAAUUCAU UU | 117 |
| MF/MS3-3/MS4-1 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AAUUCAU | 118 |
| MF/MS3-3/MS4-2 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA AAUUC | 119 |
| MF/MS3-3/MS4-3 | ACCGCUUCACCAAAAGCUGUCCCUUAGGGGAUUAGAA CUUGAGUGAAGGUGGGCUGCUUGCAUCAGCCUAAUGU CGAGAAGUGCUUUCUUCGGAAAGUAACCCUCGAAACA | 120 |
| | AA | |
| MF/MS4-3/MS5-1 | CUUCACUGAUAAAGUGGAGAACCGCUUCACCAAAAGC UGUUUAGAUUAGAACUUGAGUGAAGGUGGGCUGCUU GCAUCAGCCUAAUGUCGAGAAGUGCUUUCUUCGGAAA GUAACCCUCGAAACAAA | 121 |
| MF/MS4-3/MS5-2 | CUUCACUGAUAAAGUGGAGAACCGCUUCACCAAAAGC UUUAGAGAACUUGAGUGAAGGUGGGCUGCUUGCAUC AGCCUAAUGUCGAGAAGUGCUUUCUUCGGAAAGUAAC CCUCGAAACAAA | 122 |
| MF/MS4-3/MS5-3 | CUUCACUGAUAAAGUGGAGAACCGCUUCACCAAUUAG UUGAGUGAAGGUGGGCUGCUUGCAUCAGCCUAAUGUC GAGAAGUGCUUUCUUCGGAAAGUAACCCUCGAAACAA A | 123 |
| MF/MS4-3/MS5 | CUUCACUGAUAAAGUGGAGAACCGCUUCACUUAGAGU GAAGGUGGGCUGCUUGCAUCAGCCUAAUGUCGAGAAG UGCUUUCUUCGGAAAGUAACCCUCGAAACAAA | 124 |
| MF/MS3-3/MS5-1 | ACCGCUUCACCAAAAGCUGUUUAGAUUAGAACUUGAG UGAAGGUGGGCUGCUUGCAUCAGCCUAAUGUCGAGAA GUGCUUUCUUCGGAAAGUAACCCUCGAAACAAAUUCA UUU | 125 |
| MF/MS3-3/MS5-2 | ACCGCUUCACCAAAAGCUUUAGAGAACUUGAGUGAAG GUGGGCUGCUUGCAUCAGCCUAAUGUCGAGAAGUGCU UUCUUCGGAAAGUAACCCUCGAAACAAAUUCAUUU | 125 |
| MF/MS3-3/MS5-3 | ACCGCUUCACCAAUUAGUUGAGUGAAGGUGGGCUGCU UGCAUCAGCCUAAUGUCGAGAAGUGCUUUCUUCGGAA AGUAACCCUCGAAACAAAUUCAUUU | 127 |
| MF/MS3-3/MS5 | ACCGCUUCACUUAGAGUGAAGGUGGGCUGCUUGCAUC AGCCUAAUGUCGAGAAGUGCUUUCUUCGGAAAGUAAC CCUCGAAACAAAUUCAUUU | 128 |
| MF/MS3-3/MS4-3/MS5-3 | ACCGCUUCACCAAUUAGUUGAGUGAAGGUGGGCUGCU UGCAUCAGCCUAAUGUCGAGAAGUGCUUUCUUCGGAA AGUAACCCUCGAAACAAA | 129 |
| MF/MS3-3/MS4-1/MS5 | ACCGCUUCACUUAGAGUGAAGGUGGGCUGCUUGCAUC AGCCUAAUGUCGAGAAGUGCUUUCUUCGGAAAGUAAC CCUCGAAACAAAUUCAU | 130 |
| MF/MS3-3/MS4-2/MS5 | ACCGCUUCACUUAGAGUGAAGGUGGGCUGCUUGCAUC AGCCUAAUGUCGAGAAGUGCUUUCUUCGGAAAGUAAC CCUCGAAACAAAUUC | 131 |
| MF/MS3-3/MS4-3/MS5 | ACCGCUUCACUUAGAGUGAAGGUGGGCUGCUUGCAUC AGCCUAAUGUCGAGAAGUGCUUUCUUCGGAAAGUAAC CCUCGAAACAAA | 132 |

In addition, as an example of crRNA to which modifications at the plurality of modification sites (MS) as described above have been applied, there is provided an engineered crRNA comprising the nucleotide sequence of any one of SEQ ID NOs: 133 to 148. Specifically, the engineered crRNA of the present disclosure may comprise or consist of the nucleotide of SEQ ID NO: 133 (MS1), SEQ ID NO: 134 (MS1/MS4*-1), SEQ ID NO: 135 (MS1/MS4*-2), SEQ ID NO: 136 (MS1/MS4*-3), SEQ ID NO: 137 (mature form; MF), SEQ ID NO: 138 (MF/MS4-1), SEQ ID NO: 139 (MF/MS4-2), SEQ ID NO: 140 (MF/MS4-3), SEQ ID NO: 141 (MS1/MS2), SEQ ID NO: 142 (MS1/MS2/MS4*-1), SEQ ID NO: 143 (MS1/MS2/MS4*-2), SEQ ID NO: 144 (MS1/MS2/MS4*-3), SEQ ID NO: 145 (MF/MS2), SEQ ID NO: 146 (MF/MS2/MS4-1), SEQ ID NO: 147 (MF/MS2/MS4-2), or SEQ ID NO: 148 (MF/MS2/MS4-3).

In some embodiments, exemplary sequences of the engineered crRNA, which has one or more modifications at any one or more modification sites selected from MS1, MS2, and MS4 are provided in Table 5 below.

**[Table 5]**

| crRNA | Nucleotide sequence | SEQ ID NO |
|---|---|---|
| MS1 | GUUGCAGAACCCGAAUAGAGCAAUGAAGGAAUGCAAC | 133 |
| MS1/MS4*-1 | GAACCCGAAUAGAGCAAUGAAGGAAUGCAAC | 134 |
| MS1/MS4*-2 | GAAUAGAGCAAUGAAGGAAUGCAAC | 135 |
| MS1/MS4*-3 | AGCAAUGAAGGAAUGCAAC | 136 |
| MF | GAAUGAAGGAAUGCAAC | 137 |
| MF/MS4-1 | AUGAAGGAAUGCAAC | 138 |
| MF/MS4-2 | GAAGGAAUGCAAC | 139 |
| MF/MS4-3 | GGAAUGCAAC | 140 |
| MS1/MS2 | GUUGCAGAACCCGAAUAGAGCAAUGAAGGAAUGCAAC NNNNNNNNNNNNNNNNNNNNUUUUAUUUUUU | 141 |
| MS1/MS2/MS4*-1 | GAACCCGAAUAGAGCAAUGAAGGAAUGCAACNNNNN | 142 |
| | NNNNNNNNNNNNNNNNUUUUAUUUUUU | |
| MS1/MS2/MS4* -2 | GAAUAGAGCAAUGAAGGAAUGCAACNNNNNNNNNNN NNNNNNNNNUUUUAUUUUUU | 143 |
| MS1/MS2/MS4* -3 | AGCAAUGAAGGAAUGCAACNNNNNNNNNNNNNNNNNN NNUUUUAUUUUUU | 144 |
| MF/MS2 | GAAUGAAGGAAUGCAACNNNNNNNNNNNNNNNNNN NNUUUUAUUUUUU | 145 |
| MF/MS2/MS4-1 | AUGAAGGAAUGCAACNNNNNNNNNNNNNNNNNNNNU UUUAUUUUUU | 146 |
| MF/MS2/MS4-2 | GAAGGAAUGCAACNNNNNNNNNNNNNNNNNNNNUUU UAUUUUUU | 147 |
| MF/MS2/MS4-3 | GGAAUGCAACNNNNNNNNNNNNNNNNNNNNUUUUAU UUUUU | 148 |

In Table 5, indication of a guide sequence (spacer) is omitted from all crRNA sequences omit unless necessary, and the sequence indicated by 'NNNNNNNNNNNNNNNNNNNN' indicates any guide sequence (spacer) that can hybridize with a target sequence in a target gene. The guide sequence may be appropriately designed by a person skilled in the art depending on a desired target gene and/or a target sequence in the target gene as described above, and therefore is not limited to a specific sequence of a particular length. In another embodiment, the scaffold region of the engineered gRNA may comprise tracrRNA comprising or consisting of any one nucleotide sequence selected from the group consisting of SEQ ID NOs: 87 to 132; and crRNA comprising or consisting of any one nucleotide sequence selected from the group consisting of SEQ ID NOs: 133 to 148.

In another embodiment, the guide RNA of the present disclosure may comprise a sequence of a scaffold region of a nucleotide sequence selected from the group consisting of SEQ ID NOs: 149 to 186. Here, the scaffold region of the nucleotide sequence means the remaining region excluding the spacer region present at the 3'-end portion of crRNA (for example, the region indicated by 5'-NNNNNNNNNNNNNNNNNN-3' in the nucleotide sequence).

In another embodiment, when the engineered gRNA of the present disclosure is in the form of a single guide RNA (sgRNA), the scaffold region of the engineered sgRNA may comprise or consist of any one nucleotide sequence selected from the group consisting of SEQ ID NOs: 149 to 186. Here, the 5'-NNNNNNNNNNNNNNNNNNNN-3', 5'-NNNNNNNNNNNNNNNNNNNUUUUAUUUU-3', or 5'-NNNNNNNNNNNNNNNNNNNUUUUAUUUUU-3' sequence present at the 3'-end of SEQ ID NOs: 149 to 186 is excluded.

For example, the engineered sgRNA may be sgRNA of SEQ ID NO: 149 comprising a modification at MS1, sgRNA of SEQ ID NO: 150 comprising modifications at MS1/MS2, sgRNA of SEQ ID NO: 151 comprising modifications at MS1/MS2/MS3, sgRNA of SEQ ID NO: 152 comprising modifications at MS2/MS3/MS4, or sgRNA of SEQ ID NO: 153 comprising modifications at MS2/MS3/MS4/MS5. Here, in the nucleotide sequences of SEQ ID NOs: 150 to 153, the sequence indicated by 5'-NNNNNNNNNNNNNNNNNNNN-3' refers to a guide sequence.

In another specific example, the engineered sgRNA may be sgRNA comprising or consisting of the nucleotide sequence of SEQ ID NO: 154 (MS1/MS3-1), SEQ ID NO: 155 (MS1/MS3-2), SEQ ID NO: 156 (MS1/MS3-3), SEQ ID NO: 157 (MS1/MS4*-1), SEQ ID NO: 158 (MS1/MS4*-2), SEQ ID NO: 159 (MS1/MS4*-3), SEQ ID NO: 160 (MS1/MS5-1), SEQ ID NO: 161 (MS1/MS5-2), SEQ ID NO: 162 (MS1/MS5-3), SEQ ID NO: 163 (MS1/MS2/MS4*-2), SEQ ID NO: 164 (MS1/MS3-3/MS4*-2), SEQ ID NO: 165 (MS1/MS2/MS5-3), SEQ ID NO: 166 (MS1/MS3-3/MS5-3), SEQ ID NO: 167 (MS1/MS4*-2/MS5-3), SEQ ID NO: 168 (MS1/MS2/MS3-3/MS4*-2), SEQ ID NO: 169 (MS1/MS2/MS3-3/MS5-3), SEQ ID NO: 170 (MS1/MS2/MS4*-2/MS5-3), SEQ ID NO: 171 (MS1/MS3-3/MS4*-2/MS5-3), or SEQ ID NO: 172 (MS1/MS2/MS3-3/MS4*-2/MS5-3). Here, the sequence indicated by 5'-NNNNNNNNNNNNNNNNNNNN-3' in the nucleotide sequences refers to a guide sequence.

In addition, the sgRNA may be sgRNA comprising or consisting of the nucleotide sequence of SEQ ID NO: 173, which is a mature form (abbreviated as MF) of sgRNA.

In another embodiment, there is provided an exemplary sgRNA which comprises partial modification of the nucleotide sequence of the MF sgRNA. Specifically, the MF sgRNA may be sgRNA comprising or consisting of the nucleotide sequence of SEQ ID NO: 174 (MS3-1), SEQ ID NO: 175 (MS3-2), SEQ ID NO: 176 (MS3-3), SEQ ID NO: 177 (MS4-1), SEQ ID NO: 178 (MS4-2), SEQ ID NO: 179 (MS4-3), SEQ ID NO: 180 (MS5-1), SEQ ID NO: 181 (MS5-2), SEQ ID NO: 182 (MS5-3), SEQ ID NO: 183 (MS3-3/MS4-3), SEQ ID NO: 184 (MS3-3/MS5-3), SEQ ID NO: 185 (MS4-3/MS5-3), or SEQ ID NO: 186 (MS3-3/MS4-3/MS5-3). Here, the sequence indicated by 5'-NNNNNNNNNNNNNNNNNNNN-3' in the nucleotide sequences refers to a guide sequence.

In a preferred embodiment, the engineered sgRNA may consist of the nucleotide sequence of SEQ ID NO: 151 (Cas12f1 ver3.0), SEQ ID NO: 152 (Cas12f1 ver4.0), or SEQ ID NO: 153 (Cas12f1 ver4.1). Here, in the nucleotide sequences, the sequence indicated by 5'-NNNNNNNNNNNNNNNNNN-3' refers to a guide sequence.

The engineered sgRNA is an engineered gRNA having one or more of the five modification sites (MS1, MS2, MS3, MS4, and MS5) as shown in FIG. 20, and the specific sequences thereof are shown in Table 6 below.

**[Table 6]**

| gRNA | Sequence (5' to 3') | SEQ ID NO |
|---|---|---|
| Canonical sgRNA | CUUCACUGAUAAAGUGGAGAACCGCUUCACCAAAAGCU GUCCCuuagGGGAUUAGAACUUGAGUGAAGGUGGGCUGC UUGCAUCAGCCUAAUGUCGAGAAGUGCUUUCUUCGGAA AGUAACCCUCGAAACAAAUUCAUUUUUCCUCUCCAAUU CUGCACAAgaaaGUUGCAGAACCCGAAUAGacgaaUGAAGG AAUGCAACNNNNNNNNNNNNNNNNNNNN | 13 |
| MS1 | CUUCACUGAUAAAGUGGAGAACCGCUUCACCAAAAGCU GUCCCUUAGGGGAUUAGAACUUGAGUGAAGGUGGGCUG CUUGCAUCAGCCUAAUGUCGAGAAGUGCUUUCUUCGGA AAGUAACCCUCGAAACAAAUUCAGUGCUCCUCUCCAAU UCUGCACAAgaaaGUUGCAGAACCCGAAUAGAGCAAUGAA GGAAUGCAACNNNNNNNNNNNNNNNNNNNN | 149 |
| MS1/MS2 | CUUCACUGAUAAAGUGGAGAACCGCUUCACCAAAAGCU GUCCCUUAGGGGAUUAGAACUUGAGUGAAGGUGGGCUG CUUGCAUCAGCCUAAUGUCGAGAAGUGCUUUCUUCGGA AAGUAACCCUCGAAACAAAUUCAGUGCUCCUCUCCAAU UCUGCACAAgaaaGUUGCAGAACCCGAAUAGAGCAAUGAA GGAAUGCAACNNNNNNNNNNNNNNNNNNNNUUUUAUUU UUU | 150 |
| MS1/MS2/MS 3(ge3.0) | ACCGCUUCACCAAAAGCUGUCCCUUAGGGGAUUAGAAC UUGAGUGAAGGUGGGCUGCUUGCAUCAGCCUAAUGUCG AGAAGUGCUUUCUUCGGAAAGUAACCCUCGAAACAAAU UCAGUGCUCCUCUCCAAUUCUGCACAAgaaaGUUGCAGAA CCCGAAUAGAGCAAUGAAGGAAUGCAACNNNNNNNNNN | 151 |
| | NNNNNNNNNNUUUUAUUUUUU | |
| MS2/MS3/MS 4(ge4.0) | ACCGCUUCACCAAAAGCUGUCCCUUAGGGGAUUAGAAC UUGAGUGAAGGUGGGCUGCUUGCAUCAGCCUAAUGUCG AGAAGUGCUUUCUUCGGAAAGUAACCCUCGAAACAAAga aaGGAAUGCAACNNNNNNNNNNNNNNNNNNNNUUUUAU UUUUU | 152 |
| MS2/MS3/MS 4/MS5(ge4.1) | ACCGCUUCACUUAGAGUGAAGGUGGGCUGCUUGCAUCA GCCUAAUGUCGAGAAGUGCUUUCUUCGGAAAGUAACCC UCGAAACAAAgaaaGGAAUGCAACNNNNNNNNNNNNNNNN NNNNUUUUAUUUUUU | 153 |
| MS1/MS3-1 | GAUAAAGUGGAGAACCGCUUCACCAAAAGCUGUCCCUU AGGGGAUUAGAACUUGAGUGAAGGUGGGCUGCUUGCAU CAGCCUAAUGUCGAGAAGUGCUUUCUUCGGAAAGUAAC CCUCGAAACAAAUUCAGUGCUCCUCUCCAAUUCUGCACA AgaaaGUUGCAGAACCCGAAUAGAGCAAUGAAGGAAUGC AACNNNNNNNNNNNNNNNNNNNN | 154 |
| MS1/MS3-2 | UGGAGAACCGCUUCACCAAAAGCUGUCCCUUAGGGGAU UAGAACUUGAGUGAAGGUGGGCUGCUUGCAUCAGCCUA AUGUCGAGAAGUGCUUUCUUCGGAAAGUAACCCUCGAA ACAAAUUCAGUGCUCCUCUCCAAUUCUGCACAAgaaaGUU GCAGAACCCGAAUAGAGCAAUGAAGGAAUGCAACNNNN NNNNNNNNNNNNNNNN | 155 |
| MS1/MS3-3 | ACCGCUUCACCAAAAGCUGUCCCUUAGGGGAUUAGAAC UUGAGUGAAGGUGGGCUGCUUGCAUCAGCCUAAUGUCG AGAAGUGCUUUCUUCGGAAAGUAACCCUCGAAACAAAU UCAGUGCUCCUCUCCAAUUCUGCACAAgaaaGUUGCAGAA CCCGAAUAGAGCAAUGAAGGAAUGCAACNNNNNNNNNN NNNNNNNNNN | 156 |
| MS1/MS4*-1 | CUUCACUGAUAAAGUGGAGAACCGCUUCACCAAAAGCU GUCCCUUAGGGGAUUAGAACUUGAGUGAAGGUGGGCUG CUUGCAUCAGCCUAAUGUCGAGAAGUGCUUUCUUCGGA AAGUAACCCUCGAAACAAAUUCAGUGCUCCUCUCCAAU UCgaaaGAACCCGAAUAGAGCAAUGAAGGAAUGCAACNN NNNNNNNNNNNNNNNNNN | 157 |
| MS1/MS4*-2 | CUUCACUGAUAAAGUGGAGAACCGCUUCACCAAAAGCU GUCCCUUAGGGGAUUAGAACUUGAGUGAAGGUGGGCUG CUUGCAUCAGCCUAAUGUCGAGAAGUGCUUUCUUCGGA AAGUAACCCUCGAAACAAAUUCAGUGCUCCUCUCgaaaGA AUAGAGCAAUGAAGGAAUGCAACNNNNNNNNNNNNNNN NNNNN | 158 |
| MS1/MS4*-3 | CUUCACUGAUAAAGUGGAGAACCGCUUCACCAAAAGCU GUCCCUUAGGGGAUUAGAACUUGAGUGAAGGUGGGCUG CUUGCAUCAGCCUAAUGUCGAGAAGUGCUUUCUUCGGA AAGUAACCCUCGAAACAAAUUCAGUGCUgaaaAGCAAUGA AGGAAUGCAACNNNNNNNNNNNNNNNNNNNN | 159 |
| MS1/MS5-1 | CUUCACUGAUAAAGUGGAGAACCGCUUCACCAAAAGCU GUuuagAUUAGAACUUGAGUGAAGGUGGGCUGCUUGCAU CAGCCUAAUGUCGAGAAGUGCUUUCUUCGGAAAGUAAC CCUCGAAACAAAUUCAGUGCUCCUCUCCAAUUCUGCACA AgaaaGUUGCAGAACCCGAAUAGAGCAAUGAAGGAAUGC AACNNNNNNNNNNNNNNNNNNNN | 160 |
| MS1/MS5-2 | CUUCACUGAUAAAGUGGAGAACCGCUUCACCAAAAGCuu agGAACUUGAGUGAAGGUGGGCUGCUUGCAUCAGCCUAA UGUCGAGAAGUGCUUUCUUCGGAAAGUAACCCUCGAAA CAAAUUCAGUGCUCCUCUCCAAUUCUGCACAAgaaaGUUG CAGAACCCGAAUAGAGCAAUGAAGGAAUGCAACNNNNN NNNNNNNNNNNNNNN | 161 |
| MS1/MS5-3 | CUUCACUGAUAAAGUGGAGAACCGCUUCACCAAuuagUU GAGUGAAGGUGGGCUGCUUGCAUCAGCCUAAUGUCGAG AAGUGCUUUCUUCGGAAAGUAACCCUCGAAACAAAUUC AGUGCUCCUCUCCAAUUCUGCACAAgaaaGUUGCAGAACC CGAAUAGAGCAAUGAAGGAAUGCAACNNNNNNNNNNNN NNNNNNNN | 162 |
| MS1/MS2/MS 4*-2 | CUUCACUGAUAAAGUGGAGAACCGCUUCACCAAAAGCU GUCCCUUAGGGGAUUAGAACUUGAGUGAAGGUGGGCUG CUUGCAUCAGCCUAAUGUCGAGAAGUGCUUUCUUCGGA AAGUAACCCUCGAAACAAAUUCAGUGCUCCUCUCgaaaGA AUAGAGCAAUGAAGGAAUGCAACNNNNNNNNNNNNNNN NNNNNUUUUAUUUU | 163 |
| MS1/MS3-3/MS4*-2 | ACCGCUUCACCAAAAGCUGUCCCUUAGGGGAUUAGAAC UUGAGUGAAGGUGGGCUGCUUGCAUCAGCCUAAUGUCG AGAAGUGCUUUCUUCGGAAAGUAACCCUCGAAACAAAU UCAGUGCUCCUCUCgaaaGAAUAGAGCAAUGAAGGAAUGC AACNNNNNNNNNNNNNNNNNNNN | 164 |
| MS1/MS2/MS 5-3 | CUUCACUGAUAAAGUGGAGAACCGCUUCACCAAuuagUU GAGUGAAGGUGGGCUGCUUGCAUCAGCCUAAUGUCGAG AAGUGCUUUCUUCGGAAAGUAACCCUCGAAACAAAUUC AGUGCUCCUCUCCAAUUCUGCACAAgaaaGUUGCAGAACC CGAAUAGAGCAAUGAAGGAAUGCAACNNNNNNNNNNNNN NNNNNNNUUUUAUUUU | 165 |
| MS1/MS3-3/MS5-3 | ACCGCUUCACCAAuuagUUGAGUGAAGGUGGGCUGCUUG | 166 |
| | CAUCAGCCUAAUGUCGAGAAGUGCUUUCUUCGGAAAGU AACCCUCGAAACAAAUUCAGUGCUCCUCUCCAAUUCUGC ACAAgaaaGUUGCAGAACCCGAAUAGAGCAAUGAAGGAA UGCAACNNNNNNNNNNNNNNNNNNNN | |
| MS1/MS4*-2/MS5-3 | CUUCACUGAUAAAGUGGAGAACCGCUUCACCAAuuagUU GAGUGAAGGUGGGCUGCUUGCAUCAGCCUAAUGUCGAG AAGUGCUUUCUUCGGAAAGUAACCCUCGAAACAAAUUC AGUGCUCCUCUCgaaaGAAUAGAGCAAUGAAGGAAUGCA ACNNNNNNNNNNNNNNNNNNNN | 167 |
| MS1/MS2/MS 3-3/MS4*-2 | ACCGCUUCACCAAAAGCUGUCCCuuagGGGAUUAGAACUU GAGUGAAGGUGGGCUGCUUGCAUCAGCCUAAUGUCGAG AAGUGCUUUCUUCGGAAAGUAACCCUCGAAACAAAUUC AGUGCUCCUCUCgaaaGAAUAGAGCAAUGAAGGAAUGCA ACNNNNNNNNNNNNNNNNNNNNUUUUAUUUU | 168 |
| MS1/MS2/MS 3-3/MS5-3 | ACCGCUUCACCAAuuagUUGAGUGAAGGUGGGCUGCUUG CAUCAGCCUAAUGUCGAGAAGUGCUUUCUUCGGAAAGU AACCCUCGAAACAAAUUCAGUGCUCCUCUCCAAUUCUGC ACAAgaaaGUUGCAGAACCCGAAUAGAGCAAUGAAGGAA UGCAACNNNNNNNNNNNNNNNNNNNNUUUUAUUUU | 169 |
| MS1/MS2/MS 4*-2/MS5-3 | CUUCACUGAUAAAGUGGAGAACCGCUUCACCAAuuagUU GAGUGAAGGUGGGCUGCUUGCAUCAGCCUAAUGUCGAG AAGUGCUUUCUUCGGAAAGUAACCCUCGAAACAAAUUC AGUGCUCCUCUCgaaaGAAUAGAGCAAUGAAGGAAUGCA ACNNNNNNNNNNNNNNNNNNNNUUUUAUUUU | 170 |
| MS1/MS3-3/MS4*-2/MS5-3 | ACCGCUUCACCAAuuagUUGAGUGAAGGUGGGCUGCUUG CAUCAGCCUAAUGUCGAGAAGUGCUUUCUUCGGAAAGU AACCCUCGAAACAAAUUCAGUGCUCCUCUCgaaaGAAUAG AGCAAUGAAGGAAUGCAACNNNNNNNNNNNNNNNNNNN N | 171 |
| MS1/MS2/MS 3-3/MS4*-2/MS5-3 | ACCGCUUCACCAAuuagUUGAGUGAAGGUGGGCUGCUUG CAUCAGCCUAAUGUCGAGAAGUGCUUUCUUCGGAAAGU AACCCUCGAAACAAAUUCAGUGCUCCUCUCgaaaGAAUAG AGCAAUGAAGGAAUGCAACNNNNNNNNNNNNNNNNNNN NUUUUAUUUU | 172 |

In addition, a mature form gRNA was produced by removing the modification site MS 1 from the canonical gRNA, and the specific sequences thereof are shown in Table 7.

**[Table 7]**

| gRNA | Sequence (5' to 3') | SEQ ID NO |
|---|---|---|
| Mature form gRNA | CUUCACUGAUAAAGUGGAGAACCGCUUCACCAAAAGCU GUCCCuuagGGGAUUAGAACUUGAGUGAAGGUGGGCUGC UUGCAUCAGCCUAAUGUCGAGAAGUGCUUUCUUCGGAA AGUAACCCUCGAAACAAAUUCAUUUgaaaGAAUGAAGGA AUGCAACNNNNNNNNNNNNNNNNNNNN | 173 |
| MS3-1 | GAUAAAGUGGAGAACCGCUUCACCAAAAGCUGUCCCuua gGGGAUUAGAACUUGAGUGAAGGUGGGCUGCUUGCAUC AGCCUAAUGUCGAGAAGUGCUUUCUUCGGAAAGUAACC CUCGAAACAAAUUCAUUUgaaaGAAUGAAGGAAUGCAAC NNNNNNNNNNNNNNNNNNNN | 174 |
| MS3-2 | UGGAGAACCGCUUCACCAAAAGCUGUCCCuuagGGGAUU AGAACUUGAGUGAAGGUGGGCUGCUUGCAUCAGCCUAA UGUCGAGAAGUGCUUUCUUCGGAAAGUAACCCUCGAAA CAAAUUCAUUUgaaaGAAUGAAGGAAUGCAACNNNNNNN NNNNNNNNNNNNN | 175 |
| MS3-3 | ACCGCUUCACCAAAAGCUGUCCCuuagGGGAUUAGAACU UGAGUGAAGGUGGGCUGCUUGCAUCAGCCUAAUGUCGA GAAGUGCUUUCUUCGGAAAGUAACCCUCGAAACAAAUU CAUUUgaaaGAAUGAAGGAAUGCAACNNNNNNNNNNNNN NNNNNNN | 176 |
| MS4-1 | CUUCACUGAUAAAGUGGAGAACCGCUUCACCAAAAGCU GUCCCuuagGGGAUUAGAACUUGAGUGAAGGUGGGCUGC UUGCAUCAGCCUAAUGUCGAGAAGUGCUUUCUUCGGAA AGUAACCCUCGAAACAAAUUCAUgaaaAUGAAGGAAUGC AACNNNNNNNNNNNNNNNNNNNN | 177 |
| MS4-2 | CUUCACUGAUAAAGUGGAGAACCGCUUCACCAAAAGCU GUCCCuuagGGGAUUAGAACUUGAGUGAAGGUGGGCUGC UUGCAUCAGCCUAAUGUCGAGAAGUGCUUUCUUCGGAA AGUAACCCUCGAAACAAAUUCgaaaGAAGGAAUGCAACN NNNNNNNNNNNNNNNNNNN | 178 |
| MS4-3 | CUUCACUGAUAAAGUGGAGAACCGCUUCACCAAAAGCU GUCCCuuagGGGAUUAGAACUUGAGUGAAGGUGGGCUGC UUGCAUCAGCCUAAUGUCGAGAAGUGCUUUCUUCGGAA AGUAACCCUCGAAACAAAgaaaGGAAUGCAACNNNNNNN NNNNNNNNNNNNN | 179 |
| MS5-1 | CUUCACUGAUAAAGUGGAGAACCGCUUCACCAAAAGCU GUuuagAUUAGAACUUGAGUGAAGGUGGGCUGCUUGCAU CAGCCUAAUGUCGAGAAGUGCUUUCUUCGGAAAGUAAC CCUCGAAACAAAUUCAUUUgaaaGAAUGAAGGAAUGCAA | 180 |
| | CNNNNNNNNNNNNNNNNNNNNNN | |
| MS5-2 | CUUCACUGAUAAAGUGGAGAACCGCUUCACCAAAAGCU uuagAGAACUUGAGUGAAGGUGGGCUGCUUGCAUCAGCC UAAUGUCGAGAAGUGCUUUCUUCGGAAAGUAACCCUCG AAACAAAUUCAUUUgaaaGAAUGAAGGAAUGCAACNNNN NNNNNNNNNNNNNNNNN | 181 |
| MS5-3 | CUUCACUGAUAAAGUGGAGAACCGCUUCACCAAuuagUU GAGUGAAGGUGGGCUGCUUGCAUCAGCCUAAUGUCGAG AAGUGCUUUCUUCGGAAAGUAACCCUCGAAACAAAUUC AUUUgaaaGAAUGAAGGAAUGCAACNNNNNNNNNNNNNN NNNNNN | 182 |
| MS3-3/MS4-3 | ACCGCUUCACCAAAAGCUGUCCCuuagGGGAUUAGAACU UGAGUGAAGGUGGGCUGCUUGCAUCAGCCUAAUGUCGA GAAGUGCUUUCUUCGGAAAGUAACCCUCGAAACAAAgaa aGGAAUGCAACNNNNNNNNNNNNNNNNNNNNNN | 183 |
| MS3-3/MS5-3 | ACCGCUUCACCAAuuagUUGAGUGAAGGUGGGCUGCUUG CAUCAGCCUAAUGUCGAGAAGUGCUUUCUUCGGAAAGU AACCCUCGAAACAAAUUCAUUUgaaaGAAUGAAGGAAUG CAACNNNNNNNNNNNNNNNNNNNN | 184 |
| MS4-3/MS5-3 | CUUCACUGAUAAAGUGGAGAACCGCUUCACCAAuuagUU GAGUGAAGGUGGGCUGCUUGCAUCAGCCUAAUGUCGAG AAGUGCUUUCUUCGGAAAGUAACCCUCGAAACAAAgaaa GGAAUGCAACNNNNNNNNNNNNNNNNNNNN | 185 |
| MS3-3/MS4-3/MS5-3 | ACCGCUUCACCAAuuagUUGAGUGAAGGUGGGCUGCUUG CAUCAGCCUAAUGUCGAGAAGUGCUUUCUUCGGAAAGU AACCCUCGAAACAAAgaaaGGAAUGCAACNNNNNNNNNN NNNNNNNNNN | 186 |

### (7) Additional sequence

The engineered tracrRNA of the present disclosure may optionally further comprise an additional sequence. The additional sequence may be located at the 3'-end of the engineered tracrRNA. In addition, the additional sequence may be located at the 5'-end of the engineered tracrRNA. For example, the additional sequence may be located at the 5'-end of the first stem-loop region.

The additional sequence may consist of 1 to 40 nucleotides. In an embodiment, the additional sequence may be any nucleotide sequence or a randomly arranged nucleotide sequence. For example, the additional sequence may be 5'-AUAAAGGUGA-3' (SEQ ID NO: 187).

In addition, the additional sequence may be a known nucleotide sequence. For example, the additional sequence may be a hammerhead ribozyme nucleotide sequence. Here, the hammerhead ribozyme nucleotide sequence may be 5'-CUGAUGAGUCCGUGAGGACGAAACGAGUAAGCUCGUC-3' (SEQ ID NO: 188) or 5'-CUGCUCGAAUGAGCAAAGCAGGAGUGCCUGAGUAGUC-3' (SEQ ID NO: 189). The sequences listed above are merely examples, and the additional sequence is not limited thereto.

### (8) Chemical modification

In some embodiments, the engineered tracrRNA or engineered crRNA included in the engineered gRNA may have chemical modification in at least one nucleotide, if necessary. Here, the chemical modification may be a modification in various covalent bonds that may occur in a nucleotide base and/or sugar portion.

In an embodiment, the chemical modification may be methylation, halogenation, acetylation, phosphorylation, phosphorothioate (PS) linkage, locked nucleic acid (LNA), 2'-O-methyl 3'phosphorothioate (MS), or 2'-O-methyl 3'thioPACE (MSP). The above example is a simple example and the modification is not limited thereto.

In a case of using the hypercompact gene editing system comprising a complex of the engineered gRNA with the engineered Cas protein of the present disclosure, indel efficiency for a target gene or target nucleic acid in a cell is significantly improved compared to a case of using the guide RNA found in nature, so that a large-scale deletion effect may be exhibited. In addition, the engineered Cas protein of the present disclosure has an advantage of having altered PAM sequence specificity, leading to an expanded targetable genomic range.

Above all, the engineered gRNA may involve optimized length for high efficiency and resulting cost reduction in gRNA synthesis, creation of additional space or capacity in a case of being inserted into a viral vector, normal expression of tracrRNA, increased expression of operable gRNA, increased gRNA stability, increased stability of complex of gRNA with Cas protein, induction of formation of complex of gRNA with Cas protein at high efficiency, and increased cleavage efficiency of target nucleic acid or expanded targetable genomic range by hypercompact gene editing system comprising complex of gRNA with Cas protein.

The engineered guide RNA according to the embodiment of the present disclosure may be a single guide RNA or dual guide RNA. The dual guide RNA means that the guide RNA is composed of two RNA molecules: tracrRNA and crRNA. The single guide RNA (sgRNA) means that the 3'-end of tracrRNA and the 5'-end of crRNA are connected via a linker.

In an embodiment, the engineered single guide RNA (sgRNA) may further comprise a linker sequence, and the tracrRNA sequence and the crRNA sequence may be connected via the linker sequence. Preferably, this may include a case where the 3'-end of the tracrRNA-crRNA complementarity sequence in the tracrRNA and the 5'-end of the tracrRNA-crRNA complementarity sequence in the crRNA, which are contained in the engineered scaffold sequence, may be connected via a linker. More preferably, the tracrRNA-crRNA complementarity regions of the tracrRNA and the crRNA may be connected to each other, at the 3'-end of the tracrRNA and the 5'-end of the crRNA, by the linker 5'-GAAA-3'. For details regarding the linker, see the description of Lk of Formula (I).

In an embodiment, a sequence of the single guide RNA is such that the tracrRNA sequence, the linker sequence, the crRNA sequence, and the U-rich tail sequence are sequentially linked in a 5' to 3' direction. A part of the tracrRNA sequence and at least a part of the CRISPR RNA repeat sequence included in the crRNA sequence have sequences complementary to each other.

In addition, the engineered guide RNA according to the embodiment of the present disclosure may be a dual guide RNA in which tracrRNA and crRNA form separate RNA molecules. Here, a part of the tracrRNA and a part of the crRNA may have sequences complementary to each other so that a double-stranded RNA is formed. More specifically, in the dual guide RNA, a part containing the 3'-end of the tracrRNA and a part containing the CRISPR RNA repeat sequence of the crRNA may form a double strand. The engineered guide RNA may bind to Cas12f1 or a variant protein thereof to form a complex of the guide RNA with the protein. This complex recognizes a target sequence complementary to the guide sequence included in the crRNA sequence, which allows for editing of a target gene or target nucleic acid comprising the target sequence.

In an embodiment, the tracrRNA sequence may comprise a complementary sequence having 0 to 20 mismatches with the CRISPR RNA repeat sequence. Preferably, the tracrRNA sequence may comprise a complementary sequence having 0 to 8 or 8 to 12 mismatches with the CRISPR RNA repeat sequence.

### V. Nucleic acid

According to another aspect of the present disclosure, there is provided a nucleic acid encoding the engineered Cas protein of the present disclosure as described above or a nucleic acid encoding the guide RNA of the present disclosure as described above. In an embodiment, the nucleic acid may comprise or consist of DNA. In another embodiment, the nucleic acid may comprise or consist of RNA, such as an mRNA. In another embodiment, the nucleic acid may comprise a codon-optimized sequence for a target cell (for example, a cell intended for genome editing).

### VI. Vector or vector system

According to yet another aspect of the present disclosure, there is provided a vector system, comprising at least one vector that comprises:(i) a first nucleic acid construct to which a nucleotide sequence encoding the engineered Cas protein of the present disclosure as described above is operably linked; and (ii) a second nucleic acid construct to which a nucleotide sequence encoding the guide RNA as disclosed herein is operably linked, the guide RNA comprising a guide sequence hybridizable with a target sequence adjacent to a PAM.

In an embodiment, the first and second nucleic acid constructs may be located in the same vector or different vectors. Preferably, the first and second nucleic acid constructs may be located in the same vector.

In an embodiment, the engineered Cas protein may be a fusion protein as described above.

In an embodiment, the vector system may further comprise a nucleotide sequence encoding an additional expression element that is desired to be expressed as needed by those skilled in the art. For example, the additional expression element may be a tag. As another example, the additional expression element may be a herbicide resistance gene such as glyphosate, glufosinate ammonium, or phosphinothricin, an antibiotic resistance gene such as ampicillin, kanamycin, G418, bleomycin, hygromycin, or chloramphenicol.

In another embodiment, the vector system needs to comprise one or more regulatory and/or control components so that it is directly expressed in a cell. Specifically, the regulatory and/or control components may include, but are not limited to, a promoter, an enhancer, an intron, a polyadenylation signal, a Kozak consensus sequence, an internal ribosome entry site (IRES), a splice acceptor, a 2A sequence, and/or a replication origin. The replication origin may be, but is not limited to, an f1 origin of replication, an SV40 origin of replication, a pMB 1 origin of replication, an adeno origin of replication, an AAV origin of replication, and/or a BBV origin of replication.

In another embodiment, in order to express, in a cell, the nucleic acid sequence encoding the components included in the vector system, a promoter sequence may need to be operably linked to the sequence encoding each component so that an RNA transcription factor can be activated in the cell. The promoter sequence may be designed differently depending on the corresponding RNA transcription factor or expression environment, and is not limited as long as it can properly express the components of the gene editing system of the present disclosure in a cell.

For example, the promoter sequence may be a promoter that promotes transcription of RNA polymerase RNA Pol I, Pol II, or Pol III. Specifically, the promoter may be one of U6 promoter, EFS promoter, EF1-α promoter, H1 promoter, 7SK promoter, CMV promoter, LTR promoter, Ad MLP promoter, HSV promoter, SV40 promoter, CBA promoter, or RSV promoter.

In another embodiment, when a sequence of the vector comprises the promoter sequence, transcription of a sequence operably linked to the promoter is induced by an RNA transcription factor. The vector may comprise a termination signal that induces termination of transcription of the RNA transcription factor. The termination signal may vary depending on the type of the promoter sequence. Specifically, when the promoter is a U6 or H1 promoter, the promoter recognizes a TTTTT (T5) or TTTTTT (T6) sequence, which is a thymidine (T) repeat sequence, as a termination signal.

The sequence of the engineered guide RNA provided herein may comprise a U-rich tail sequence at its 3'-end. Accordingly, the sequence encoding the engineered guide RNA comprises a T-rich sequence corresponding to the U-rich tail sequence at its 3'-end. As described above, some promoter sequences recognize a thymidine (T) repeat sequence, for example, a sequence consisting of five or more consecutive thymidine (T) residues, as a termination signal, and therefore, in some cases, the T-rich sequence may be recognized as a termination signal. In other words, when the vector sequence provided herein comprises a sequence encoding the engineered guide RNA, a sequence encoding the U-rich tail sequence included in the engineered gRNA sequence may be used as a termination signal.

In an embodiment, when the vector sequence comprises a U6 or H1 promoter sequence and a sequence encoding the engineered guide RNA operably linked thereto, a sequence portion that encodes the U-rich tail sequence included in the guide RNA sequence may be recognized as a termination signal. Specifically, the U-rich tail sequence may comprise a sequence consisting of five or more consecutive uridine (U) residues.

In an embodiment, the vector may be a viral vector. Specifically, the viral vector may be at least one selected from the group consisting of a retrovirus vector, a lentivirus vector, an adenovirus vector, an adeno-associated virus vector, a vaccinia virus vector, a poxvirus vector, a herpes simplex virus vector, and a phagemid vector. Preferably, the viral vector may be an adeno-associated virus vector (AAV). In addition, the viral vector includes, but is not limited to, a SIN lentivirus vector, a retrovirus vector, a foamy virus vector, an adenovirus vector, an adeno-associated virus (AAV) vector, a hybrid vector and/or a plasmid transposon (for example, the Sleeping Beauty transposon system), or an integrase-based vector system.

In another embodiment, the vector may be a non-viral vector. Specifically, the non-viral vector may be at least one selected from the group consisting of, but not limited to, plasmid, naked DNA, DNA complex, mRNA (transcript), and amplicon. For example, the plasmid may be selected from the group consisting of pcDNA series, pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19.

The term "naked DNA" refers to DNA (for example, histone-free DNA) that encodes a protein, such as Cas12f1 or a variant thereof of the present disclosure, cloned into a suitable expression vector (for example, plasmid) in an appropriate orientation for expression.

The term "amplicon," when used with respect to a nucleic acid, means a product of copying the nucleic acid, wherein the product has a nucleotide sequence that is identical with or complementary to at least a portion of the nucleotide sequence of the nucleic acid. For example, an amplicon may be produced by any of a variety of amplification methods that use a nucleic acid or an amplicon thereof, as a template including polymerase extension, polymerase chain reaction (PCR), rolling circle amplification (RCA), multi-displacement amplification (MDA), ligation extension, or ligation chain reaction. The amplicon may be a nucleic acid molecule having a single copy of a particular nucleotide sequence (for example, a PCR product) or multiple copies of the nucleotide sequence (for example, a concatemeric product of RCA).

The vector disclosed herein may be designed in the form of a linear or circular vector. In a case where the vector is a linear vector, RNA transcription is terminated at the 3'-end even if a sequence of the linear vector does not separately comprise a termination signal. However, in a case where the vector is a circular vector, RNA transcription is not terminated unless a sequence of the circular vector separately comprises a termination signal. Therefore, when using a circular vector, a termination signal corresponding to a transcription factor related to each promoter sequence has to be included in order for the vector to express an intended target.

In an embodiment, the viral vector or non-viral vector may be delivered by a delivery system such as liposomes, polymeric nanoparticles (for example, lipid nanoparticles), oil-in-water nanoemulsions, or combinations thereof, or in the form of a virus.

### VII. Recombinant virus

According to still yet another aspect of the present disclosure, there is provided a recombinant virus or recombinant virus particle produced by the vector system provided in the present disclosure.

In an embodiment, the viral vector may be, for example, at least one selected from the group consisting of a retrovirus vector, a lentivirus vector, an adenovirus vector, an adeno-associated virus vector, a vaccinia virus vector, a poxvirus vector, a herpes simplex virus vector, and a phagemid vector. Preferably, the viral vector may be an adeno-associated virus vector.

In another embodiment, the virus may be selected from the group consisting of a retrovirus, a lentivirus, an adenovirus, an adeno-associated virus, a vaccinia virus, a poxvirus, a herpes simplex virus, and a phage.

In yet another embodiment, the phage may be selected from the group consisting of λgt4λB, λ-charon, λΔz1, and M13.

In order to efficiently deliver the gene editing system into a target cell or target site via a virus, in particular, an adeno-associated virus (AAV), it is important to design a size of the nucleotide sequence encoding all components of the editing system to be within 4.7 kb that is a packaging limit of AAV. This has an advantage in that in a case where the engineered Cas protein and the (engineered) guide RNA of the present disclosure are used, a very small size thereof allows sufficient packaging by an AAV delivery vehicle even if additional components are further included.

### VIII. Gene editing system (CRISPR/Cas system) or composition

According to still yet another aspect of the present disclosure, there is provided a CRISPR/Cas system or composition, comprising (i) at least one engineered Cas 12f protein or a nucleic acid encoding the protein, and (ii) at least one guide RNA or a nucleic acid encoding the guide RNA.

In an embodiment, the engineered Cas12f protein may be any one of the engineered Cas12f proteins disclosed herein and described in detail above.

In another embodiment, the engineered Cas12f protein may be in the form of a fusion protein disclosed herein and described in detail above.

In yet another embodiment, the guide RNA may be any one of the wild-type guide RNA or the engineered guide RNA disclosed herein and described in detail above.

In still yet another embodiment, the system or composition may comprise a nucleic acid encoding the engineered Cas protein as disclosed herein and a nucleotide sequence encoding the nucleic acid as disclosed herein. For the nucleic acids, see the details as described above.

In still yet another embodiment, the engineered Cas12f protein and the guide RNA may be included in a complex form, for example, in the form of a ribonucleoprotein particle (RNP). The complex may comprise a guide RNA and two Cas12f1 or a variant protein thereof (see Satoru N. Takeda et al., Molecular Cell, 81, 1-13, (2021)). The complex may be formed by an interaction between the guide RNA and the Cas12f1 molecule.

In still yet another embodiment, the composition may be a pharmaceutical composition. In an embodiment, the pharmaceutical composition may further comprise a pharmaceutically acceptable carrier or excipient. In another embodiment, the pharmaceutical composition may be formulated according to the mode of administration to be used. With respect to the pharmaceutically acceptable carrier or excipient, and the formulation method, materials and/or methods well known in the art may be used.

In an embodiment, the composition may comprise a gene transfer enhancer. The gene transfer enhancer may be a polyanion, a polycation (including poly-L-glutamic acid (LGS)), or a lipid.

In an embodiment, the composition comprising one or more vectors included in the above-described vector system may comprise a gene transfer enhancer, such as a lipid, a liposome (including lecithin liposomes, or other liposomes known in the art), a DNA-liposome mixture, a calcium ion, a viral protein, a polyanion, a polycation, or a nanoparticle, or other known gene transfer enhancer. Preferably, the gene transfer enhancer is a polyanion, a polycation (for example, poly-L-glutamic acid (LGS)), or lipid.

### IX. Method for modifying nucleic acid

According to still yet another aspect of the present disclosure, there is provided a method for modifying a nucleic acid in a cell, comprising bringing, into contact with the cell, the engineered Cas protein as disclosed herein and a guide RNA, the system or composition as disclosed herein, the vector system as disclosed herein, or the recombinant virus as disclosed herein. The engineered Cas protein forms a complex with the guide RNA; and when the complex binds to a target nucleic acid, the target nucleic acid is modified.

In an embodiment, the modifying a nucleic acid in a cell comprises editing the target nucleic acid in the cell.

In an embodiment, the modifying a nucleic acid in a cell comprises regulating expression of the target nucleic acid in the cell.

In an embodiment, the modifying a nucleic acid in a cell comprises targeting the target nucleic acid in a cell.

In an embodiment, the bringing-into-contact may be performed *in vitro, in vivo,* or *ex vivo.*

In an embodiment, the cell may be a plant cell, a non-human animal cell, or a human cell. In addition, the cell may be a eukaryotic cell or a prokaryotic cell.

### [Exemplary embodiments]

### [Embodiment 1]

An engineered Cas protein, comprising amino acid substitution(s) at one or more amino acid residues selected from the group consisting of amino acids at positions 159, 164, 170, 174, 184, 188, 191, 225, 230, and 272 with respect to SEQ ID NO: 1 or at amino acid residue(s) corresponding thereto, wherein the engineered Cas protein comprises an amino acid sequence having at least 80% sequence identity to the sequence represented by SEQ ID NO: 1.

### [Embodiment 2]

The engineered Cas protein of the above-described embodiment,
wherein the substitution(s) comprises amino acid substitution(s) at one or more amino acid residues selected from the group consisting of amino acids at positions 170, 174, 184, 188, 191, 225, 230, and 272 or at amino acid residue(s) corresponding thereto, wherein the engineered Cas protein shows altered protospacer-adjacent motif (PAM) recognition specificity.

### [Embodiment 3]

The engineered Cas protein of any one of the above-described embodiments,
wherein the substitution(s) comprises amino acid substitution(s) at one or more amino acid residues selected from the group consisting of amino acids at positions 159 and 164 or at amino acid residue(s) corresponding thereto, wherein the engineered Cas protein has an altered editing window for PAM-proximal regions.

### [Embodiment 4]

The engineered Cas protein of any one of the above-described embodiments,
wherein the substitution(s) further comprises amino acid substitution(s) at one or more amino acid residues selected from the group consisting of amino acids at positions 159 and 164 or at amino acid residue(s) corresponding thereto.

### [Embodiment 5]

The engineered Cas protein of any one of the above-described embodiments,
wherein the substitution(s) comprises one or more amino acid substitutions selected from the group consisting of following (1) to (10):
(1) 159W;
(2) 164Y;
(3) 170C or 170T;
(4) 174H, 174A, 174E, 174K, 174N, 174R, or 174T;
(5) 184H, 184N, 184R, 184S, or 184T;
(6) 188G, 188H, 188K, 188N, 188Q, 188R, 188S, 188T, or 188V;
(7) 191G, 191H, 191K, 191Q, or 191W;
(8) 225F or 225T;
(9) 230A, 230H, 230I, 230S, or 230T; and
(10) 272C, 272K, or 272R.

### [Embodiment 6]

The engineered Cas protein of any one of the above-described embodiments,
wherein the substitution(s) comprises one or more substitutions selected from the group consisting of following (1) to (10):
(1) I159W;
(2) S164Y;
(3) S170C or S170T;
(4) Y174H, Y174A, Y174E, Y174K, Y174N, Y174R, or Y174T;
(5) A184H, A184N, A184R, A184S, or A184T;
(6) S188G, S188H, S188K, S188N, S188Q, S188R, S188S, S188T, or S188V;
(7) R191G, R191H, R191K, R191Q, or R191W;
(8) Q225F or Q225T;
(9) Y230A, Y230H, Y230I, Y230S, or Y230T; and
(10) Q272C, Q272K, or Q272R.

### [Embodiment 7]

The engineered Cas protein of any one of the above-described embodiments,
wherein the substitution(s) comprises one or more amino acid substitutions selected from the group consisting of the amino acid substitutions described in Table 1.

### [Embodiment 8]

The engineered Cas protein of any one of the above-described embodiments,
wherein the engineered Cas protein recognizes a PAM sequence that a wild-type Cas12f protein either cannot recognize or poorly recognizes.

### [Embodiment 9]

The engineered Cas protein of any one of the above-described embodiments,
wherein the engineered Cas protein recognizes at least one PAM sequence selected from the group consisting of 5'-TVTN-3', 5'-TTVV-3', 5'-TGGG-3', and 5'-TTTN-3' (wherein N is A, T, G, or C, and V is A, G, or C).

### [Embodiment 10]

The engineered Cas protein of any one of the above-described embodiments,
wherein the engineered Cas protein is an engineered Un1Cas12f1 protein or an engineered CWCas12f protein.

### [Embodiment 11]

The engineered Cas protein of any one of the above-described embodiments,
wherein the engineered Cas protein further comprises at least one substitution selected from the group consisting of I159W and S164Y, and
the engineered Cas protein is an engineered Un1Cas12f1 protein or an engineered CWCas12f protein.

### [Embodiment 12]

The engineered Cas protein of any one of the above-described embodiments,
wherein the engineered Cas protein has eliminated DNA cleavage activity.

### [Embodiment 13]

The engineered Cas protein of any one of the above-described embodiments,
wherein the engineered Cas protein further comprises amino acid substitution(s) at one or more amino acid residues selected from the group consisting of amino acids at positions 354, 450, 518, and 538 with respect to SEQ ID NO: 1 or at amino acid residue(s) corresponding thereto.

### [Embodiment 14]

The engineered Cas protein of any one of the above-described embodiments,
wherein the substitution(s) further comprises at least one amino acid substitution selected from following (a) to (d):
(a) 354A, 354Q, 354L, 354W, or 354V;
(b) 450A, 450Q, 450L, 450W, or 450V;
(c) 518A, 518Q, 518L, 518W, or 518V; and
(d) 538A, 538Q, 538L, 538W, or 538V.

### [Embodiment 15]

The engineered Cas protein of any one of the above-described embodiments,
wherein the engineered Cas protein further comprises deletion, insertion, substitution, or addition of at least one amino acid and is capable of forming a complex with a guide RNA.

### [Embodiment 16]

A fusion protein comprising (i) the engineered Cas protein of any one of the above-described embodiments, (ii) a functional domain, and optionally (iii) a linker.

### [Embodiment 17]

The fusion protein of the above-described embodiment,
wherein the functional domain has nuclease activity, nickase activity, recombinase activity, deaminase activity, methyltransferase activity, methylase activity, acetylase activity, acetyltransferase activity, transcriptional activation activity, transcriptional inhibition activity, or reverse transcriptase activity.

### [Embodiment 18]

The fusion protein of any one of the above-described embodiments,
wherein the functional domain has deaminase activity.

### [Embodiment 19]

The fusion protein of any one of the above-described embodiments,
wherein the functional domain comprises at least one expression regulatory domain.

### [Embodiment 20]

The fusion protein of any one of the above-described embodiments,
wherein the expression regulatory domain is selected from the group consisting of VP64, VPR, KRAB, MeCP2, DNMT, HAT, HDAC, TET, and p300.

### [Embodiment 21]

A polynucleotide encoding the engineered Cas protein of any one of the above-described embodiments or the fusion protein of any one of the above-described embodiments.

### [Embodiment 22]

An engineered CRISPR/Cas system or composition, comprising:
(i) the engineered Cas protein of any one of the above-described embodiments, or a nucleic acid encoding the protein, and
(ii) a guide RNA comprising a guide sequence hybridizable to a target sequence adjacent to a PAM, or a nucleic acid encoding the guide RNA.

### [Embodiment 23]

The system or composition of any one of the above-described embodiments,
wherein the guide RNA is an engineered guide RNA.

### [Embodiment 24]

The system or composition of any one of the above-described embodiments,
wherein the guide RNA comprises a U-rich tail sequence linked to the 3'-end of the guide sequence, and the U-rich tail is represented by 5'-(UₘV)ₙUₒ-3', wherein V is each independently A, C, or G; m and o are integers between 1 and 20; and n is an integer between 0 and 5.

### [Embodiment 25]

The system or composition of any one of the above-described embodiments,
wherein the guide RNA comprises an engineered scaffold region, and the engineered scaffold region comprises a nucleotide sequence having at least 50% sequence identity to a scaffold region of a wild-type Cas12f1 guide RNA sequence, which sequentially comprises, from the 5'-end, a first stem-loop region, a second stem-loop region, a third stem-loop region, a fourth stem-loop region, and a tracrRNA-crRNA complementarity region, wherein the guide RNA comprises at least one modification selected from the group consisting of following (1) to (4) with respect to the wild-type Cas12f1 guide RNA sequence:
(1) deletion of at least a part of the first stem-loop region;
(2) deletion of at least a part of the second stem-loop region;
(3) deletion of at least a part of the tracrRNA-crRNA complementarity region; and
(4) replacement of one or more uracil (U) residues with A, G, or C in three or more consecutive U residues when the consecutive U residues are present in the tracrRNA-crRNA complementarity region.

### [Embodiment 26]

The system or composition of any one of the above-described embodiments,
wherein the wild-type Cas12f1 guide RNA comprises tracrRNA comprising the nucleotide sequence of SEQ ID NO: 11 and crRNA comprising the nucleotide sequence of SEQ ID NO: 12.

### [Embodiment 27]

The system or composition of any one of the above-described embodiments,
wherein the engineered scaffold region comprises a sequence having at least 80% sequence identity to a sequence represented by Formula (I):

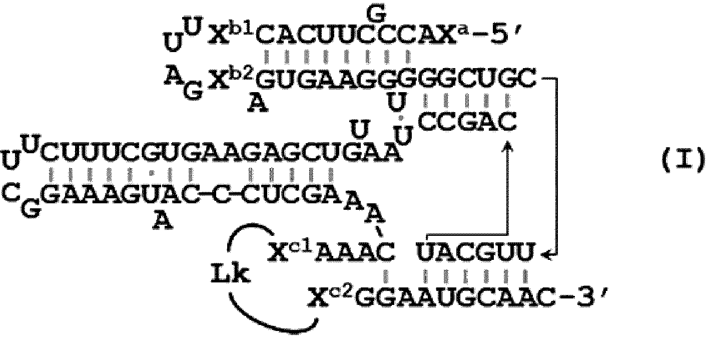

in Formula (I),
X^{a} comprises the nucleotide sequence of SEQ ID NO: 14 or a nucleotide sequence having the sequence of SEQ ID NO: 14 from which 1 to 20 nucleotides are deleted,
X^{b1} comprises the nucleotide sequence of SEQ ID NO: 25 or a nucleotide sequence having the sequence of SEQ ID NO: 25 from which 1 to 13 nucleotides are deleted,
X^{b2} comprises the nucleotide sequence of SEQ ID NO: 29 or a nucleotide sequence having the sequence of SEQ ID NO: 29 from which 1 to 14 nucleotides are deleted,
X^{c1} comprises the nucleotide sequence of SEQ ID NO: 39 or a nucleotide sequence having the sequence of SEQ ID NO: 39 from which 1 to 28 nucleotides are deleted,
x^{c2} comprises the nucleotide sequence of SEQ ID NO: 58 or a nucleotide sequence having the sequence of SEQ ID NO: 58 from which 1 to 27 nucleotides are deleted, and
Lk is a polynucleotide linker of 2 to 20 nucleotides in length or absent.

### [Embodiment 28]

The system or composition of any one of the above-described embodiments,
wherein in a case where three or more consecutive uracil (U) residues are present in a sequence of X^{c1}, the sequence of X^{c1} comprises a modification in which at least one U residue thereof is replaced with A, G, or C.

### [Embodiment 29]

The system or composition of any one of the above-described embodiments,
wherein the deletion in the nucleotide sequence of X^{a}, the deletion in the nucleotide sequences of X^{b1} and X^{b2}, and/or the deletion in the nucleotide sequences of X^{c1} and x^{c2} comprises deletion of one or more pairs of complementary nucleotides.

### [Embodiment 30]

The system or composition of any one of the above-described embodiments,
wherein the sequence 5'-X^{b1}LTUAGX^{b2}-3' in Formula (I) is selected from the group consisting of SEQ ID NOs: 34 to 38 and 5'-UUAG-3'.

### [Embodiment 31]

The system or composition of any one of the above-described embodiments,
wherein the sequence 5'-X^{c1}-Lk-x^{c2}-3' in Formula (I) is selected from the group consisting of SEQ ID NOs: 80 to 86 and 5'-Lk-3'.

### [Embodiment 32]

The system or composition of any one of the above-described embodiments,
wherein Lk comprises a nucleotide sequence selected from the group consisting of 5'-GAAA-3', 5'-UUAG-3', 5'-UGAAAA-3', 5'-UUGAAAAA-3', 5'-UUCGAAAGAA-3' (SEQ ID NO: 76), 5'-UUCAGAAAUGAA-3' (SEQ ID NO: 77), 5'-UUCAUGAAAAUGAA-3' (SEQ ID NO: 78), and 5'-UUCAUUGAAAAAUGAA-3' (SEQ ID NO: 79).

### [Embodiment 33]

The system or composition of any one of the above-described embodiments,
wherein the scaffold region comprises an engineered tracrRNA consisting of a nucleotide sequence selected from the group consisting of SEQ ID NOs: 87 to 132 and/or an engineered crRNA consisting of a nucleotide sequence selected from the group consisting of SEQ ID NOs: 133 to 148.

### [Embodiment 34]

The system or composition of any one of the above-described embodiments,
wherein the guide RNA comprises a scaffold region sequence consisting of a nucleotide sequence selected from the group consisting of SEQ ID NOs: 13 and 149 to 186.

### [Embodiment 35]

The system or composition of any one of the above-described embodiments,
wherein the engineered Cas12 protein forms a complex with the guide RNA.

### [Embodiment 36]

A vector system, comprising at least one vector that comprises:
(i) a first nucleic acid construct to which a nucleotide sequence encoding the engineered Cas protein of any one of the above-described embodiments is operably linked; and
(ii) a second nucleic acid construct to which a nucleotide sequence encoding a guide RNA is operably linked, the guide RNA comprising a guide sequence hybridizable with a target sequence adjacent to a PAM,
wherein the nucleic acid constructs (i) and (ii) are located in the same vector or different vectors.

### [Embodiment 37]

The vector system of the above-described embodiment,
wherein the guide RNA comprises a U-rich tail sequence linked to the 3'-end of the guide sequence, and the U-rich tail is represented by 5'-(UₘV)ₙUₒ-3', wherein V is each independently A, C, or G; m and o are integers between 1 and 20; and n is an integer between 0 and 5.

### [Embodiment 38]

The vector system of any one of the above-described embodiments,
wherein the guide RNA comprises an engineered scaffold region, and the engineered scaffold region comprises a nucleotide sequence having at least 50% sequence identity to a scaffold region of a wild-type Cas12f1 guide RNA sequence, which sequentially comprises, from the 5'-end, a first stem-loop region, a second stem-loop region, a third stem-loop region, a fourth stem-loop region, and a tracrRNA-crRNA complementarity region, wherein the guide RNA comprises at least one modification selected from the group consisting of the following (1) to (4) with respect to the wild-type Cas12f1 guide RNA sequence:
(1) deletion of at least a part of the first stem-loop region;
(2) deletion of at least a part of the second stem-loop region;
(3) deletion of at least a part of the tracrRNA-crRNA complementarity region; and
(4) replacement of one or more uracil (U) residues with A, G, or C in three or more consecutive U residues when the consecutive U residues are present in the tracrRNA-crRNA complementarity region.

### [Embodiment 39]

The vector system of any one of the above-described embodiments,
wherein the wild-type Cas12f1 guide RNA comprises tracrRNA comprising the nucleotide sequence of SEQ ID NO: 11 and crRNA comprising the nucleotide sequence of SEQ ID NO: 12.

### [Embodiment 40]

The vector system of any one of the above-described embodiments,
wherein the engineered scaffold region comprises a sequence having at least 80% sequence identity to a sequence represented by Formula (I): 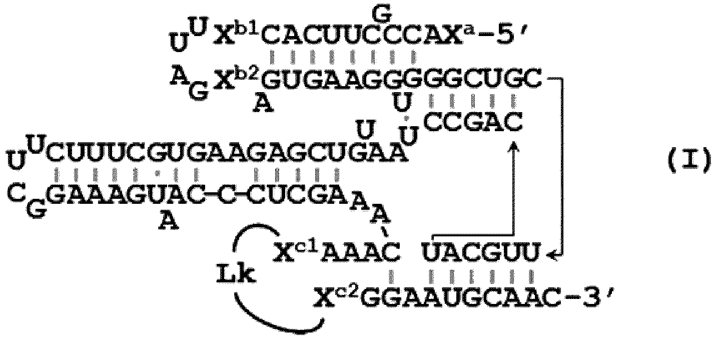 in Formula (I),
X^{a} comprises the nucleotide sequence of SEQ ID NO: 14 or a nucleotide sequence having the sequence of SEQ ID NO: 14 from which 1 to 20 nucleotides are deleted,
X^{b1} comprises the nucleotide sequence of SEQ ID NO: 25 or a nucleotide sequence having the sequence of SEQ ID NO: 25 from which 1 to 13 nucleotides are deleted,
X^{b2} comprises the nucleotide sequence of SEQ ID NO: 29 or a nucleotide sequence having the sequence of SEQ ID NO: 29 from which 1 to 14 nucleotides are deleted,
X^{c1} comprises the nucleotide sequence of SEQ ID NO: 39 or a nucleotide sequence having the sequence of SEQ ID NO: 39 from which 1 to 28 nucleotides are deleted,
x^{c2} comprises the nucleotide sequence of SEQ ID NO: 58 or a nucleotide sequence having the sequence of SEQ ID NO: 58 from which 1 to 27 nucleotides are deleted, and
Lk is a polynucleotide linker of 2 to 20 nucleotides in length or absent.

### [Embodiment 41]

The vector system of any one of the above-described embodiments,
wherein in a case where three or more consecutive uracil (U) residues are present in a sequence X^{c1}, the sequence of X^{c1} comprises a modification in which at least one U residue thereof is replaced with A, G, or C.

### [Embodiment 42]

The vector system of any one of the above-described embodiments,
wherein the deletion in the nucleotide sequence of X^{a}, the deletion in the nucleotide sequences of X^{b1} and X^{b2}, and/or the deletion in the nucleotide sequences of X^{c1} and x^{c2} comprises deletion of one or more pairs of complementary nucleotides.

### [Embodiment 43]

The vector system of any one of the above-described embodiments,
wherein the sequence 5'-X^{b1}UUAGX^{b2}-3' in Formula (I) is selected from the group consisting of SEQ ID NOs: 34 to 38 and 5'-UUAG-3'.

### [Embodiment 44]

The vector system of any one of the above-described embodiments,
wherein the sequence 5'-X^{c1}-Lk-X^{c2}-3' in Formula (I) is selected from the group consisting of SEQ ID NOs: 80 to 86 and 5'-Lk-3'.

### [Embodiment 45]

The vector system of any one of the above-described embodiments,
wherein Lk comprises a nucleotide sequence selected from the group consisting of 5'-GAAA-3', 5'-UUAG-3', 5'-UGAAAA-3', 5'-UUGAAAAA-3', 5'-UUCGAAAGAA-3' (SEQ ID NO: 76), 5'-UUCAGAAAUGAA-3' (SEQ ID NO: 77), 5'-UUCAUGAAAAUGAA-3' (SEQ ID NO: 78), and 5'-UUCAUUGAAAAAUGAA-3' (SEQ ID NO: 79).

### [Embodiment 46]

The vector system of any one of the above-described embodiments,
wherein the scaffold region comprises an engineered tracrRNA consisting of a nucleotide sequence selected from the group consisting of SEQ ID NOs: 87 to 132 and/or an engineered crRNA consisting of a nucleotide sequence selected from the group consisting of SEQ ID NOs: 133 to 148.

### [Embodiment 47]

The vector system of any one of the above-described embodiments,
wherein the guide RNA comprises a scaffold region sequence consisting of a nucleotide sequence selected from the group consisting of SEQ ID NOs: 13 and 149 to 186.

### [Embodiment 48]

The vector system of any one of the above-described embodiments,
wherein the vector is selected from the group consisting of a retroviral (retrovirus) vector, a lentiviral (lentivirus) vector, an adenoviral (adenovirus) vector, an adeno-associated viral (adeno-associated virus) vector, a vaccinia viral (vaccinia virus) vector, a poxviral (poxvirus) vector, a herpes simplex viral (herpes simplex virus) vector, and a phagemid vector.

### [Embodiment 49]

The vector system of any one of the above-described embodiments,
wherein the vector is selected from the group consisting of plasmid, naked DNA, DNA complex, mRNA (transcript), and amplicon.

### [Embodiment 50]

A recombinant virus produced by the vector system of any one of the above-described embodiments.

### [Embodiment 51]

A method for modifying a target nucleic acid in a cell, comprising bringing, into contact with the cell, the engineered Cas protein of any one of the above-described embodiments and a guide RNA, the system or composition of any one of the above-described embodiments, the vector system of any one of the above-described embodiments, or the recombinant virus of the above-described embodiment.

### [Embodiment 52]

The method of any one of the above-described embodiments,
wherein the bringing-into-contact is performed *in vitro.*

### [Embodiment 53]

The method of any one of the above-described embodiments,
wherein the bringing-into-contact is performed *in vivo.*

Hereinafter, the present disclosure will be described in more detail by way of the following examples. These examples are intended only to illustrate the present disclosure, and that the scope of the present disclosure is not limited by these examples.

### Example 1. Experimental methods and materials

### Example 1.1. Production of Cas12f1 protein

CWCas12f gene was codon-optimized for expression in human cells (SEQ ID NO: 6), and the codon-optimized sequence was synthesized for vector construction. Finally, to the sequence encoding the Cas12f1 protein were added the chicken β-actin promoter, nuclear localization signal sequences at the 5'- and 3'-end, and a sequence encoding eGFP linked via a self-cleaving T2A peptide.

Template DNA encoding (engineered) CWCas12f guide RNA was synthesized and cloned into the pTwist Amp plasmid vector (Twist Bioscience). When necessary, the vector was used as a template for amplification of the guide RNA-encoding sequence using a U6-complementary forward primer and a protospacer-complementary reverse primer.

A vector for the engineered CRISPR/CWCas12f system was prepared by cloning a nucleic acid encoding the guide RNA into the vector comprising the codon-optimized CWCas12f gene using Gibson assembly.

### Example 1.2. Construction of Cas12f1 PAM variant

Construction of CWCas12f PAM variants was performed by site-directed mutagenesis. To prepare CWCas12f PAM variants, the following candidate amino acids were selected from CWCas12f based on the structural characteristics of Un1Cas12f1: S170, Y174, A184, S188, R191, Q225, Y230, V271, and Q272. Each candidate site was mutated to all 19 possible amino acids. Each PAM variant candidate was incubated with the PAM library plasmid vector (Example 1.8) and gRNA (Example 1.6) as a final mixture, as shown in FIG. 1B, and prepared as PCR amplicons. The final PCR products were subjected to 150-bp paired-end sequencing using Illumina iSeq 100. Each product was tested with respect to its *in vitro* dsDNA cleavage activity for the altered PAM according to the method for determining PAM preference in Example 1.10.

PAM variant candidates were selected based on the criteria of 1) high total sequencing reads and 2) high ratio of sequencing reads for a specific PAM. *In vitro* cleavage and deep-sequencing analysis enabled screening of PAM variant candidates. Specifically, cleavage properties for various PAM sequences were investigated by counting the percentage of reads for a specific PAM sequence.

PCR amplification was performed using Q5 Hot Start high-fidelity DNA polymerase (NEB), and ligation of the PCR products was performed using KLD Enzyme Mix (NEB). The ligated products were transformed into DH5α *Escherichia coli* (*E. coli*) cells. Mutagenesis was identified by Sanger sequencing. The modified plasmid vectors were purified using the NucleoBond Xtra Midi EF kit (MN).

### Example 1.3. Production of dead Cas12f1 protein

Based on the information on amino acid residues involved in catalytic activity of Un1Cas12f1 protein, mutations (D354A, E450A, R518A, and D538A) were introduced into CWCas12f to construct four catalytically inactive CWCas12f mutants (dCWCas12f(dTnpB)). These dCWCas12f mutants were generated by site-directed mutagenesis with primers, including nucleotide changes. Each dCWCas12f was tested to see whether its DNA cleavage activity was completely eliminated while retaining its gene-targeting ability.

*In vitro* DNA degradation analysis and indel efficiency analysis at *NLRC4* locus in HEK293T cells were performed. Briefly, a plasmid vector was constructed to contain a protospacer and a PAM sequence for sgRNA. 5µg of plasmid vector was incubated with 1 µg of gRNA and 2 µg of CWCas12f or dCWCas12f at 37°C for 1 hour. The digested vector sample was analyzed on a 0.8% agarose gel. As a result, all dCWCas12fs were confirmed to have null endonuclease activity (FIGS. 2A and 2B). Among the mutants, dCWCas12f (D354A) was selected based on the previous experiments.

### Example 1.4. Production of adenine base editor fusion protein

A nucleotide sequence encoding an adenine base editor (ABE) fusion protein to be used in each experiment was specified, and then the fusion protein was produced using the following method: The encoded nucleotide sequence was cloned into the pMAL-c2 plasmid vector for replication. The plasmid vector was used to transform BL21 (DE3) *E. coli.* Each of the transformed *E. coli* colonies was grown in LB medium at 37°C until the culture reached an optical density of 0.7. The transformed *E. coli* was incubated overnight at 18°C in the presence of 0.1 mM isopropylthio-β-D-galactoside.

Thereafter, the transformed *E. coli* was collected by centrifugation at 3,500 g for 30 minutes. The collected transformed *E. coli* was resuspended in 20 mM Tris-HCl (pH 7.6), 500 mM NaCl, 5 mM β-mercaptoethanol, and 5% glycerol. The resuspended *E. coli* was dissolved and disrupted by sonication. The sample containing the disrupted *E. coli* was centrifuged at 15,000 g for 30 minutes, and the supernatant was filtered through a 0.45 µm syringe filter (Millipore). The dCWCas12f protein bound to adenosine deaminase present in the filtered supernatant was loaded onto a Ni²⁺-affinity column using an FPLC purification system (KTA Purifier, GE Healthcare). The loaded dCWCas12f protein was eluted with a gradient of 80-400 mM imidazole, 20 mM Tris-HCl (pH 7.5).

The eluted protein was treated with TEV protease for 16 hours. The separated proteins were purified on a Heparin column with a linear gradient of 0.15-1.6 M NaCl. The recombinant Cas12f1 proteins purified on the Heparin column were dialyzed against 20 mM Tris (pH 7.6), 150 mM NaCl, 5 mM β-mercaptoethanol, 5% glycerol. The dialyzed proteins were purified by passage through an MBP column, and then repurified on a monoS column (GE Healthcare) or EnrichS with a linear gradient of 0.5-1.2 M NaCl. The repurified proteins were collected and dialyzed against 20 mM Tris (pH 7.6), 150 mM NaCl, 5 mM β-mercaptoethanol, 5% glycerol to purify a hypercompact base editing construct. The concentration of the produced protein was quantified with the Bradford assay using bovine serum albumin (BSA) as a standard and measured by electrophoresis on a coomassie bluestained SDS-PAGE gel.

### Example 1.5. Exemplary CWCas12f variants

CWCas12f variants (PAM variants, protospacer protrusion-induced variants, and catalytically inactive variants) used in this example are those produced by substitution of a specific amino acid(s) in the wild-type CWCas12f protein of SEQ ID NO: 1, and are shown in Table 8 below.

**[Table 8]**

| Variant type | Mutation location | Description | SEQ ID NO |
|---|---|---|---|
| variant 1 | D354A | catalytically inactive variant of CWCas12f | 223 |
| variant 2 | E450A | catalytically inactive variant of CWCas12f | 224 |
| variant 3 | R518A | catalytically inactive variant of CWCas12f | 225 |
| variant 4 | D538A | catalytically inactive variant of CWCas12f | 226 |
| variant 5 | I159W | protospacer protrusion-induced variant | 227 |
| variant 6 | S164Y | protospacer protrusion-induced variant | 228 |
| variant 7 | I159W/S164Y | protospacer protrusion-induced variant | 229 |
| variant 8 | S170C | PAM variant | 230 |
| variant 9 | S170T | PAM variant | 231 |
| variant 10 | S170Y | PAM variant | 232 |
| variant 11 | Y174H | PAM variant | 233 |
| variant 12 | Y174K | PAM variant | 234 |
| variant 13 | Y174T | PAM variant | 235 |
| variant 14 | A184G | PAM variant | 236 |
| variant 15 | A184H | PAM variant | 237 |
| variant 16 | A184N | PAM variant | 238 |
| variant 17 | A184R | PAM variant | 239 |
| variant 18 | A184S | PAM variant | 240 |
| variant 19 | A184W | PAM variant | 241 |
| variant 20 | S188H | PAM variant | 242 |
| variant 21 | S188K | PAM variant | 243 |
| variant 22 | S188N | PAM variant | 244 |
| variant 23 | S188Q | PAM variant | 245 |
| variant 24 | S188R | PAM variant | 246 |
| variant 25 | R191K | PAM variant | 247 |
| variant 26 | R191Q | PAM variant | 248 |
| variant 27 | R191W | PAM variant | 249 |
| variant 28 | Q225F | PAM variant | 250 |
| variant 29 | Q225R | PAM variant | 251 |
| variant 30 | Q225T | PAM variant | 252 |
| variant 31 | Y230C | PAM variant | 253 |
| variant 32 | Y230H | PAM variant | 254 |
| variant 33 | Y230K | PAM variant | 255 |
| variant 34 | Y230R | PAM variant | 256 |
| variant 35 | Y230S | PAM variant | 257 |
| variant 36 | Y230T | PAM variant | 258 |
| variant 37 | V271T | PAM variant | 259 |
| variant 38 | Q272C | PAM variant | 260 |
| variant 39 | Q272K | PAM variant | 261 |
| variant 40 | Q272R | PAM variant | 262 |
| variant 41 | S188Q/Q272K | multiple PAM variant | 263 |
| variant 42 | S188K/Q272K | multiple PAM variant | 264 |
| variant 43 | S188Q/R191K/Q272K | multiple PAM variant | 265 |
| variant 44 | Y174A | PAM variant | 345 |
| variant 45 | Y174E | PAM variant | 346 |
| variant 46 | Y174N | PAM variant | 347 |
| variant 47 | Y174R | PAM variant | 348 |
| variant 48 | A184T | PAM variant | 349 |
| variant 49 | S188G | PAM variant | 350 |
| variant 50 | S188S | PAM variant | 351 |
| variant 51 | S188T | PAM variant | 352 |
| variant 52 | S188V | PAM variant | 353 |
| variant 53 | R191G | PAM variant | 354 |
| variant 54 | R191H | PAM variant | 355 |
| variant 55 | Y230A | PAM variant | 356 |
| variant 56 | Y230I | PAM variant | 357 |

### Example 1.6. Preparation of guide RNA

A nucleotide sequence of a guide RNA to be used in each experiment was specified, and then the guide RNA was prepared using the following method:

For the engineered CWCas12f single guide RNA (sgRNA), a pre-designed guide RNA to make the same was chemically synthesized. Then, a PCR amplicon comprising the pre-designed guide RNA sequence and T7 promoter sequence was produced.

Ligation of a U-rich tail to the 3'-end of the engineered CWCas12f sgRNA was performed using Pfu PCR Master Mix5 (Biofact) in the presence of sequence-modified primers and a CWCas12f guide RNA plasmid vector. The PCR amplicon was purified using HiGene^{™} Gel & PCR Purification System (Biofact).

In addition, modification of the second, fourth, and fifth regions in the engineered scaffold region of the engineered CWCas12f sgRNA was performed by cloning synthetic oligonucleotides comprising the modified sequences into a vector encoding the linearized guide RNA using ApoI and BamHI restriction enzymes.

Modification of the first region in the engineered scaffold region of the engineered CWCas12f sgRNA was performed by PCR amplification of the canonical or engineered template plasmid vector using a forward primer that targets the 5'-end portion of tracrRNA and a reverse primer that targets the U6 promoter region.

The PCR amplification was performed by Q5 Hot Start high-fidelity DNA polymerase (NEB), and the PCR products were ligated using KLD Enzyme Mix (NEB). The ligated PCR products were transformed into DH5α *E. coli* cells. The modifications were identified by Sanger sequencing.

The modified plasmid vectors were purified using NucleoBond Xtra Midi EF kit (MN). 1 µg of the purified plasmid was used as a template for mRNA synthesis using T7 RNA polymerase (NEB) and NTPs (Jena Bioscience). The prepared engineered CWCas12f guide RNA was purified using Monarch RNA cleanup kit (NEB), aliquoted into cryogenic vials, and stored in liquid nitrogen.

To prepare amplicons of the guide RNA and the engineered guide RNAs, the template DNA plasmids of the canonical guide RNA and the engineered guide RNAs were subjected to PCR amplification with a U6-complementary forward primer and a protospacer sequencecomplementary reverse primer using KAPA HiFi HotStart DNA polymerase (Roche) or Pfu DNA polymerase (Biofact). The PCR amplification products were purified using Higene^{™} Gel & PCR purification system (Biofact) to obtain the amplicons of the guide RNA and the engineered guide RNAs.

Using each PCR amplicon as a template, *in vitro* transcription was performed with T7 polymerase (NEB). The product obtained from the *in vitro* transcription was treated with NEB DNase I, and then purified using the Monarch RNA Cleanup Kit (NEB) to obtain the guide RNA. Thereafter, a plasmid vector containing the pre-designed guide RNA sequence and the T7 promoter sequence was constructed according to the Tblunt plasmid cloning method.

The vector was double-cut at both ends of the guide RNA sequence containing the T7 promoter sequence and then purified. The resultant product was subjected to *in vitro* transcription using T7 polymerase (NEB). The *in vitro* transcription product was treated with DNase I (NEB), and purified using the Monarch RNA Cleanup Kit (NEB) to obtain the guide RNA.

Hereinafter, the guide RNA sequences used in the following examples were canonical sgRNA of SEQ ID NO: 13, ge3.0 of SEQ ID NO: 151, ge4.0 of SEQ ID NO: 152, and ge4.1 of SEQ ID NO: 153. The guide RNA has a structure of 5'-scaffold-spacer-U-rich tail-3', and the spacer is designed with an RNA sequence equivalent to the protospacer in each example (wherein the RNA sequence is obtained by replacing T in the DNA sequence with U).

### Example 1.7. Production of ribonucleoprotein (RNP) particles

Combinations of the engineered CWCas12f proteins (CWCas12f PAM variants, dead CWCas12f proteins, and/or dCWCas12f-adenine base editor fusion proteins) and the guide RNAs to be used in respective experiments were specified, and then RNP particles were prepared using the following method:

300 nM of each of the engineered CWCas12f proteins prepared according to Examples 1.1 to 1.4 and 900 nM of each of the guide RNAs prepared according to Example 1.6 were incubated for 10 minutes at room temperature to prepare ribonucleoprotein (RNP) particles.

### Example 1.8. Construction of plasmid vector

The human codon-optimized CWCas12f gene was synthesized and the CWCas12f-coding sequence was replaced with the Cas12f1 sequence, which was then cloned into the pCas 12f-2A-EGFP vector (Addgene). Then, various versions of the Tad sequence were fused to the 5'- or 3'-region of CWCas12f with linkers of 10 to 40 amino acids in length using NEBuilder HiFi DNA Assembly Master Mix (New England BioLabs). The guide RNA sequence was positioned under the U6 promoter, 5'-upstream of the CMV promoter, using Mlul restriction enzyme. The vector was digested with BbsI restriction enzyme at 37°C for 1 hour, and cloning of the spacer sequence was performed. The plasmid vector for cell transfection was prepared using Nucleobond Xtra midi (MACHEREY-NEGEL). For all vector constructs, the sequence was verified using Sanger sequencing.

### Example 1.9. Construction of PAM library

For oligonucleotides comprising the protospacer (5'-CACACACACAGTGGGCTACC-3') and the PAM library sequence (NNNN), a request for synthesis thereof was made to Bionics. The synthesized oligonucleotides were cloned into the PUC19 vector using All in One PCR cloning kit (Biofact). Each of the cloned vectors was transformed into DH5α *E. coli* cells using an electroporator (Bio-Rad). Each of the transformed colonies was grown in LB medium (broth) at 37°C until the culture reached an optical density of 0.6. The cells were collected by centrifugation at 3,500 g for 15 minutes. The plasmid vectors were prepared using the plasmid preparation kit (Biofact). The sequences thereof were verified using Sanger sequencing. The respective vectors were quantified at 265 nm using a spectrophotometer and mixed at an equal molar ratio to produce 256 PAM library vectors (FIG. 1A).

### Example 1.10. Determination of PAM preference

The plasmid vectors encoding the respective CWCas12f PAM variants constructed in Example 1.2 were transformed into BL21(DE3) *E. coli* cells. Each of the transformed colonies was grown in LB medium at 37°C until the culture reached an optical density of 0.6. The cells were incubated overnight at 18°C in the presence of 0.1 mM isopropylthio-β-D galactoside, and then collected by centrifugation at 3,500 g for 15 minutes. The cells were resuspended in 20 mM Tris-HCl (pH 7.6), 500 mM NaCl, 5 mM β-mercaptoethanol, and 5% glycerol. The cells were sonicated, and then centrifuged at 15,000 g for 15 minutes to prepare a cell lysate. The CWCas12f protein was purified on a Ni²⁺-affinity column and a Heparin column. The guide RNA was synthesized using T7 RNA polymerase (NEB) in the presence of 1 µg of purified plasmid and 4 mM NTP (Jena Bioscience), purified using the Monarch RNA cleanup kit (NEB), aliquoted into cryogenic vials, and then stored in liquid nitrogen. The purified CWCas12f (5 µg), the gRNA(1 µg), and the PAM library plasmid vector (1 µg) were mixed in a final volume of 100 µl with a buffer containing 5 mM Tris-HCl (pH 7.5), 25 mM NaCl, 5 mM MgCl₂, and 1 mM dithiothreitol, and incubated at 37°C for 2 hours. The incubated sample was end-repaired at 20°C for 30 minutes using NEBNext Ultra II End Repair/dA-Tailing Module (NEB, E7546), followed by incubation at 65°C for 30 minutes to terminate the reaction. The resultant was treated with 100 mg/ml RNase A at room temperature for 15 minutes, and then the plasmid DNA was purified using HiGene^{™} Gel&PCR Purification Kit (Biofact). The purified DNA (200 ng per 10 µl) was ligated with adapter DNA (5'-AGATCGGAAGAGCACACGTCTGAACTCCAGTCAC-3') (200 ng) using LigaFast Rapid DNA Ligation Kit (Promega). The DNA was subjected to PCR amplification with the forward primer (5'-GTAAAACGACGGCCAGT-3') and the reverse primer (5'-GTGACTGGAGTTC-3') using KOD One^{™} PCR Master Mix (TOYOBO). The produced PCR amplicon was labeled with Illumina TruSeq HT dual indexes. The final PCR product was subjected to 150-bp paired-end sequencing using Illumina iSeq 100 (FIG. 1B).

### Example 1.11. Construction of PAM-variant cell line

To determine relative indel efficiency of the CWCas12f PAM variants, a PAM-variant cell line was constructed. HEK293T cells (LentX-293T, Takara) were maintained in DMEM (Corning) supplemented with 10% heat-inactivated fetal bovine serum (VWR) and 1% penicillin-streptomycin (WELGENE) in an incubator (37°C, 5% CO₂ atmosphere). Next, a PAM-modified oligonucleotide (90-mer) was synthesized as a donor DNA, and different HEK293T clones, each of which harbors a different PAM mutation at the *NLRC4* locus (5'-TTTAGAGGGAGACACAAGTTGATA-3'), were prepared through homology-directed repair (HDR) (FIG. 3). The donor DNA (SEQ ID NO: 196) was designed to have a modified PAM sequence relative to the PAM sequences of Cas9 and CWCas12f, with a 48-nt left homology arm and a 44-nt right homology arm based on the PAM sequence. For transfection, 4 µg of SpCas9 plasmid vector was transfected, together with 4 µg of donor DNA, into 4 × 10⁵ HEK293T cells using the Neon transfection system (Invitrogen). The electroporation conditions were as follows: 1,300 V, 20 mA, 2 pulses. Three days after transfection, the single cells were seeded into each well of a 24-well plate (Corning) and grown for 3 weeks. Genomic DNA was prepared from the cells in each colony using PureHelix genomic DNA preparation kit (NanoHelix). The PAM-containing region was amplified using KOD One^{™} PCR Master Mix (TOYOBO) according to the manufacturer's instructions. The PAM sequence was verified by deep sequencing using Illumina iSeq 100.

### Example 1.12. Cell culture and transfection

HEK293T (LentX-293T, Takara) cells were cultured in DMEM medium supplemented with 10% heat-inactivated FBS, 1% penicillin/streptomycin, and 0.1 mM non-essential amino acids at 37°C with 5% CO₂.

For transfection, into cells, of the vector constructed in Example 1.8, 1.0 × 10⁵ HEK293T cells were seeded 1 day before transfection. Transfection into the cells was performed by electroporation or lipofection.

For electroporation, each 2 to 5 µg of the plasmid vector encoding the engineered CWCas12f protein and the DNA encoding the guide RNA was transfected into 4 × 10⁵ HEK293T cells using the Neon transfection system (Invitrogen).

For lipofection, 6 to 15 µl of FuGene reagent (Promega) was mixed with 2 to 5 µg of the plasmid vector encoding the engineered CWCas12f protein and 1.5 to 5 µg of the PCR amplicon for 15 minutes. The mixture (300 µl) was added to 1.5 ml of DMEM medium plated with 1 × 10⁶ cells 1 day before transfection. The cells were cultured in the presence of the mixture for 1 to 10 days. After culture, the cells were harvested, and the genomic DNA of the cells was isolated manually using PureHelix^{™} Genomic DNA Preparation Kit (NanoHelix) or Maxwell RSC Cultured cells DNA Kit (Promega).

In addition, the ribonucleoprotein (RNP) particles produced in Example 1.7 were transfected using electroporation or lipofection. Then, 1 day later, the guide RNA prepared according to Example 1.6 was transfected using electroporation.

### Example 1.13. Measurement of indel efficiency

HEK293T cells were transfected with vectors using lipofection method. HEK293T cells were seeded in 24-well plates at a density of 1.0×10⁵ per well 1 day before transfection. 6 µl of FuGene reagent (Promega) were mixed with 1.5 µg of CWCas12f-ABE vector + 500 ng of gRNA-encoding PCR amplicon in 300 µl of Opti-MEM and the mixture was incubated at room temperature for 15 minutes. The mixture was added to each well, and the cells were grown at 37°C and 5% CO₂ for 3 to 5 days. Genomic DNA was extracted by cell lysis using Martin solution (50 mM Tris-HCl (pH 8.5), 1 mM EDTA, 0.005% SDS, and proteinase K). Samples for deep sequencing analysis were prepared by three rounds of PCR amplification. For the first PCR, 1 µl of cell lysate was amplified with target-specific primers designed to amplify the targeted locus in a total volume of 10 µl. 1 µl of the first PCR product was amplified with primers containing Illumina adapter sequences to generate amplicons of 150 bp in length. Finally, the Illumina TruSeq HT dual indexes were labeled onto the PCR amplicons by PCR reaction. All PCR reactions were performed using KOD One^{™} PCR Master Mix (TOYOBO) according to the manufacturer's instructions. The pooled amplicons were column purified using a PCR purification kit (BioFact). The final PCR products were subjected to 150 bp paired-end sequencing using iSeq Control software (v.1.4.1.1700) installed on Illumina iSeq 100. Indel frequency was calculated by MAUND available at https://github.com/ibs-cge/maund. For the tested sites (target sequences), see Table 9.

**[Table 9]**

| Target name (site) | Gene name | Target sequence | Chr | Location |
|---|---|---|---|---|
| site 1 | NLRC4 | [TTTA]GAGGGAGACACAAGTTGATA | chr2 | 32228455 |
| site 2 | KRT1 | [TTTG]CATCCCCAGGACACACACAC | chr12 | 52679076 |
| site 3 | Intergene | [TTTA]AGAACACATACCCCTGGGCC | chr5 | 148270685 |
| site 4 | Intergene | [TTTG]CACACACACAGTGGGCTACC | chr3 | 120228353 |
| site 5 | Intergene | [TTTA]CAAAGACACTCACCCTGTTG | chr4 | 54520536 |
| site 6 | ErbB4 | [TTTG]ATACAGAAATCCTAAATGG | chr2 | 212124887 |
| site 7 | EGFR | [TGTG]TGAAACCATCTCTTCTTAAA | chr7 | 55030839 |
| site 8 | EGFR | [TGTG]CTAAAGGAACACACGTCAGA | chr7 | 55132671 |
| site 9 | EGFR | [TGTG]TGAAAGGGCTGGCACATCGC | chr7 | 55189362 |
| site 10 | EGFR | [TCTG]CTAAATGCTGTCCCTCCCAC | chr7 | 55109063 |
| site 11 | EGFR | [TCTG]TCAAAGCTAAACATTCAGGG | chr7 | 55113727 |
| site 12 | EPHA3 | [TCTG]GTAAACCTAAAACGACCTCC | chr3 | 89152307 |
| site 13 | EPHA3 | [TGTA]GCAAACCTGTACATGGCCCA | chr3 | 89207445 |
| site 14 | EPHA3 | [TGTA]GTAAATTGTTATAGCAGCCC | chr3 | 89262247 |
| site 15 | EPHA3 | [TGTA]GTAAATTGTTATAGCAGCCC | chr3 | 89262270 |

### Example 1.14. Analysis of intracellular base editing activity

A target site in the target nucleic acid or the target gene was amplified by PCR, and the final PCR products were analyzed using targeted deep sequencing.

The target site was amplified for library generation using KAPA HiFi HotStart PCR Kit (KAPA Biosystems #: KK2501). The library was sequenced using MiniSeq with TruSeq HT Dual Index System (Illumina).

### Example 1.15. Statistical Analysis

Statistical significance tests were performed by a two-tailed Student's t-test or Welch's t-test using SigmaPlot software (version 14.0). In a case where normality was not satisfied, the Mann-Whitney Rank Sum test was used. A p-value < 0.05 was considered significant. Data points in the violin plots represented the full range of values along with the interquartile range (25th to 75th percentiles), and the mean value is indicated by a horizontal line. Error bars in all point and bar plots represent standard deviations and were plotted with SigmaPlot (v. 14.0). The sample size was not determined a priori based on a statistical method.

### Example 2. Gene editing activity of system comprising wild-type CWCas12f protein

### Indel efficiency of Un1Cas12f1 and CWCas12f protein

The CWCas12f (TnpB) protein (SEQ ID NO: 1) is a modified form of the Un1Cas12f1 (Cas12f1) protein (SEQ ID NO: 5), with 28 amino acid residues added to its 5' terminus, and it shares a perfectly identical nucleotide sequence with the Un1Cas12f1 (Cas12f1) protein in all regions except for the added amino acids. The CWCas12f was used together with the canonical gRNA or engineered gRNA (for example, ge3.0, 4.0, and 4.1), which was produced according to the method for preparation of gRNA in Example 1.6, to construct the TaRGET system, and indel efficiency thereof was checked. In HEK293T cells, the CWCas12f did not exhibit indel formation activity when used with the canonical gRNA (left of FIG. 4A). However, the CWCas12f exhibited significantly increased indel activity when used with the engineered (augment) gRNA (right of FIG. 4A). Despite slight target-dependent differences in indel level between the Un1Cas12f1 and the CWCas12f, the two proteins showed almost identical overall cleavage patterns depending on the type of engineered gRNA used for three genes (NLRC4 (site 1), KRT1 (site 2), and Intergene (site 3)). This indicates orthogonal use of the engineered RNAs for the two Cas12f1 nucleases (FIG. 4B). Here, for the information on sites 1 to 3 and all sites as described below, see Table 9 in Example 1.13.

### Comparison of indel efficiency among TaRGET, ISDra2TnpB, and AmaTnpB

Since base editing efficiency generally depends on indel efficiency of a wild-type Cas effector protein, it is necessary to start with a Cas system that exhibits sufficiently high indel efficiency. Thus, the indel efficiency among TaRGET, ISDra2TnpB, and AmaTnpB was compared at the *PCSK9* locus in HEK293T cells. The target sites are not exactly shared due to differences in their respective PAM sequences. Thus, 11 sites between exon 5 and exon 8 in the *PCSK9* genomic sequence were selected instead. As a result, the TaRGET system showed significantly higher indel efficiency than ISDra2TnpB and AmaTnpB (FIG. 5). Therefore, it has been concluded that the TaRGET system is suitable for use as a platform for development of a small base editing system.

### Confirmation of formation of homodimer between CWCas 12f and gRNA

Using the method for production of ribonucleoprotein particles in Example 1.7, the CWCas12f protein was incubated with the gRNA to produce a ribonucleoprotein complex. The ribonucleoprotein complex was analyzed by size exclusion chromatography using a Superdex 200 column. The sgRNA-bound CWCas12f was estimated to have a molecular weight of approximately 194 kDa, suggesting that the CWCas12f formed a homodimer in the presence of the engineered gRNA, similar to the Un1Cas12f1 (FIGS. 6A and 6B).

### Example 3. Evaluation of CWCas12f PAM variant's PAM preference

*In vitro* cleavage assay revealed that like the Un1Cas12f1, the CWCas12f exhibited PAM preference for TTTR (TTTA and TTTG), indicating that its targetable sites are quite limited (FIG. 7). Thus, the CWCas12f variants having preference for non-TTTR PAM were developed, and it was intended to apply the PAM variants to a wider range of sites. To this end, as shown in FIG. 1B, individual PAM clones (4⁴ = 256 clones) with diversity in PAM sequences were obtained, and then *in vitro* dsDNA cleavage activity assay was performed on CWCas12f PAM variant candidates comprising the PAM library plasmid vectors (Example 1.9), the gRNAs (Example 1.6), and the CWCas12f PAM variants (Example 1.2). For S170, Y174, A184, S188, R191, Q225, Y230, V271, and Q272, which are candidate amino acid residues, the preference of each variant for various PAM sequences is shown in a heatmap table in FIGS. 8A to 8I. Here, promising PAM variants are highlighted by shading in the "Total read" row. The left column shows that most PAM variants exhibit preference within the "TNTN" sequence. In addition, for the candidate amino acid residues, an additional experiment for indel analysis was performed by co-transfection of each variant and NLRC4-targeting sgRNA into the PAM variant cell line (HEK293T) with an *NLRC4* locus target which was generated by the process illustrated in FIG. 3. The results are shown in FIGS. 21A to 21P.

The promising PAM variants identified from the above results were as follows: S170C (variant 8), S170T (variant 9), Y174H (variant 11), A184H (variant 15), A184S (variant 18), S188H (variant 20), S188K (variant 21), S188N (variant 22), S188Q (variant 23), R191K (variant 25), R191Q (variant 26), R191W (variant 27), Q225F (variant 28), Q225T (variant 30), Y230C (variant 31), Y230S (variant 35), Y230T (variant 36), Q272K (variant 39), and Q272R (variant 40).

For example, S170T, S188Q, S188H, Q225T, Q225F, and Q272K variants exhibited high preference for the TGTA PAM. Among them, S188Q variant exhibited the highest indel frequency for the TGTA PAM compared to the other variants when tested in HEK293T cells with a modified PAM sequence according to Example 1.10 (FIG. 9A). Similarly, S188Q, S188K, and R191K variants exhibited high indel frequency for the TCTG, TGTG, and TTTC PAMs, respectively (FIGS. 9B to 9D). S188K variant exhibited broader PAM specificity, wherein it exhibited preference for TTTT and TTTC as well as TTTA and TTTG, that is, TTTN (FIG. 10). The PAM preference of each PAM variant identified in this example is shown in Table 10 below.

**[Table 10]**

| Cas protein | Type | Amino acid substitution | Modified PAM preference |
|---|---|---|---|
| Un1Cas12f1 | wild-type | - | TTTA, TTTG |
| | wild-type | - | TTTA, TTTG |
| | variant 8 | S170C | TTTT, TTTG, TTTA |
| CWCas12f | variant 9 | S170T | TATA, TTTA, TTTT, TTTG, TGTA, TCTA, TGGG, TTAG, TTCA, TCTG, TGTG, TGGG |
| | variant 11 | Y174H | TGTA, TTTG, TTTA |
| | variant 44 | Y174A | TGTA, TTTG, TTTA, CGAG |
| | variant 13 | Y174T | TGTA, TTTG, TTGA, CGAG |
| | variant 15 | A184H | TGTA, TGTG |
| | variant 16 | A184N | TTTC, TGTA, TGTG, TTTG |
| | variant 17 | A184R | TGTG, TGTA |
| | variant 18 | A184S | TCTG, TTTC, TGTA, TTTG, TTTA |
| | variant 48 | A184T | TTTG, TTTA, TGTA, TCTG |
| | variant 49 | S188G | TTTG, TCTT, GACC, CGGA |
| | variant 20 | S188H | TGTA, TGTG, TGCA |
| | variant 21 | S188K | TTTA, TTTG, TTTC, TGTA, TGTG, TGGG, TTAG |
| | variant 22 | S188N | TATC, TGTC, TCTC, TCTG, TTCG, TTTC, TTTG, TTTA, TGTG, TGTA, TTCA, TGCA |
| | variant 23 | S188Q | TATC, TTTT, TTTG, TTTC, TGTA, TGTC, TGTG, TCTT, TCTG, TCTC, TATA, TGGG, TTTA, TGCA |
| | variant 24 | S188R | TTTG, TTTC, TTTA, TGTG, TGTA, TTCG, TTCA, TGCA |
| | variant 50 | S188S | TTTG, TTTA, TGTA, TCTG, TTGA |
| | variant 51 | S188T | TTTG, TTTA, TGTA, TGTG, TCTG, TTGA, TTCG, TTCA, TTAG |
| | variant 52 | S188V | TTTG, TTTA, TGTA, TTGA, TTAG |
| | variant 53 | R191G | TTTA, TTTG, TTTC, TGTA |
| | variant 54 | R191H | TTTA, TTTG, TTTC, TTCA |
| | variant 25 | R191K | TTTC |
| | variant 26 | R191Q | TATT, TATC, TTTT, TTTG, TTTC, TGTA, TGTC, TCTT, TCTC, TTTA, TCTG, TGTG, TATG, TCTT |
| | variant 27 | R191W | TCTG |
| | variant 28 | Q225F | TATA, TATT, TGTA, TGTG, TCTT, TGGG, TTTG, TATG, TATA |
| | variant 30 | Q225T | TATA, TGTA, TGTG, TCTT, TGGG, TCTA, TGCA |
| | variant 55 | Y230A | TTTG, TTTA, TGTA, TCTG, TTCA |
| | variant 32 | Y230H | TCTC, TTTG, TTTA, TGTA, TCTG, TTCA |
| | variant 56 | Y230I | TTTG, TTTA, TGTA, TCTG, TATT |
| | variant 35 | Y230S | TTTG, TCTG, TATA, TTTA, TTCA |
| | variant 36 | Y230T | TGTA, TCTC |
| | variant 38 | Q272C | TCTA |
| | variant 39 | Q272K | TATT, TATG, TTTA, TTTT, TTTG, TGTC, TCTT, TCTC, TTAG, TTCA, TTTC, TGTA, TCTG, TCTT, TATA, TTCG |
| | variant 40 | Q272R | TTTC, TGTA, TCTG, TTTG, TTGA, TTCG, TTCA, TTAG |
| | variant 41 | S188Q/Q272K | TATC, TCTG, TCTA, TGTC, TGTG, TGTT, TATG, TATA, TTTG, TTTA, TGTA |
| | variant 42 | S188K/Q272K | TATC, TCTA, TCTT, TGTC, TGTA, TGTT, TATG, TATA, TTTG, TTTT, TTTA, TCTG, TGTG |
| | variant 43 | S188Q/R191K/Q272K | TATC, TCTG, TCTA, TCTT, TGTG, TGTT, TATA, TTTC, TTTG, TTTA, TGTC, TGTA, TATG |

### Example 4. CWCas12f variant for expansion of targetable sites

### Example 4.1. Selection of dCWCas12f-based adenine base editor

dCWCas12f-based adenine base editor (ABE) fusion proteins were constructed in four types depending on the orientation in which deaminase was fused: TaRGET-ABE-N1 (SEQ ID NO: 201), TaRGET-ABE-N2 (SEQ ID NO: 202), TaRGET-ABE-C1 (SEQ ID NO: 203), and TaRGET-ABE-C2 (SEQ ID NO: 204). Specifically, wild-type Tad-mutant Tad (Tad-Tad*) or Tad*-Tad, which constitutes ABE, were fused to the N- or C-terminus of dCWCas12f to construct four types of CWCas12f-based adenine base editors (FIG. 11A).

These constructs were tested against two validated targets (sites 4 and 2), one representing an A-rich sequence in the PAM-proximal region (site 4) and the other representing an A-rich sequence in the PAM-distal region (site 2). As shown in FIG. 11B, the test results indicated that the architectures, in which the deaminase is fused in the C-terminal orientation, (TaRGET-ABE-C1 and TaRGET-ABE-C2) exhibited a significant level of A to G conversion activity, whereas the modules, in which the deaminase is fused in the N-terminal orientation, (TaRGET-ABE-N1 and TaRGET-ABE-N2) exhibited minimal such conversion activity. Furthermore, the A to G conversion was observed only in the PAM-proximal region.

TaRGET-ABE-C2 (dCWCas12f-linker-Tad-linker-Tad*-NLS) was used to identify a base editing window due to its higher conversion rate than other ABEs. Here, the base editing window represents positions at which base conversion can be induced by ABE, and the expansion of the window as described below means expansion of the positions at which base conversion occurs. Additionally, to define the base editing window of TaRGET-ABE-C2, the substitution profile of TaRGET-ABE-C2 was explored for five endogenous sites (sites a to e) in HEK293T cells, as shown in FIGS. 12A to 12C. The endogenous sites were selected from validated sites that show significant A to G conversion activity and also have multiple adenine nucleotides in the PAM-proximal region. The TaRGET-ABE system induced A to G conversions in a range of positions 2 to 6, although the conversion was most predominant at positions 3 and 4 (A3 and A4). Here, the position refers to a location relative to the PAM-proximal sequence. The sequence profile was obtained by deep-sequencing analysis for these positions after transfection with the TaRGET-ABE-C2 vector.

Various Tad variants developed to date were fused to the C-terminus of dCWCas12f (D354A, variant 1), and these fusion products were compared in terms of their conversion rates from A3 to G3. As a result, it was found that the codon-optimized Tad-Tad* (V106W, D108Q) exhibited the highest conversion rate compared to the other forms (FIG. 13A).

The optimized Tad dimer was denoted as Tad-Tad**, and this form of ABE was named TaRGET-ABE-C3.0 (SEQ ID NO: 206). dCWCas12f and the Tad dimer (Tad-Tad**) were linked to each other via linkers of various lengths, and these products were compared in terms of A3 to G3 conversion efficiency. As a result, the linker was determined to have a length of 32 amino acids (FIG. 13B). Based on the heat map results (FIG. 13D) illustrated in FIG. 13C, which show A to G conversion efficiency of several adenine base editor constructs, the base editing window of TaRGET-ABE-C3.0 was formed in a relatively narrow range (positions 3 to 5), similar to the recently reported ABEMINI. The overall conversion efficiency of TaRGET-ABE-C3.0 was significantly higher than that of Cas12f-based ABEMINI, and was lower than that of SpCas9 nickase-based ABEs such as ABE7.10, ABE8e25, and ABE9. Here, for ABE8e, a monomeric Tad in its engineered form was used, while for ABE9, a monomeric Tad in its additionally engineered form (V82S/Q154R) was used.

To achieve optimal base editing results, it is necessary to select the most suitable gRNA for CWCas12f. In order to compare indel activity results obtained by using three different versions of sgRNA (ge3.0, ge4.0, and ge4.1) described in Example 1.6, 18 targets were selected and tested. As a result, a correlation was observed between indel efficiency and conversion efficiency for the gRNA versions (ge3.0, ge4.0, and ge4.1) in 15 out of 18 sites (FIG. 13E). Therefore, TaRGET-ABE-C3.0 guided by the optimal gRNA version exhibits optimal base editing performance.

### Example 4.2. Multiplexed base editing activity of adenine base editor to which PAM variant has been applied

To test application of PAM variants for adenine base editing in a non-TTTR PAM context, A to G conversion activity of the PAM variants was tested on various sites with modified PAMs. Here, TaRGET-ABE-C3.0 described in Example 4.1 was used as an adenine base editor fusion protein.

As shown in FIG. 14A, levels of conversion activity from A3 to G3, A4 to G4, and A5 to G5 vary depending on the PAM variants. To track conversion efficiency, sites with A at positions 3, 4, and 5 were selected. Here, Tests 1 to 3 indicate that the same experiment was repeated three times.

The PAM variants may be used selectively (individually) for specific sequence contexts, or the variants exhibiting multiple PAM preference may be used for multiplexed base editing. To investigate this possibility, base editing activity of variants having stacked PAM mutations (that is, having multiple PAM mutations) was tested. As a result, it was found that the S188Q/Q272K variant (variant 41) and the S188K/Q272K variant (variant 42) exhibited broader PAM preference, including TTTA, TTTG, TTTC, TATG, TGTA, TGTG, TGTC, and TCTG, and the S188Q/R191K/Q272K variant (variant 43) also exhibited broad PAM preference (FIG. 14B). The base editing activity of the variants having stacked mutations was measured in HEK293T cells harboring the PAM-modified *NLRC4* locus as illustrated in FIG. 3. The multiplexed base editing was validated in wild-type HEK293T cells by transfection of the PAM variants of CWCas12f having stacked mutations together with multiple gRNAs. In particular, the S188Q/Q272K and S188K/Q272K variants exhibited base editing potential at five different endogenous loci (sites 5, 6, 7, 11, and 15) at the same time (FIG. 14C). The PAM preference for these variants having stacked mutations are shown in Table 1 above. Taken together, the engineering of CWCas12f expanded occupancy of targetable base editing sites from 0.78% to 3.12%.

### Example 4.3. Expansion of targetable site through mutation in protospacer binding region of CWCas12f-gRNA ribonucleoprotein complex

Despite expansion of the targetable site using PAM variants in Example 4.2, the editing occurrence rate is still limited because the predominant editing window was formed at positions 3 and 4. Although the editing window limited to positions 3 and 4 is sometimes advantageous for specific editing, expanding or shifting the window may be additional options to expand applicability of the TaRGET-ABE system. Therefore, structural modeling of the CWCas12f-gRNA ribonucleoprotein complex was performed to identify potential mutation sites of Ile159 and Ser164.

The structural modeling revealed that bases at positions 5 and 6 of the protospacer are hidden in the pocket of WED domain (FIG. 15). Thus, it was speculated that replacing Ile159 and Ser164 with bulkier amino acids would cause the bases at positions 5 and 6 to protrude further as shown in FIG. 16A, allowing the deaminase to access these bases more easily. The I159W variant (variant 5), S164Y variant (variant 6), or I159W/S164Y variant (variant 7) were constructed and applied to adenine base editing for several targets having A at different positions, respectively. Here, TaRGET-ABE-C2.0 described in Example 4.1 was used as an adenine base editor fusion protein. Comparison of editing efficiency between these variants and the wild-type TaRGET-ABE-C2 demonstrated that the S164Y variant exhibited a dramatically reduced A to G conversion rate at positions 3 and 4 without any window expansion. In contrast, the I159W variant retained A3 and A4 conversions while maintaining conversion rates at positions 5 and 6 (FIG. 16B).

### Example 4.4. Expansion of targetable site through modification of deaminase module

The dead variant dCWCas12f (D538A) was used for the I159W Tad variant (in addition to D538A, D354A, E450A, and R518A were also applicable). This approach was relevant to different architectures of the deaminase module. While constructing different combinations of Tad variants, it was accidentally discovered that the dCWCas12f-Tad-Tad8e (WQ) module exhibited a window expansion at position 2. The eTad (Tad8e) sequence was originally used as a monomeric deaminase for the ABE8e version. A fusion of dCWCas12f (D354A) and the Tad-Tad8e (WQ) dimeric module (hereafter referred to as TaRGET-ABE-C3.1) induced dramatically increased conversion at position 2, along with sustained conversion efficiency at positions 3 and 4 (FIG. 17A).

In addition, the TaRGET-ABE-C3.1 system was validated for 25 endogenous regions (specific genes) (FIG. 17B). The information on target genes and primers is shown in Table 11 below.

**[Table 11]**

| Gene name | PAM | Protospacer | Primer | |
|---|---|---|---|---|
| | | | Forward (5'→3') | Reverse (5'→3') |
| LOC1053703 93 | TTTA | AAGAAAGCTAC AGGAAAGCA(SE Q ID NO: 266) | TGACCAGGGAGT TCCTCACA(SEQ ID NO: 267) | GAAGCCGAGCTC AGTTACCC(SEQ ID NO: 268) |
| intergenic | TTTA | AAGAGGGGAGG TTGACTTTG(SE Q ID NO: 269) | CCTCCCAGATCC CACTCAGA(SEQ ID NO: 270) | CCCAAAGTCAAC CTCCCCTC(SEQ ID NO: 271) |
| ZNF10 | TTTA | AATAAGTCTTAC CACGTGTC(SEQ ID NO: 272) | GTTCGTGCCACT GTACTCCA(SEQ ID NO: 273) | TTCTGCTTCCAG CCGAGATG(SEQ ID NO: 274) |
| FUS | TTTA | ACAAAGAAACC AGCAGTGGC(SE Q ID NO: 275) | TACTTCCCCTGCC AGCATTG(SEQ ID NO: 276) | GGAAGATCCCCC CAAACTGG(SEQ ID NO: 277) |
| OR4K17 | TTTA | ACAAGTTCAGA ATCACCTTA(SE Q ID NO: 278) | TGACCAGCTCCC AGGATGTA(SEQ ID NO: 279) | GTGAAGGAGAC CCAAGAGCC(SE Q ID NO: 280) |
| POLRMT | TTTA | AGGACTATGTG TGGCCAGTG(SE Q ID NO: 281) | GAGACGGGGTTT CACTGTGT(SEQ ID NO: 282) | CTCTCTGTCTGT TGCCCAGG(SEQ ID NO: 283) |
| LOC1001283 98 | TTTA | CAAAGAAATGT ACTGCCTTA(SE Q ID NO: 284) | GGGCCACTGGTC ACCATATT(SEQ ID NO: 285) | GCACCATGTCGT CCCATTTG(SEQ ID NO: 286) |
| CARS | TTTA | CAACAGCCTCA CCAGGAACA(SE Q ID NO: 287) | ACAAGCCAGGGT GATCCATG(SEQ ID NO: 288) | ATCACACCACTG CACTCTGG(SEQ ID NO: 289) |
| RPH3AL | TTTA | CACAAGGGATC TGAGACTTG(SE Q ID NO: 290) | GCCCCTCCGATC ATAAGCTC(SEQ ID NO: 291) | GAGGGTGAAGTT GGGTCCAG(SEQ ID NO: 292) |
| P2RX5-TAX1BP3 | TTTA | CACATAGGCCAT TCAGAAAC(SEQ ID NO: 293) | GTCTACTGAAAT GGGGGCCC(SEQ ID NO: 294) | GGCCAGGTTGGT CTTGAACT(SEQ ID NO: 295) |
| GAK | TTTA | CAGAGTCCCGG GAACAAGCC(SE Q ID NO: 296) | CAGCTCCTCAGA AGAGCCAG(SEQ ID NO: 297) | AGTGAGTGTGAG GCTCCAGA(SEQ ID NO: 298) |
| Intergene | TTTA | CATACAGGGCT CTGTACCCA(SE Q ID NO: 299) | GGCCCAGAACCT TGCTCTTT(SEQ ID NO: 300) | ACCACCCTGCGT AACATAGC(SEQ ID NO: 301) |
| Intergene | TTTA | CTGAGATTTGC GAAGAGTTA(SE Q ID NO: 302) | GTGGCAGTGGCT GTATTCCT(SEQ ID NO: 303) | GCTTGAAAGCCA AAGCCTCC(SEQ ID NO: 304) |
| Intergene | TTTA | CTTAGTAGTCTC AGAACCAA(SEQ ID NO: 305) | CTTGCTAAAGGG CTGGCTCT(SEQ ID NO: 306) | GCCCAGCTGATT CTAAGGCA(SEQ ID NO: 307) |
| Intergene | TTTA | GAAATATGACT GGAAGTAAA(SE Q ID NO: 308) | CACTCTCGCTTAC CCTGCTC(SEQ ID NO: 309) | ACACAGCCAAC ACCCCTATG(SEQ ID NO: 310) |
| KLHL29 | TTTA | GAGAGACCGCT CAGGCTGGA(SE Q ID NO: 311) | GCCGAAAGCCTA CACCTCTT(SEQ ID NO: 312) | AGTGAGAGGAG AGAGGCTGG(SE Q ID NO: 313) |
| LOC1053703 93 | TTTA | GCAGTACACCT GAGGGAACA(SE Q ID NO: 314) | TGACCAGGGAGT TCCTCACA(SEQ ID NO: 315) | GAAGCCGAGCTC AGTTACCC(SEQ ID NO: 316) |
| OSBPL5 | TTTA | GCATTAAGGCC AGCGCTGGG(SE | CAGAGTGGCCCT AACCTGTC(SEQ | CGATCCTGGTTC CTGCTCTG(SEQ |
| | | Q ID NO: 317) | ID NO: 318) | ID NO: 319) |
| LOC1053695 97 | TTTA | GCCATGGTGAA GGTGAAATC(SE Q ID NO: 320) | ACTGTGCTGCTG AGAGGAAC(SEQ ID NO: 321) | GAAGCAGGGAT GTCAGTGCT(SEQ ID NO: 322) |
| CCDC127 | TTTA | GGCAAGGGTCT TGATGCATC(SE Q ID NO: 323) | CACCAGCTTGAA GGCTCTGA(SEQ ID NO: 324) | CCTCCTGGGTTC AAGCGATT(SEQ ID NO: 325) |
| ZMYM2 | TTTA | GTAGGCTGCTG TTGGACAGA(SE Q ID NO: 326) | GAGCTCTGAGTT ACCCGACA(SEQ ID NO: 327) | CAGGCTTGTGGG AGAAGGAA(SEQ ID NO: 328) |
| Intergene | TTTA | GTCAAATAAAG AAAAATACG(SE Q ID NO: 329) | TGGAGAGATGGG ATGGCTGA(SEQ ID NO: 330) | AGCAGAGAAATT GGGAAGCA(SEQ ID NO: 331) |
| Intergene | TTTA | CAAAGACACTC ACCCTGTTG(SE Q ID NO: 332) | GGCCCAGAACCT TGCTCTTT(SEQ ID NO: 333) | ACCACCCTGCGT AACATAGC(SEQ ID NO: 334) |
| Intergene | TTTA | AGAACACATAC CCCTGGGCC(SE Q ID NO: 335) | CACCACCACACC TGGCTAAT(SEQ ID NO: 336) | ATGTCCGAGCAA GGCTTTGA(SEQ ID NO: 337) |
| INIP | TTTA | AGAGCAGCGAT TGTAAGGAG(SE Q ID NO: 338) | ACAGGGCCATCT TGTGACAG(SEQ ID NO: 339) | AAGTGACGTCTG CACTGAGG(SEQ ID NO: 340) |

The A to G conversion efficiency for the target genes is shown in FIGS. 18A to 18G. Here, the conversion rate at each position was calculated from deep sequencing data using MAUND software. The position of Ain the protospacer sequence was highlighted by shading. The value is a sum of all possible conversion rates. "wt" represents a conversion rate of ABE-untreated control cells. According to the distribution, the most predominant base editing effects were observed at positions 2 to 5 even without application of the I159W mutation; however, it was found that for position 6, a remarkable window expansion occurred when the I159W mutation was applied (FIG. 17A), indicating that the window expanded to a range beyond position 6. Nevertheless, two or more targets were identified in which positions 17 and/or 18 were edited with relatively high efficiency (FIG. 17B).

PAM-proximal editing may occur from gRNA-independent editing within the R-loop or from a non-canonical window. A one to one comparison of all versions of TaRGET-ABE (ABEMINI, TaRGET-ABE-C2, TaRGET-ABE-C3.0, and TaRGET-ABE-C3.1) in terms of base editing window and efficiency is shown in FIG. 19. High A to G conversion may be observed in TaRGET-ABE-C3.0 and TaRGET-ABE-C3.1. In addition, comparison between Cas12f1-based ABEMINI and TaRGET-ABE-C2 reveals that ABEMINI has significantly low base editing efficiency in the base editing window. This demonstrates that the CWCas12f is a desirable nuclease for hypercompact adenine base editors.

In summary, engineering and reconfiguration of the CWCas12f and Tad modules significantly expanded the base editing range, which would otherwise be very limited, by expanding a PAM recognition range and shifting or expanding a base editing window.

## Claims

1. An engineered Cas protein comprising amino acid substitution(s) at one or more amino acid residues selected from the group consisting of amino acids at positions 159, 164, 170, 174, 184, 188, 191, 225, 230, and 272 with respect to SEQ ID NO: 1 or at amino acid residue(s) corresponding thereto, wherein the engineered Cas protein comprises an amino acid sequence having at least 80% sequence identity to the sequence represented by SEQ ID NO: 1.

2. The engineered Cas protein of claim 1, wherein the substitution(s) comprises amino acid substitution(s) at one or more amino acid residues selected from the group consisting of amino acids at positions 170, 174, 184, 188, 191, 225, 230, and 272 or at amino acid residue(s) corresponding thereto, wherein the engineered Cas protein shows altered protospacer-adjacent motif (PAM) recognition specificity.

3. The engineered Cas protein of claim 1, wherein the substitution(s) comprises amino acid substitution(s) at one or more amino acid residues selected from the group consisting of amino acids at positions 159 and 164 or at amino acid residue(s) corresponding thereto, wherein the engineered Cas protein has an altered editing window for PAM-proximal regions.

4. The engineered Cas protein of claim 2, wherein the substitution(s) further comprises amino acid substitution(s) at one or more amino acid residues selected from the group consisting of amino acids at positions 159 and 164 or at amino acid residue(s) corresponding thereto.

5. The engineered Cas protein of claim 1, wherein the substitution(s) comprises one or more amino acid substitutions selected from the group consisting of following (1) to (10):
(1) 159W;
(2) 164Y;
(3) 170C or 170T;
(4) 174H, 174A, 174E, 174K, 174N, 174R, or 174T;
(5) 184H, 184N, 184R, 184S, or 184T;
(6) 188G, 188H, 188K, 188N, 188Q, 188R, 188S, 188T, or 188V;
(7) 191G, 191H, 191K, 191Q, or 191W;
(8) 225F or 225T;
(9) 230A, 230H, 230I, 230S, or 230T; and
(10) 272C, 272K, or 272R.

6. The engineered Cas protein of claim 1, wherein the substitution(s) comprises one or more substitutions selected from the group consisting of following (1) to (10):
(1) I159W;
(2) S164Y;
(3) S170C or S170T;
(4) Y174H, Y174A, Y174E, Y174K, Y174N, Y174R, or Y174T;
(5) A184H, A184N, A184R, A184S, or A184T;
(6) S188G, S188H, S188K, S188N, S188Q, S188R, S188S, S188T, or S188V;
(7) R191G, R191H, R191K, R191Q, or R191W;
(8) Q225F or Q225T;
(9) Y230A, Y230H, Y230I, Y230S, or Y230T; and
(10) Q272C, Q272K, or Q272R.

7. The engineered Cas protein of claim 2, wherein the substitution(s) comprises one or more amino acid substitutions selected from the group consisting of the amino acid substitutions described in Table 1.

8. The engineered Cas protein of claim 2, wherein the engineered Cas protein recognizes a PAM sequence that a wild-type Cas12f protein either cannot recognize or poorly recognizes.

9. The engineered Cas protein of claim 2, wherein the engineered Cas protein recognizes at least one PAM sequence selected from the group consisting of 5'-TVTN-3', 5'-TTVV-3', 5'-TGGG-3', and 5'-TTTN-3' (wherein N is A, T, G, or C, and V is A, G, or C).

10. The engineered Cas protein of claim 8, wherein the engineered Cas protein is an engineered Un1Cas12f1 protein or an engineered CW Cas12f protein.

11. The engineered Cas protein of claim 7, wherein the engineered Cas protein further comprises at least one substitution selected from the group consisting of I159W and S164Y.

12. The engineered Cas protein of claim 1, wherein the engineered Cas protein has eliminated DNA cleavage activity.

13. The engineered Cas protein of claim 12, wherein the engineered Cas protein further comprises amino acid substitution(s) at one or more amino acid residues selected from the group consisting of amino acids at positions 354, 450, 518, and 538 with respect to SEQ ID NO: 1 or at amino acid residue(s) corresponding thereto.

14. The engineered Cas protein of claim 13, wherein the substitution(s) further comprises at least one amino acid substitution selected from following (a) to (d):
(a) 354A, 354Q, 354L, 354W, or 354V;
(b) 450A, 450Q, 450L, 450W, or 450V;
(c) 518A, 518Q, 518L, 518W, or 518V; and
(d) 538A, 538Q, 538L, 538W, or 538V.

15. The engineered Cas protein of claim 1, wherein the engineered Cas protein further comprises deletion, insertion, substitution, or addition of at least one amino acid and is capable of forming a complex with a guide RNA.

16. A fusion protein comprising (i) the engineered Cas protein of any one of claims 1 to 15, (ii) a functional domain, and optionally (iii) a linker.

17. The fusion protein of claim 16, wherein the functional domain has nuclease activity, nickase activity, recombinase activity, deaminase activity, methyltransferase activity, methylase activity, acetylase activity, acetyltransferase activity, transcriptional activation activity, transcriptional inhibition activity, or reverse transcriptase activity.

18. The fusion protein of claim 16, wherein the functional domain has deaminase activity.

19. The fusion protein of claim 16, wherein the functional domain comprises at least one expression regulatory domain.

20. The fusion protein of claim 19, wherein the expression regulatory domain is selected from the group consisting of VP64, VPR, KRAB, MeCP2, DNMT, HAT, HDAC, TET, and p300.

21. A polynucleotide encoding the engineered Cas protein of any one of claims 1 to 15 or the fusion protein of any one of claims 16 to 20.

22. An engineered CRISPR/Cas system or composition, comprising:
(i) the engineered Cas protein of any one of claims 1 to 15, or a nucleic acid encoding the protein, and
(ii) a guide RNA comprising a guide sequence hybridizable to a target sequence adjacent to a PAM, or a nucleic acid encoding the guide RNA.

23. The system or composition of claim 22, wherein the guide RNA is an engineered guide RNA.

24. The system or composition of claim 23, wherein the guide RNA comprises a U-rich tail sequence linked to the 3'-end of the guide sequence, and the U-rich tail is represented by 5'-(UₘV)ₙUₒ-3', wherein V is each independently A, C, or G; m and o are integers between 1 and 20; and n is an integer between 0 and 5.

25. The system or composition of claim 23, wherein the guide RNA comprises an engineered scaffold region, and the engineered scaffold region comprises a nucleotide sequence having at least 50% sequence identity to a scaffold region of a wild-type Cas12f1 guide RNA sequence, which sequentially comprises, from the 5'-end, a first stem-loop region, a second stem-loop region, a third stem-loop region, a fourth stem-loop region, and a tracrRNA-crRNA complementarity region, wherein the guide RNA comprises at least one modification selected from the group consisting of following (1) to (4) with respect to the wild-type Cas12f1 guide RNA sequence:
(1) deletion of at least a part of the first stem-loop region;
(2) deletion of at least a part of the second stem-loop region;
(3) deletion of at least a part of the tracrRNA-crRNA complementarity region; and
(4) replacement of one or more uracil (U) residues with A, G, or C in three or more consecutive U residues when the consecutive U residues are present in the tracrRNA-crRNA complementarity region.

26. The system or composition of claim 25, wherein the wild-type Cas12f1 guide RNA comprises tracrRNA comprising the nucleotide sequence of SEQ ID NO: 11 and crRNA comprising the nucleotide sequence of SEQ ID NO: 12.

27. The system or composition of claim 25, wherein the engineered scaffold region comprises a sequence having at least 80% sequence identity to a sequence represented by Formula (I): 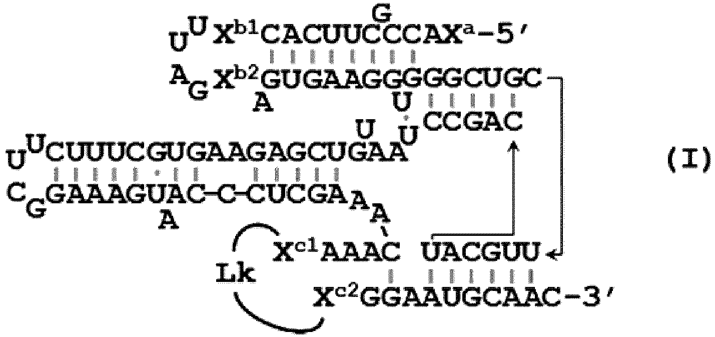 in Formula (I),
X^{a} comprises the nucleotide sequence of SEQ ID NO: 14 or a nucleotide sequence having the sequence of SEQ ID NO: 14 from which 1 to 20 nucleotides are deleted,
X^{b1} comprises the nucleotide sequence of SEQ ID NO: 25 or a nucleotide sequence having the sequence of SEQ ID NO: 25 from which 1 to 13 nucleotides are deleted,
X^{b2} comprises the nucleotide sequence of SEQ ID NO: 29 or a nucleotide sequence having the sequence of SEQ ID NO: 29 from which 1 to 14 nucleotides are deleted,
X^{c1} comprises the nucleotide sequence of SEQ ID NO: 39 or a nucleotide sequence having the sequence of SEQ ID NO: 39 from which 1 to 28 nucleotides are deleted,
X^{c2} comprises the nucleotide sequence of SEQ ID NO: 58 or a nucleotide sequence having the sequence of SEQ ID NO: 58 from which 1 to 27 nucleotides are deleted, and
Lk is a polynucleotide linker of 2 to 20 nucleotides in length or absent.

28. The system or composition of claim 27, wherein in a case where three or more consecutive uracil (U) residues are present in a sequence of X^{c1}, the sequence of X^{c1} comprises a modification in which at least one U residue thereof is replaced with A, G, or C.

29. The system or composition of claim 27, wherein the deletion in the nucleotide sequence of X^{a}, the deletion in the nucleotide sequences of X^{b1} and X^{b2}, and/or the deletion in the nucleotide sequences of X^{c1} and X^{c2} comprises deletion of one or more pairs of complementary nucleotides.

30. The system or composition of claim 27, wherein the sequence 5'-X^{b1}UUAGX^{b2}-3' in Formula (I) is selected from the group consisting of SEQ ID NOs: 34 to 38 and 5'-UUAG-3'.

31. The system or composition of claim 27, wherein the sequence 5'-X^{c1}-Lk-X^{c2}-3' in Formula (I) is selected from the group consisting of SEQ ID NOs: 80 to 86 and 5'-Lk-3'.

32. The system or composition of claim 27, wherein Lk comprises a nucleotide sequence selected from the group consisting of 5'-GAAA-3', 5'-UUAG-3', 5'-UGAAAA-3', 5'-UUGAAAAA-3', 5'-UUCGAAAGAA-3' (SEQ ID NO: 76), 5'-UUCAGAAAUGAA-3' (SEQ ID NO: 77), 5'-UUCAUGAAAAUGAA-3' (SEQ ID NO: 78), and 5'-UUCAUUGAAAAAUGAA-3' (SEQ ID NO: 79).

33. The system or composition of claim 25, wherein the scaffold region comprises an engineered tracrRNA consisting of a nucleotide sequence selected from the group consisting of SEQ ID NOs: 87 to 132 and/or an engineered crRNA consisting of a nucleotide sequence selected from the group consisting of SEQ ID NOs: 133 to 148.

34. The system or composition of claim 22, wherein the guide RNA comprises a scaffold region sequence consisting of a nucleotide sequence selected from the group consisting of SEQ ID NOs: 13 and 149 to 186.

35. The system or composition of claim 22, wherein the engineered Cas12 protein forms a complex with the guide RNA.

36. A vector system, comprising at least one vector that comprises:
(i) a first nucleic acid construct to which a nucleotide sequence encoding the engineered Cas protein of any one of claims 1 to 15 is operably linked; and
(ii) a second nucleic acid construct to which a nucleotide sequence encoding a guide RNA is operably linked, the guide RNA comprising a guide sequence hybridizable with a target sequence adjacent to a PAM,
wherein the nucleic acid constructs (i) and (ii) are located in the same vector or different vectors.

37. The vector system of claim 36, wherein the guide RNA comprises a U-rich tail sequence linked to the 3'-end of the guide sequence, and the U-rich tail is represented by 5'-(UₘV)ₙUₒ-3', wherein V is each independently A, C, or G; m and o are integers between 1 and 20; and n is an integer between 0 and 5.

38. The vector system of claim 36, wherein the guide RNA comprises an engineered scaffold region, and the engineered scaffold region comprises a nucleotide sequence having at least 50% sequence identity to a scaffold region of a wild-type Cas12f1 guide RNA sequence, which sequentially comprises, from the 5'-end, a first stem-loop region, a second stem-loop region, a third stem-loop region, a fourth stem-loop region, and a tracrRNA-crRNA complementarity region, wherein the guide RNA comprises at least one modification selected from the group consisting of the following (1) to (4) with respect to the wild-type Cas12f1 guide RNA sequence:
(1) deletion of at least a part of the first stem-loop region;
(2) deletion of at least a part of the second stem-loop region;
(3) deletion of at least a part of the tracrRNA-crRNA complementarity region; and
(4) replacement of one or more uracil (U) residues with A, G, or C in three or more consecutive U residues when the consecutive U residues are present in the tracrRNA-crRNA complementarity region.

39. The vector system of claim 38, wherein the wild-type Cas12f1 guide RNA comprises tracrRNA comprising the nucleotide sequence of SEQ ID NO: 11 and crRNA comprising the nucleotide sequence of SEQ ID NO: 12.

40. The vector system of claim 38, wherein the engineered scaffold region comprises a sequence having at least 80% sequence identity to a sequence represented by Formula (I): 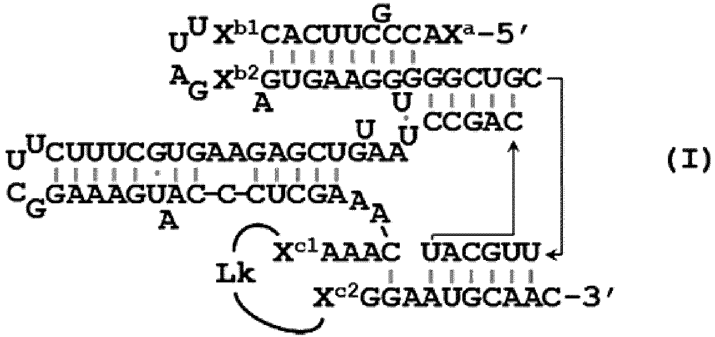 in Formula (I),
X^{a} comprises the nucleotide sequence of SEQ ID NO: 14 or a nucleotide sequence having the sequence of SEQ ID NO: 14 from which 1 to 20 nucleotides are deleted,
X^{b1} comprises the nucleotide sequence of SEQ ID NO: 25 or a nucleotide sequence having the sequence of SEQ ID NO: 25 from which 1 to 13 nucleotides are deleted,
X^{b2} comprises the nucleotide sequence of SEQ ID NO: 29 or a nucleotide sequence having the sequence of SEQ ID NO: 29 from which 1 to 14 nucleotides are deleted,
X^{c1} comprises the nucleotide sequence of SEQ ID NO: 39 or a nucleotide sequence having the sequence of SEQ ID NO: 39 from which 1 to 28 nucleotides are deleted,
X^{c2} comprises the nucleotide sequence of SEQ ID NO: 58 or a nucleotide sequence having the sequence of SEQ ID NO: 58 from which 1 to 27 nucleotides are deleted, and
Lk is a polynucleotide linker of 2 to 20 nucleotides in length or absent.

41. The vector system of claim 40, wherein in a case where three or more consecutive uracil (U) residues are present in a sequence X^{c1}, the sequence of X^{c1} comprises a modification in which at least one U residue thereof is replaced with A, G, or C.

42. The vector system of claim 40, wherein the deletion in the nucleotide sequence of X^{a}, the deletion in the nucleotide sequences of X^{b1} and X^{b2}, and/or the deletion in the nucleotide sequences of X^{c1} and X^{c2} comprises deletion of one or more pairs of complementary nucleotides.

43. The vector system of claim 40, wherein the sequence 5'-X^{b1}UUAGX^{b2}-3' in Formula (I) is selected from the group consisting of SEQ ID NOs: 34 to 38 and 5'-UUAG-3'.

44. The vector system of claim 40, wherein the sequence 5'-X^{c1}-Lk-x^{c2}-3' in Formula (I) is selected from the group consisting of SEQ ID NOs: 80 to 86 and 5'-Lk-3'.

45. The vector system of claim 40, wherein Lk comprises a nucleotide sequence selected from the group consisting of 5'-GAAA-3', 5'-UUAG-3', 5'-UGAAAA-3', 5'-UUGAAAAA-3', 5'-UUCGAAAGAA-3' (SEQ ID NO: 76), 5'-UUCAGAAAUGAA-3' (SEQ ID NO: 77), 5'-UUCAUGAAAAUGAA-3' (SEQ ID NO: 78), and 5'-UUCAUUGAAAAAUGAA-3' (SEQ ID NO: 79).

46. The vector system of claim 38, wherein the scaffold region comprises an engineered tracrRNA consisting of a nucleotide sequence selected from the group consisting of SEQ ID NOs: 87 to 132 and/or an engineered crRNA consisting of a nucleotide sequence selected from the group consisting of SEQ ID NOs: 133 to 148.

47. The vector system of claim 38, wherein the guide RNA comprises a scaffold region sequence consisting of a nucleotide sequence selected from the group consisting of SEQ ID NOs: 13 and 149 to 186.

48. The vector system of claim 36, wherein the vector is selected from the group consisting of a retroviral (retrovirus) vector, a lentiviral (lentivirus) vector, an adenoviral (adenovirus) vector, an adeno-associated viral (adeno-associated virus) vector, a vaccinia viral (vaccinia virus) vector, a poxviral (poxvirus) vector, a herpes simplex viral (herpes simplex virus) vector, and a phagemid vector.

49. The vector system of claim 36, wherein the vector is selected from the group consisting of plasmid, naked DNA, DNA complex, mRNA (transcript), and amplicon.

50. A recombinant virus produced by the vector system of any one of claims 36 to 49.

51. A method for modifying a target nucleic acid in a cell, comprising bringing, into contact with the cell, the engineered Cas protein of any one of claims 1 to 15 and a guide RNA, the system or composition of any one of claims 22 to 35, the vector system of any one of claims 36 to 49, or the recombinant virus of claim 50.

52. The method of claim 51, wherein the bringing-into-contact is performed *in vitro.*

53. The method of claim 51, wherein the bringing-into-contact is performed *in vivo.*
